# EUROPÄISCHE PATENTSCHRIFT

(11) **EP 2 714 844 B1**
(45) Veröffentlichungstag und Bekanntmachung des Hinweises auf die Patenterteilung: **28.12.2016**
(21) Anmeldenummer: 12722295.8
(22) Anmeldetag: 15.05.2012
(51) Int. Cl.: C09K 19/20, C09K 19/46, C09K 19/34, C09K 19/02, C07C 69/54, C07C 69/602, C08F 120/30, C08F 122/26, C09K 19/54, C09K 19/04, C07C 22/08, C07C 25/13, C07C 25/18, C07C 25/24

(54) **POLYMERISIERBARE VERBINDUNGEN UND IHRE VERWENDUNG IN FLÜSSIGKRISTALLMEDIEN UND FLÜSSIGKRISTALLANZEIGEN**
POLYMERIZABLE COMPOUNDS AND THEIR USE IN LIQUID CRYSTAL MEDIA AND LIQUID CRYSTAL DISPLAYS
COMPOSÉS POLYMÉRISABLES ET UTILISATION DESDITS COMPOSÉS DANS DES MILIEUX CRISTAUX LIQUIDES ET DES ÉCRANS À BASE DE CRISTAUX LIQUIDES

(30) Priorität: 27.05.2011 DE 102011102592
(43) Veröffentlichungstag der Anmeldung: 09.04.2014
(73) Patentinhaber: Merck Patent GmbH, 64293 Darmstadt (DE)
(72) Erfinder: WITTEK, Michael, 64390 Erzhausen (DE); TANAKA, Norihiko, 64285 Darmstadt (DE); TAUGERBECK, Andreas, 64285 Darmstadt (DE)
(86) Internationale Anmeldenummer: PCT/EP2012/002078
(87) Internationale Veröffentlichungsnummer: WO 2012/163478

(56) Entgegenhaltungen:
- US-A1- 2004 011 996
- US-A1- 2009 059 132
- US-A1- 2009 109 392
- US-A1- 2009 268 143
- US-A1- 2010 078 593
- US-A1- 2010 103 366

## Beschreibung

Die vorliegende Erfindung betrifft polymerisierbare Verbindungen, flüssigkristalline Medien enthaltend die Verbindungen, insbesondere Flüssigkristall (FK)-Medien und FK-Anzeigen mit polymerstabilisierter blauer Phase, sowie FK-Medien für FK-Anzeigen des PS- oder PSA-Typs ("polymer sustained" bzw. "polymer sustained alignment").

Im Stand der Technik sind Medien für Anzeigenelemente bekannt, die im Betrieb in der flüssigkristallinen blauen Phase (kurz: blaue Phase) arbeiten. Solche Anzeigen sind beispielsweise in WO 2004/046805 A1 und WO 2008/061606 A1 beschrieben.

Die blaue Phase wird in der Regel am Übergang vom chiral nematischen zum optisch isotropen Zustand beobachtet. Das Medium in der flüssigkristallinen blauen Phase (blau: historische Herkunft) ist in der Regel für die Displayanwendung farblos. Ziel bisheriger Anstrengungen war es, den Temperaturbereich der blauen Phase von weniger als einem Grad auf einen praktisch nutzbaren Bereich auszudehnen (vgl. H. Kikuchi et al., Nature Materials (2002), 1(1), 64-68; Kikuchi, H. et al., Polymeric Materials Science and Engineering, (2003), 89, 90-91).

Zu diesem Zweck wird im Stand der Technik vorgeschlagen, dem FK-Medium eine polymerisierbare Verbindung beizufügen, welche dann im FK-Medium in situ polymerisiert wird. Das dabei gebildete Polymer oder Polymernetzwerk soll die blaue Phase stabilisieren.

Die bisher im Stand der Technik beschriebenen polymerstabilisierten blauen Phasen verwenden beispielsweise als Monomere ein monoreaktives nicht-mesogenes Monomer zusammen mit einem direaktiven mesogenen Monomer.

Die WO 2005/080529 A1 beschreibt beispielsweise polymerstabilisierte blaue Phasen mit mono- und multireaktiven Monomeren.

Der vorliegenden Erfindung lag als eine Aufgabe zugrunde, geeignete Monomere und entsprechende Polymere für die Stabilisierung von blauen Phasen zu finden. Das Polymer soll insbesondere folgende Effekte auf die Eigenschaften der stabilisierten FK-Phase haben:
- breiter Temperaturbereich der blauen Phase,
- schnelle Schaltzeit,
- geringe Betriebsspannung (Vₒₚ),
- geringe Variation der Betriebsspannung mit der Temperatur,
- geringe Hysterese der Transmission einer Zelle bei Änderung der Betriebsspannung zum Erzielen definierter Graustufen.

Es werden außerdem Monomere benötigt, die stabil gegenüber Belastungen durch Licht und Temperatur sind. Weiterhin ist eine gute Löslichkeit in LC-Materialien bzw. eine gute Mischbarkeit mit dem FK-Medium notwendig, um eine gute Verteilung im FK-Medium zu erreichen.

Der vorliegenden Erfindung liegt somit die Aufgabe zugrunde, verbesserte polymerisierbare Verbindungen, sowie solche Verbindungen enthaltende FK-Medien, zur Verfügung zu stellen, insbesondere für die Verwendung in FK-Anzeigen mit einer polymerstabilisierten blauen Phase. Die erfindungsgemäßen polymerisierbaren Verbindungen sollen die blaue Phase stabilisieren. Die erfindungsgemäßen FK-Medien sollen eine oder mehrere verbesserte Eigenschaften, insbesondere ausgewählt aus den oben genannten Eigenschaften, aufweisen. Insbesondere sollen die FK-Medien eine breite blaue Phase aufweisen, ein schnelles Schalten ermöglichen, eine gute Voltage holding ratio (VHR) aufweisen, geringe Spannungen (Vₒₚ) für den Schaltprozess benötigen und eine geringe Hysterese (ΔV) zeigen und einen geringen Memory Effekt (ME) aufweisen. Die FK-Medien sollen stabil gegenüber Belastungen durch Licht- und Temperatur sein.

Weiterhin sind im Stand der Technik sogenannte PS- bzw. PSA-Anzeigen bekannt ("Polymer Sustained" bzw. "Polymer Sustained Alignment"), für die auch gelegentlich der Begriff "Polymer Stabilized" verwendet wird. In diesen Anzeigen wird dem FK-Medium eine geringe Menge (zum Beispiel 0.3 Gew.%, typischerweise <1 Gew.%) einer oder mehrerer polymerisierbarer Verbindung(en) zugesetzt, welche nach Einfüllen in die FK-Zelle mit oder ohne angelegte elektrische Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen.

Nachfolgend wird der Begriff "PSA", falls nicht anders angegeben, stellvertretend für PS-Anzeigen und PSA-Anzeigen verwendet.

Mittlerweile wird das PS(A)-Prinzip in diversen klassischen FK-Anzeigen angewendet. So sind beispielsweise PSA-VA-, PSA-OCB-, PSA-IPS-, PSA-FFS- und PSA-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne angelegte elektrische Spannung. Wie man in Testzellen nachweisen kann, führt das PS(A)-Verfahren zu einem "pretilt" (Anstellwinkel) in der Zelle. Bei PSA-OCB-Anzeigen beispielsweise kann man erreichen, dass die Bend-Struktur stabilisiert wird, so dass man ohne OffsetSpannung auskommt oder diese reduzieren kann. Im Falle von PSA-VA-Anzeigen wirkt sich der "pretilt" positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise auch mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt.

PSA-VA-Anzeigen sind beispielsweise in JP 10-036847 A, EP 1 170 626 A2, US 6,861,107 (US 2004/01996 A1), US 7,169,449, US 2004/0191428 A1, US 2006/0066793 A1, US 2009/0109392 A1 und US 2006/0103804 A1 beschrieben. US 2009/0109392 offenbart eine allgemeine Formel für reaktive Monomere, welche Verbindungen der Formel I umfasst. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PSA-IPS-Anzeigen sind zum Beispiel in US 6,177,972 und Appl. Phys. Lett. 1999, 75(21), 3264 beschrieben. PSA-TN-Anzeigen sind zum Beispiel in Optics Express 2004, 12(7), 1221 beschrieben.

PSA-Anzeigen können ebenso wie die oben beschriebenen konventionellen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. "thin film transistor" bzw. "TFT"), bei Passivmatrix-Anzeigen üblicherweise nach dem Multiplex-Verfahren, wobei beide Verfahren aus dem Stand der Technik bekannt sind.

Es sind jedoch nicht alle Kombinationen bestehend aus FK-Mischung und polymerisierbarer Komponente für PSA-Anzeigen geeignet, weil sich zum Beispiel kein oder kein ausreichender Tilt einstellt, oder weil zum Beispiel die sogenannte "Voltage Holding Ratio" (VHR oder HR) für TFT-Displayanwendungen unzureichend ist. Zudem hat sich gezeigt, dass bei Verwendung in PSA-Anzeigen die aus dem Stand der Technik bekannten FK-Mischungen und RMs noch einige Nachteile aufweisen. So eignet sich nicht jedes bekannte, in FK-Mischungen lösliche RM zur Verwendung in PSA-Anzeigen.

Darüber hinaus sollte die gewählte Kombination FK-Hostmischung/RM eine möglichst geringe Rotationsviskosität sowie möglichst gute elektrische Eigenschaften aufweisen, insbesondere sollte sie eine möglichst hohe VHR besitzen. Bei PSA-Anzeigen ist vor allem eine hohe VHR nach Bestrahlung mit UV-Licht erforderlich, da die UV-Belichtung ein notwendiger Teil des Herstellungsprozesses der Anzeige ist, aber auch als normale Belastung im Betrieb der fertigen Anzeige auftritt.

Insbesondere wäre es wünschenswert, neue Materialien für PSA-Anzeigen zur Verfügung zu haben, die einen besonders kleinen "pretilt"-Winkel erzeugen. Hierbei sind Materialien bevorzugt, die während der Polymerisation bei gleicher Belichtungszeit einen niedrigeren "pretilt"-Winkel erzeugen als die bisher bekannten Materialien, und/oder durch deren Verwendung der mit den bekannten Materialien erzielbare (höhere) "pretilt"-Winkel bereits nach kürzerer Belichtungszeit erreicht werden kann. Dadurch könnten die Produktionszeit (engl. "tact time") der Anzeige verkürzt und die Kosten des Produktionsprozesses verringert werden.

Ein weiteres Problem bei der Herstellung von PSA-Anzeigen ist das Vorhandensein bzw. die Entfernung von Restmengen unpolymerisierter RMs insbesondere nach dem Polymerisationsschritt zur Erzeugung des "pretilt"-Winkels in der Anzeige. Beispielsweise können solche nicht abreagierten RMs die Eigenschaften der Anzeige nachteilig beeinflussen, indem sie z.B. nach Fertigstellung der Anzeige während des Betriebes unkontrolliert polymerisieren.

So zeigen die aus dem Stand der Technik bekannten PSA-Anzeigen oft den unerwünschten Effekt des sogenannten "image sticking" oder "image burn", d.h. dass das in der FK-Anzeige durch vorübergehende Ansteuerung einzelner Bildpunkte (pixel) erzeugte Bild auch nach Abschalten des elektrischen Feldes in diesen Bildpunkten, oder nach Ansteuerung anderer Bildpunkte, noch sichtbar bleibt.

Es ist deshalb wünschenswert, dass die Polymerisation der RMs bei der Herstellung der PSA-Anzeige möglichst vollständig abläuft und die Anwesenheit von unpolymerisierten RMs in der Anzeige möglichst ausgeschlossen oder auf ein Minimum reduziert wird. Hierzu werden Materialien benötigt, die eine möglichst effektive und vollständige Polymerisation ermöglichen.

Es besteht somit immer noch ein großer Bedarf an PSA-Anzeigen sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Zudem besteht ein großer Bedarf nach PSA-Anzeigen, sowie Materialien zur Verwendung in PSA-Anzeigen, die vorteilhafte Eigenschaften aufweisen, insbesondere einen hohen spezifischen Widerstand bei gleichzeitig großem Arbeitstemperaturbereich, kurze Schaltzeiten auch bei tiefen Temperaturen und niedriger Schwellenspannung, einen niedrigen "pretilt"-Winkel, eine Vielzahl von Graustufen, einen hohen Kontrast und einen weiten Blickwinkel ermöglichen, sowie hohe Werte der "voltage holding ratio" (VHR) nach UV-Belastung und der Tieftemperaturstabilität, auch als "LTS" (low temperature stability) bezeichnet, d.h. der Stabilität der FK-Mischung gegen spontane Auskristallisation einzelner Komponenten.

Der Erfindung liegt somit die weitere Aufgabe zugrunde, neue geeignete Materialien, insbesondere RMs und diese enthaltende FK-Medien, für die Verwendung in PSA-Anzeigen bereitzustellen, die die oben angegebenen Nachteile nicht oder in geringerem Maße aufweisen, möglichst schnell und vollständig polymerisieren, eine möglichst schnelle Einstellung eines niedrigen "pretilt"-Winkels ermöglichen, das Auftreten von "image sticking" in der Anzeige verringern oder vermeiden, und vorzugsweise gleichzeitig sehr hohe spezifische Widerstände, niedrige Schwellenspannungen und niedrige Schaltzeiten ermöglichen. Zudem sollten die FK-Medien günstige FK-Phaseneigenschaften sowie hohe VHR- und LTS-Werte aufweisen.

Die oben beschriebenen Aufgaben wurden erfindungsgemäß gelöst durch die Bereitstellung von Materialien, Verfahren und FK-Anzeigen wie in der vorliegenden Anmeldung beschrieben. Insbesondere wurde überraschend gefunden, dass die oben beschriebenen Aufgaben teilweise oder vollständig gelöst werden können, indem man zur Herstellung solcher FK-Anzeigen FK-Medien verwendet, welche eine oder mehrere erfindungsgemäße polymerisierbare Verbindungen wie nachstehend beschrieben enthalten, bzw. indem man FK-Anzeigen mit blauer Phase bzw. PSA-Anzeigen bereitstellt, die eine oder mehrere erfindungsgemäße Verbindungen in polymerisierter Form enthalten.

Die erfindungsgemäßen polymerisierbaren Verbindungen enthalten eine zentrale mesogene Gruppe und mindestens zwei polymerisierbare Gruppen, welche mit der mesogenen Gruppe direkt oder über Abstandsgruppen (engl. "spacer") verknüpft sind, wobei die zentrale mesogene Gruppe aus zwei cyclischen Resten besteht, welche durch eine verbrückende Gruppe (z.B. -CF₂O-, -(CO)O-, etc.) miteinander verknüpft sind.

Die Verwendung der erfindungsgemäßen polymerisierbaren Verbindungen in erfindungsgemäßen FK-Medien für FK-Anzeigen mit polymerstabilisierter blauer Phase führt zu einer deutlichen Stabilisierung der blauen Phase. Zudem hat sich überraschend gezeigt, dass bei der Verwendung der erfindungsgemäßen polymerisierbaren Verbindungen in FK-Medien mit polymerstabilisierter blauer Phase eine deutliche Verringerung der Hysterese (ΔV₅₀) und eine Erhöhung des Kontrastes erzielt wird, im Vergleich zu polymerisierbaren Verbindungen und FK-Medien wie im Stand der Technik beschrieben.

In PSA-Anzeigen führt die Verwendung der erfindungsgemäßen polymerisierbaren Verbindungen in erfindungsgemäßen FK-Medien zu einem besonders schnellen Erreichen des gewünschten "pretilts" und zu deutlich verkürzten Zeiten bei der Herstellung der Anzeige.

Im Stand der Technik, wie beispielsweise in der US 7,440,160 (WO 2004/046805 A1) und den darin zitierten Dokumenten, werden FK-Medien für FK-Anzeigenelemente beschrieben, die im Betrieb in der flüssigkristallinen blauen Phase (kurz: blaue Phase) arbeiten. Die WO 2005/080529 A1 beschreibt polymerstabilisierte blaue Phasen mit mono- und multireaktiven Monomeren. Die US 2009/0267025 A1 (WO 2006/063662 A1) US 2009/051855 A1, US 2009/0059132 A1, US 2009/0059157 A1 und WO 2008/061606 A1 beschreiben die Polymerstabilisierung blauer Phasen mit flüssigkristallinen reaktiven Komponenten (auch als reaktive Mesogene, kurz "RM"s, bezeichnet). In den vorgenannten Veröffentlichungen werden jedoch vorzugsweise RMs mit drei Phenylresten verwendet, wie beispielsweise die folgenden RMs: 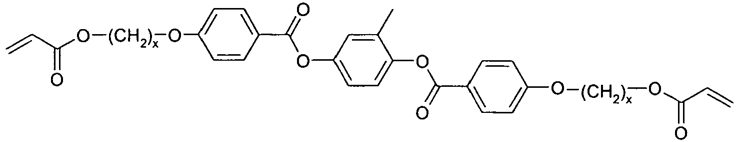 worin x entweder beide 3 oder 6 bedeuten.

Die Verwendung von reaktiven Komponenten, die vorzugsweise aus erfindungsgemäßen polymerisierbaren Verbindungen bestehen, für polymerstabilisierte blaue Phasen oder in PSA-Anzeigen werden im Stand der Technik jedoch weder beschrieben noch nahegelegt.

In der US 7,070,838 werden polymerisierbare Verbindungen enthaltend einen 2-Di- oder Trifluormethyl-1,4-phenylring, sowie deren Verwendung in polymerisierbaren Mischungen, FK-Polymeren und FK-Anzeigen mit cholesterischer Phase sowie in optischen Filmen beschrieben. Darin werden auch konkrete Verbindungen einer Formel 1a-2-19 mit folgender Struktur offenbart:

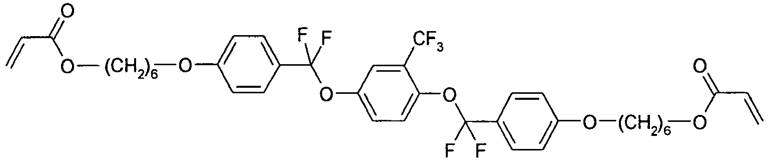

Jedoch werden keine Eigenschaften dieser Verbindung bei Verwendung in einer FK-Anzeige offenbart. Zudem wird die Verwendung solcher Verbindungen zur Stabilisierung von blauen Phasen oder in PSA-Anzeigen durch die US 7,070,838 weder beschrieben noch nahegelegt.

In der JP 2005-015473 A werden polymerisierbare Verbindungen mit ungesättigten Spacergruppen (Alkinylen oder Alkenylen) offenbart. Darin werden auch konkrete Verbindungen der Formeln 1-13-77 bis 1-13-84, 1-13-134, 1-13-135, 1-56-9, 1-56-10, 1-56-23, 1-56-24 offenbart, die über CF_{z}O-Brücken verknüpfte Phenylringe enthalten, sowie deren Verwendung zur Herstellung von optisch anisotropen Filmen und in ferroelektrischen FK-Medien. Darin werden auch konkrete Verbindungen beispielsweise mit folgenden Strukturen offenbart.

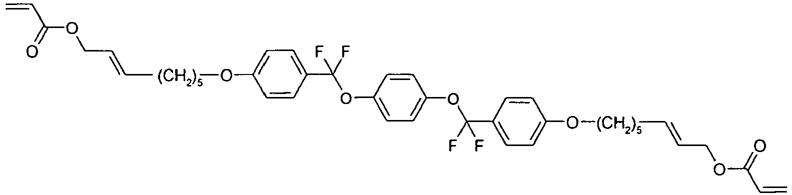

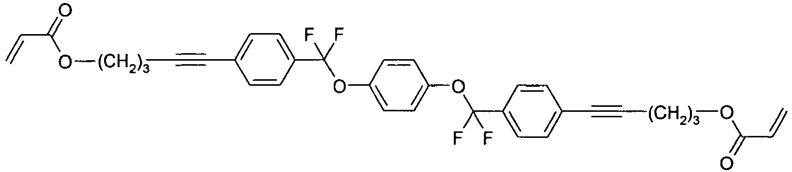

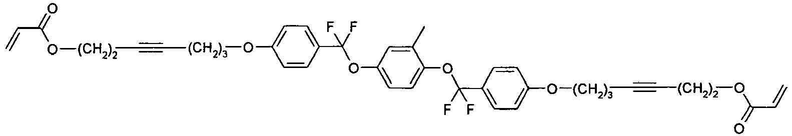

Jedoch wird die Verwendung solcher Verbindungen zur Stabilisierung von blauen Phasen oder in PSA-Anzeigen durch die JP 2005-015473 A weder beschrieben noch nahegelegt.

In den Druckschriften US 2009/0268143 und US 2010/0078593 werden difluoroxymethylenverbrückte polymerisierbare Verbindungen enthaltend ein Ringsystem mit negativer dielektrischer Anisotropie als Komponente in Flüssigkristallmischungen für anisotrope Filme beansprucht.

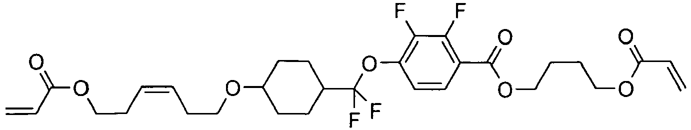

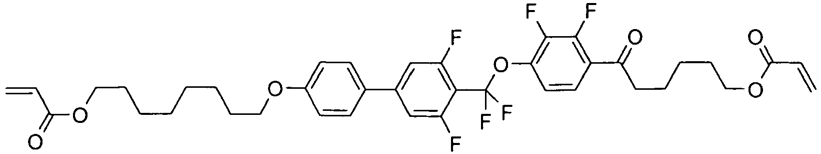

Jedoch werden keine Eigenschaften dieser Verbindungen bei Verwendung in einer FK-Anzeige offenbart. Zudem wird durch diese Druckschriften die Verwendung solcher Verbindungen zur Stabilisierung von blauen Phasen oder in PSA-Anzeigen weder beschrieben noch nahegelegt.

Gegenstand der Erfindung sind die Verbindungen der Formel I und ein FK-Medium enthaltend eine oder mehrere Verbindungen der Formel I

P^{a}-(Sp^{a})ₛ₁-(A¹)ₙ₁-A²-Q¹-A³-(Sp^{b})ₛ₂-P^{b} I

worin die einzelnen Reste folgende Bedeutung besitzen
- P^{a}: eine polymerisierbare Gruppe,
- P^{b}: eine polymerisierbare Gruppe, H oder F, vorzugsweise eine polymerisierbare Gruppe,
- Sp^{a}, Sp^{b}: jeweils unabhängig voneinander eine Abstandsgruppe,
- s1: jeweils unabhängig voneinander 0 oder 1,
- n1, n2: jeweils unabhängig voneinander 0 oder 1, bevorzugt 0,
- Q¹: -CF₂O-,
- A²: eine Gruppe der Formel

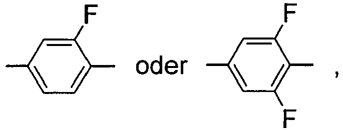

- A³: eine Gruppe der Formel 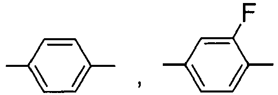 oder 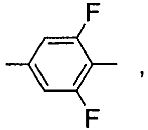
- A¹: jeweils unabhängig voneinander einen Rest ausgewählt aus der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können,
- L: bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
- R⁰, R⁰⁰: jeweils unabhängig voneinander H, F oder geradkettiges oder verzweigtes Alkyl mit 1 bis 12 C-Atomen, worin auch ein oder mehrere H-Atome durch F ersetzt sein können,
- M: -O-, -S-, -CH₂-, -CHY¹- oder -CY¹Y²-, und
- Y¹ und Y²: jeweils unabhängig voneinander eine der oben für R⁰ angegebenen Bedeutungen, Cl oder CN, eine der Gruppen Y¹ und Y² alternativ auch -OCF₃, vorzugsweise H, F, Cl, CN oder CF₃,
oder ein Medium enhaltend ein Polymer erhältlich durch Polymerisation einer oder mehrerer Verbindungen der Formel I,
und die Verwendung in FK-Anzeigen mit blauer Phase oder in FK-Anzeigen des PS- oder PSA-Typs.

Ein weiterer Gegenstand der Erfindung ist ein FK-Medium enthaltend eine oder mehrere Verbindungen der Formel I sowie gegebenenfalls zusätzlich eine oder mehrere weitere polymerisierbare Verbindungen.

Ein weiterer Gegenstand der Erfindung ist ein FK-Medium enthaltend eine oder mehrere Verbindungen der Formel I sowie eine oder mehrere zusätzliche Verbindungen welche auch mesogen, flüssigkristallin und/oder polymerisierbar sein können.

Ein weiterer Gegenstand der Erfindung ist ein FK-Medium enthaltend ein Polymer erhältlich durch Polymerisation einer oder mehrerer Verbindungen der Formel I, sowie optional enthaltend eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente enthaltend eine oder mehrere polymerisierbare Verbindungen der Formel I, oder die polymerisierte Form dieser polymerisierbaren Komponente, sowie
- eine flüssigkristalline Komponente, im Folgenden auch als "FK-Hostmischung" bezeichnet, enthaltend eine oder mehrere, vorzugsweise zwei oder mehr niedermolekulare (d.h. monomere und unpolymerisierbare) Verbindungen wie vor- und nachstehend beschrieben, welche vorzugsweise mesogen oder flüssigkristallin sind.

Ein weiterer Gegenstand der Erfindung ist die Verwendung von FK-Medien enthaltend eine oder mehrere Verbindungen der Formel I in FK-Anzeigen mit blauer Phase oder in FK-Anzeigen des PS- oder PSA-Typs.

Ein weiterer Gegenstand der Erfindung ist ein Verfahren zur Herstellung eines FK-Mediums wie vor- und nachstehend beschrieben, indem man eine oder mehrere niedermolekulare flüssigkristalline Verbindungen oder eine FK-Hostmischung (Flüssigkristallmischung) wie vor- und nachstehend beschrieben, mit einer oder mehreren Verbindungen der Formel I und gegebenenfalls mit chiralen und/oder optisch aktiven Verbindungen und/oder Additiven, mischt.

Ein weiterer Gegenstand der Erfindung ist die Verwendung von Verbindungen der Formel I und diese enthaltende erfindungsgemäße FK-Medien in FK-Anzeigen zur Stabilisierung der blauen Phase, insbesondere über einen möglichst großen Temperaturbereich.

Ein weiterer Gegenstand der Erfindung ist die Verwendung von Verbindungen der Formel I und diese enthaltende erfindungsgemäße FK-Medien in PS- und PSA-Anzeigen zur Erzeugung eines Tiltwinkels im FK-Medium durch *in situ*-Polymerisation der Verbindung(en) der Formel I in der PSA-Anzeige, vorzugsweise unter Anlegen eines elektrischen oder magnetischen Feldes.

Ein weiterer Gegenstand der Erfindung ist eine FK-Anzeige enthaltend eine oder mehrere Verbindungen der Formel I, ein Polymer erhältlich durch Polymerisation einer oder mehrerer Verbindungen der Formel I oder ein erfindungsgemäßes FK-Medium, insbesondere eine PS- oder PSA-Anzeige, besonders bevorzugt eine Anzeige mit blauer Phase, eine PSA-VA-, PSA-OCB-, PSA-IPS-, PSA-FFS- oder PSA-TN-Anzeige.

Ein weiterer Gegenstand der Erfindung ist eine FK-Anzeige des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, vorzugsweise unter Anlegen einer elektrischen Spannung an die Elektroden, dadurch gekennzeichnet, dass mindestens eine der polymerisierbaren Verbindungen aus Formel I ausgewählt ist.

Ein weiterer Gegenstand der Erfindung ist ein Verfahren zur Herstellung einer FK-Anzeige wie vor- und nachstehend beschrieben, indem man ein FK-Medium, enthaltend eine oder mehrere niedermolekulare flüssigkristalline Verbindungen oder eine FK-Hostmischung wie vor- und nachstehend beschrieben sowie eine oder mehrere polymerisierbare Verbindungen, wovon mindestens eine aus Formel I ausgewählt ist, in eine FK-Zelle mit zwei Substraten und zwei Elektroden wie vor- und nachstehend beschrieben füllt, und die polymerisierbaren Verbindungen, vorzugsweise unter Anlegen einer elektrischen Spannung an die Elektroden, polymerisiert.

Die erfindungsgemäßen PS- und PSA-Anzeigen weisen zwei Elektroden, vorzugsweise in Form von transparenten Schichten, auf, wobei diese auf einem oder beiden der Substrate aufgebracht sind, die die FK-Zelle bilden. Dabei ist entweder jeweils eine Elektrode auf je einem der beiden Substrate aufgebracht, wie zum Beispiel in erfindungsgemäßen PSA-VA-, PSA-OCB- oder PSA-TN-Anzeigen, oder beide Elektroden sind auf nur einem der beiden Substrate aufgebracht, während das andere Substrat keine Elektrode aufweist, wie zum Beispiel in erfindungsgemäßen PSA-IPS- oder PSA-FFS-Anzeigen.

Weiterer Gegenstand der Erfindung sind neue Verbindungen der Formel I, Verfahren zu ihrer Herstellung, sowie in diesen Verfahren verwendete oder daraus erhaltene neue Zwischenprodukte, insbesondere Verbindungen der Formel I, sowie deren Unterformeln wie vor- und nachstehend definiert, worin einer oder mehrere der Reste A¹ und A² ausgewählt ist aus der Gruppe d) wie in Formel I definiert, bestehend aus optional substituierten, gesättigten oder teilweise oder vollständig ungesättigten, polycyclischen Resten mit 5 bis 20 cyclischen C-Atomen, von denen auch ein oder mehrere durch Heteroatome ersetzt sein können.

Besonders bevorzugt ist ein FK-Medium, eine FK-Anzeige, ein Verfahren oder die Verwendung wie vor- und nachstehend beschrieben, worin das FK-Medium bzw. die darin enthaltene polymerisierbare oder polymerisierte Komponente keine Verbindungen der folgenden Formel enthalten: 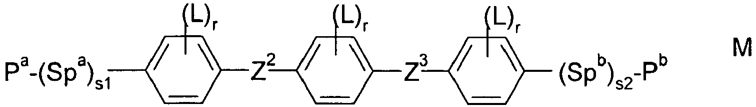 worin P^{a}, P^{b}, Sp^{a}, Sp^{b}, s1, s2 und L r die vor- und nachstehend angegebene Bedeutung besitzen, r 0, 1, 2, 3 oder 4 bedeutet, und Z² und Z³ jeweils unabhängig voneinander -(CO)O- oder -O(CO)- bedeuten.

Vor- und nachstehend gelten folgende Bedeutungen:

Der Begriff "cyclisches C-Atom" bedeutet ein C-Atom, welches mit anderen C-Atomen und/oder Heteroatomen einen carbo- oder heterocyclischen Rest bildet.

Die Begriffe "Tilt" und "Tiltwinkel" beziehen sich auf eine gekippte oder geneigte Orientierung der FK-Moleküle eines FK-Mediums relativ zu den Oberflächen der Zelle in einer FK-Anzeige (hier vorzugsweise einer PS-oder PSA-Anzeige). Der Tiltwinkel bezeichnet dabei den durchschnittlichen Winkel (<90°) zwischen den Moleküllängsachsen der FK-Moleküle (FK-Direktor) und der Oberfläche der planparallelen Trägerplatten, welche die FK-Zelle bilden. Ein niedriger Wert des Tiltwinkels (d.h. eine große Abweichung vom 90°-Winkel) entspricht dabei einem großen Tilt. Eine geeignete Methode zur Messung des Tiltwinkels findet sich in den Beispielen. Soweit nicht anders angegeben, beziehen sich vor- und nachstehend offenbarte Werte des Tiltwinkels auf diese Messmethode.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung die mesogene Gruppe und die polymerisierbare(n) Gruppe(n) miteinander verbindet.

Der Begriff "reaktives Mesogen" oder "RM" bezeichnet eine Verbindung enthaltend eine mesogene Gruppe und eine oder mehrere funktionelle Gruppen, die zur Polymerisation geeignet sind (auch als polymerisierbare Gruppe oder Gruppe P bezeichnet).

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

"Halogen" bedeutet F, Cl, Br oder I.

Definitionen wie "Alkyl, Alkoxy, Alkylcarbonyl, Alkoxycarbonyl, Alkylcarbonyloxy oder Alkoxycarbonyloxy mit 1 bis 12 C-Atomen" etc., bedeuten, dass die Reste enthaltend eine Carbonylgruppe (CO) sowie die ungesättigten Reste wie Alkenyl und Alkinyl mindestens zwei C-Atome, und die verzweigten Reste mindestens drei C-Atome aufweisen.

Die polymerisierbare Gruppe P^{a,b} ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P^{a,b} sind ausgewählt aus der Gruppe bestehend aus CH₂=CW¹-CO-O-, CH₂=CW¹-CO-, 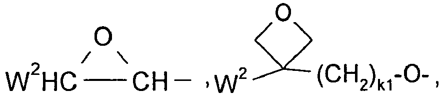 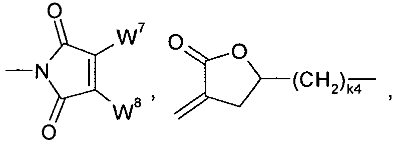 CH₂=CW²-(O)ₖ₃-, CW¹=CH-CO-(O)ₖ₃-, CW¹=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substituiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P^{a,b} sind ausgewählt aus der Gruppe bestehend aus CH₂=CW¹-CO-O-, CH₂=CW¹-CO-, 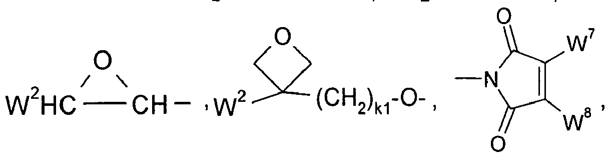 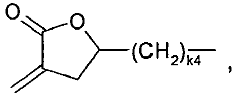 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P^{a,b} sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 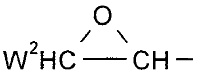 und

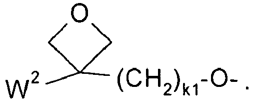

Weitere ganz besonders bevorzugte Gruppen P^{a,b} sind ausgewählt aus der Gruppe bestehend aus Vinyloxy-, Acrylat-, Methacrylat-, Fluoracrylat-, Chloracrylat-, Oxetan- und Epoxygruppen, und bedeuten besonders bevorzugt eine Acrylat- oder Methacrylatgruppe.

Bevorzugte Abstandsgruppen Sp^{a,b} sind ausgewählt aus der Formel Sp"-
- X",: so dass der Rest P^{a/b}-Sp^{a/b}- der Formel P^{a/b}-Sp"-X"- entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C=C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.
X' ist vorzugsweise -O-, -S -CO-, -COO-, -OCO-, -O-COO-, -CO-NR⁰-, -NR⁰-CO-, -NR⁰-CO-NR⁰- oder eine Einfachbindung.

Typische Abstandsgruppen Sp" sind beispielsweise -(CH₂)ₚ₁-, -(CH₂CH₂O)_{q1}-CH₂CH₂-, -CH₂CH₂-S-CH₂CH₂-, -CH₂CH₂-NH-CH₂CH₂- oder -(SiR⁰⁰R⁰⁰⁰-O)ₚ₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)ₚ₁-, -(CH₂)ₚ₁-O-, -(CH₂)ₚ₁-O-CO-, -(CH₂)ₚ₁-O-CO-O-, worin p1 und q1 die oben angegebene Bedeutung haben.

Besonders bevorzugte Gruppen Sp" sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

In einer weiteren bevorzugten Ausführungsform der Erfindung bedeuten P^{a} und/oder P^{b} in Formel I einen Rest mit zwei oder mehreren polymerisierbaren Gruppen (multifunktionelle polymerisierbare Reste). Geeignete Reste dieses Typs, sowie diese enthaltende polymerisierbare Verbindungen und ihre Herstellung sind beispielsweise in US 7,060,200 B1 oder US 2006/0172090 A1 beschrieben. Besonders bevorzugt sind multifunktionelle polymerisierbare Reste ausgewählt aus folgenden Formeln

-X-alkyl-CHP¹-CH₂-CH₂P² I*a

-X-alkyl-C(CH₂P¹)(CH₂P²)-CH₂P³ I*b

-X-alkyl-CHP¹CHP²-CH₂P³ I*c

-X-alkyl-C(CH₂P¹)(CH₂P²)-CₐₐH₂ₐₐ₊₁ I*d

-X-alkyl-CHP¹-CH₂P² I*e

-X-alkyl-CHP¹P² I*f

-X-alkyl-CP¹P²-CₐₐH₂ₐₐ₊₁ I*g

-X-alkyl-C(CH₂P¹)(CH₂P²)-CH₂OCH₂-C(CH₂P³)(CH₂P⁴)CH₂P⁵ I*h

-X-alkyl-CH((CH₂)ₐₐP¹)((CH₂)_{bb}P²) I*i

-X-alkyl-CHP¹CHR²-CₐₐH₂ₐₐ₊₁ I*k

-X'-alkyl-C(CH₃)(CH₂P¹)(CH₂P²) I*m

worin
- alkyl: eine Einfachbindung oder geradkettiges oder verzweigtes Alkylen mit 1 bis 12 C-Atomen bedeutet, worin eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl oder CN ersetzt sein können, wobei R⁰⁰ und R⁰⁰⁰ die oben angegebene Bedeutung haben,
- aa und bb: jeweils unabhängig voneinander 0, 1, 2, 3, 4, 5 oder 6 bedeuten,
- X: eine der für X' angegebenen Bedeutungen besitzt, und
- P¹⁻⁵: jeweils unabhängig voneinander eine der für P^{a} angegebenen Bedeutungen besitzen.

Vorzugsweise bedeutet A¹ in Formel I jeweils unabhängig voneinander einen Rest ausgewählt aus der Gruppe bestehend aus folgenden Formeln: 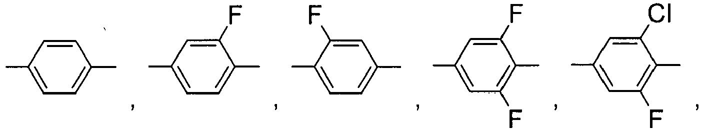 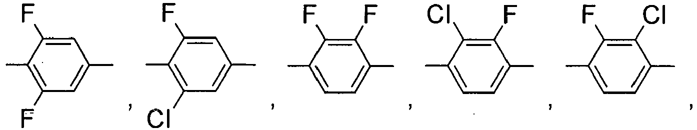 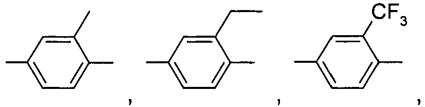 worin die einzelnen Ringe auch zusätzlich ein- oder mehrfach durch L wie vor- und nachstehend beschrieben substituiert sein können.

Besonders bevorzugt bedeutet A¹ in Formel I jeweils unabhängig voneinander einen Rest ausgewählt aus der Gruppe bestehend aus folgenden Formeln:

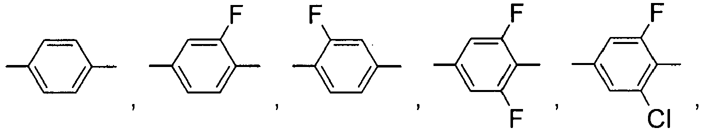

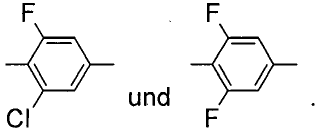

Weitere besonders bevorzugte Verbindungen der Formel I sowie deren vor- und nachstehend angegebenen Unterformeln sind solche worin
- s1 und s2 jeweils 1 bedeuten
- n1 und n2 jeweils 0 bedeuten,
- n1 1 und n2 0 bedeutet oder n1 0 und n2 1 bedeutet,
- A² eine Gruppe der Formel 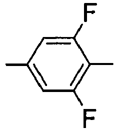 bedeutet.

Die Verbindungen der Formel I sind daher besonders bevorzugt Verbindungen der Formel 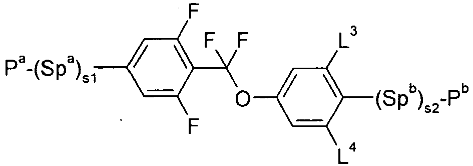 worin P^{a}, P^{b}, Sp^{a}, Sp^{b}, s1, s2 wie für Formel I definiert sind,
und L³ und L⁴ unabhängig voneinander H oder F bedeuten.

Ein hoher Fluorierungsgrad in den Ringen A² und A³ macht die polymerisierbaren Verbindungen sehr gut kombinierbar mit Mischungen aus Verbindungen mit mehrfach fluorierten Aromaten.

Bevorzugt werden die erfindungsgemäßen Verbindungen durch die Formel I* wiedergegeben:

P^{a}-(Sp^{a})ₛ₁-(A¹)ₙ₁-A²-Q¹-A³-(Sp^{b})ₛ₂-P^{b} I*

worin die variablen Gruppen wie für Formel I definiert sind, und bevorzugt die dort bevorzugten Bedeutungen annehmen.

Besonders bevorzugte Verbindungen der Formel I sind ausgewählt aus der Gruppe bestehend aus den folgenden Formeln: 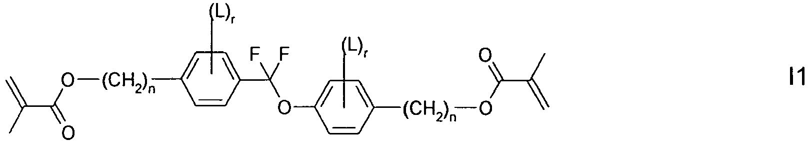 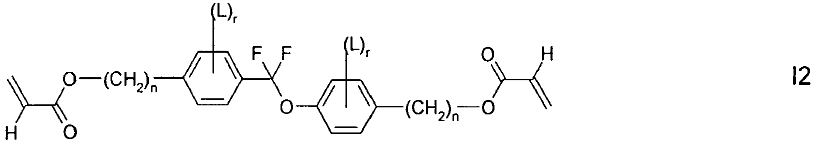 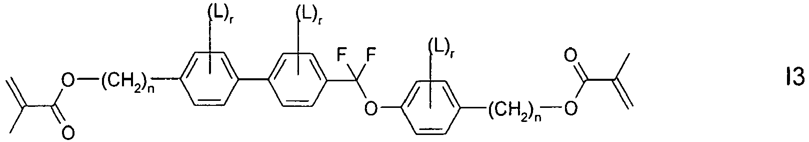 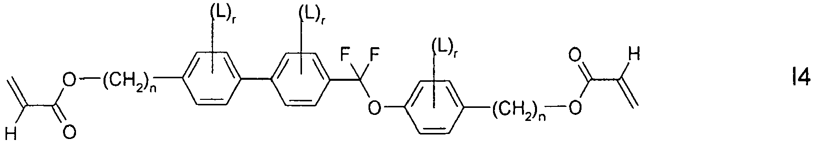 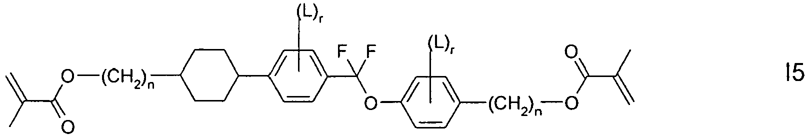 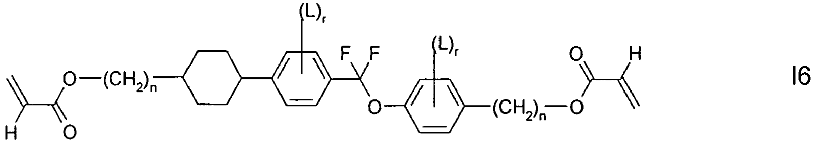 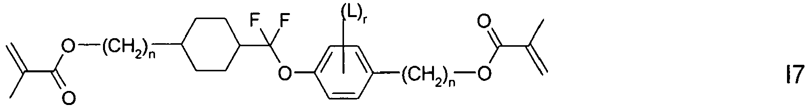 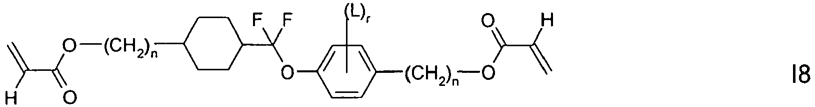 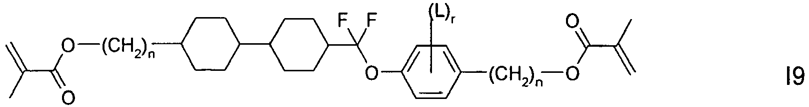 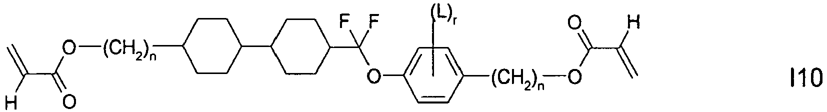 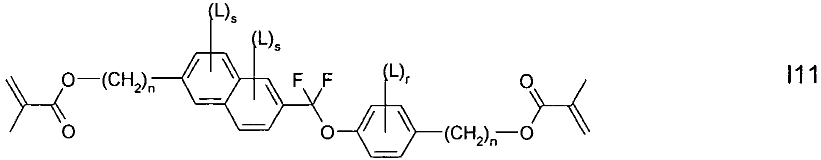 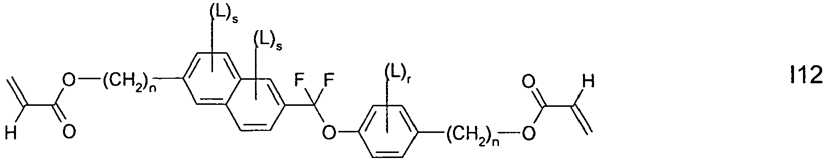 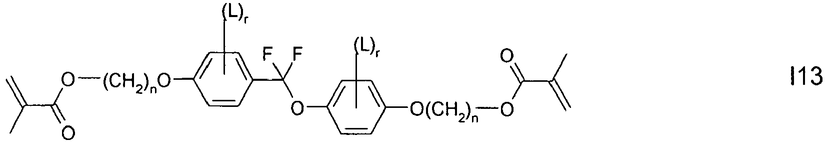 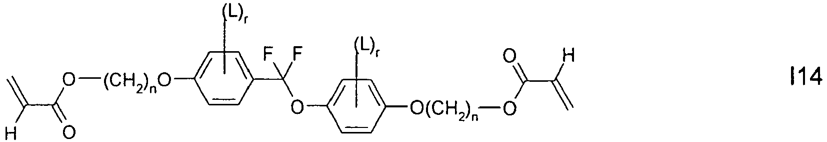 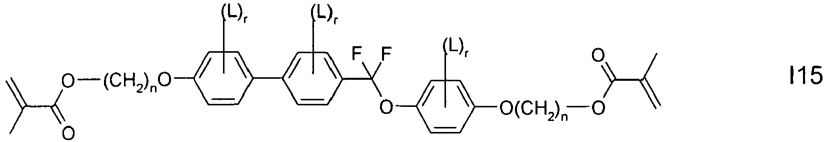 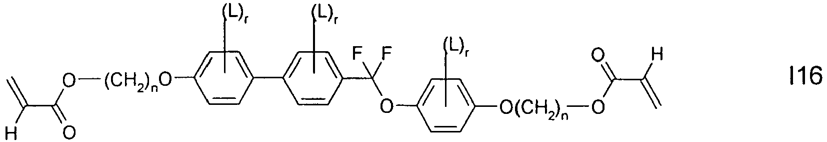 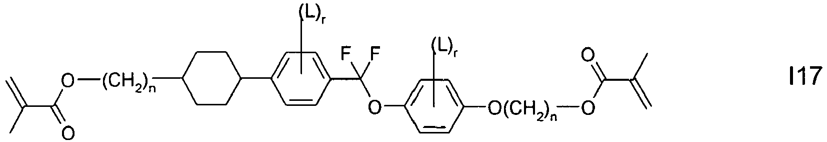 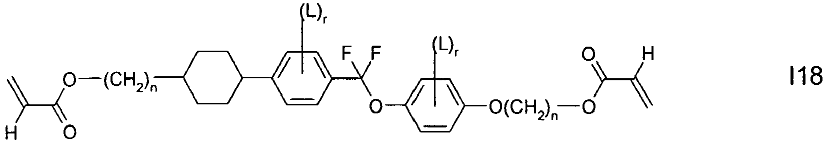 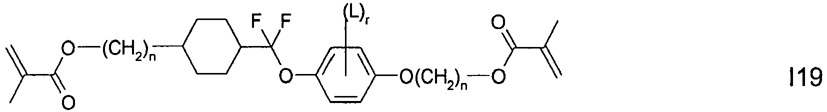 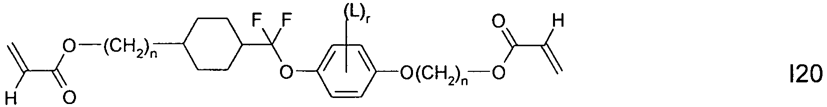 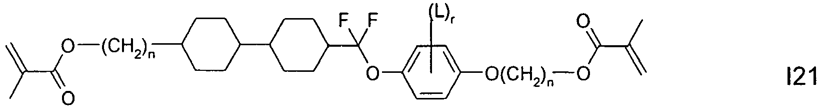 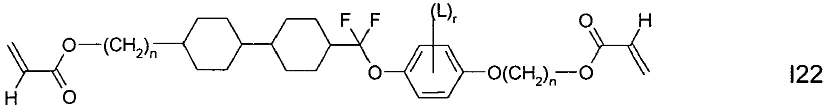 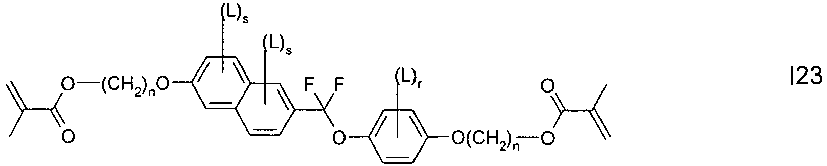 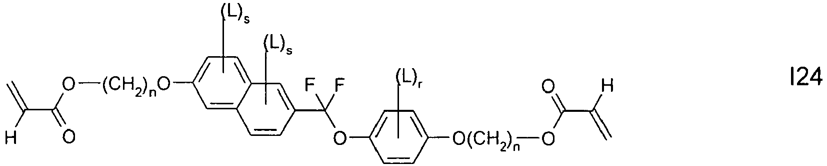 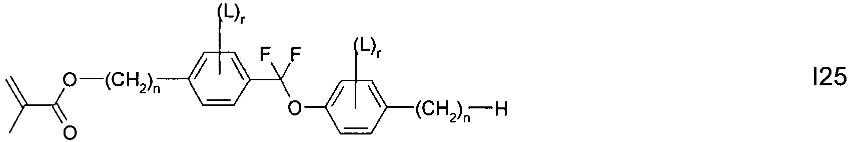 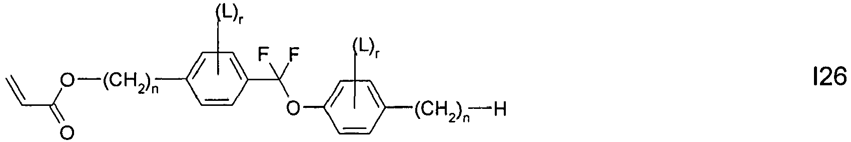 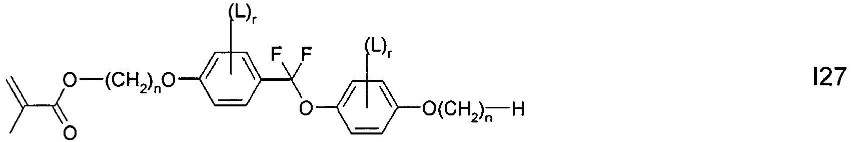 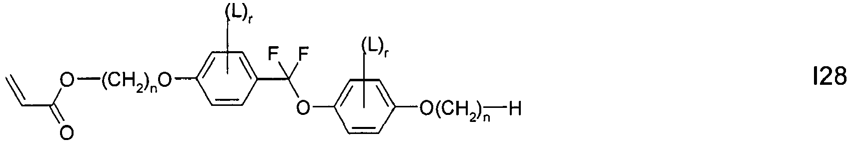 worin L bei jedem Auftreten gleich oder verschieden eine der vor- und nachstehend angegebenen Bedeutungen besitzt, r 0, 1, 2, 3 oder 4 bedeutet, s 0, 1, 2 oder 3 bedeutet und n eine ganze Zahl zwischen 1 und 24, bevorzugt zwischen 1 und 12, ganz besonders bevorzugt zwischen 2 und 8 bedeutet, und worin, falls ein Rest am Ende einer Einfach- bzw. Doppelbindung nicht genannt ist, es sich um eine endständige CH₃- bzw. CH₂-Gruppe handelt. r ist bevorzugt 0, 1 oder 2, und besonders bevorzugt 1 oder 2. Ganz besonders bevorzugt sind darunter die Verbindungen der Formeln I1, I2, I3 und 14, und ganz besonders die Verbindungen der Formel 11 und 12.

Unter den Verbindungen der Formel I sind solche mit zwei Ringsystemen (n1, n2 = 0) bevorzugt, insbesondere Verbindungen ausgewählt aus den Verbindungen der Formeln la, Ib, Id, le, If und Ih: 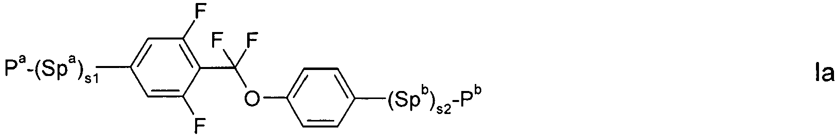 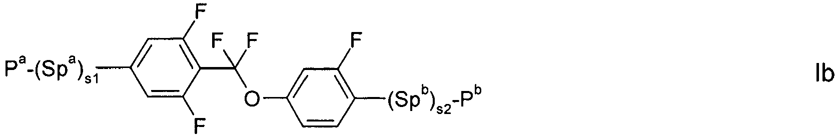 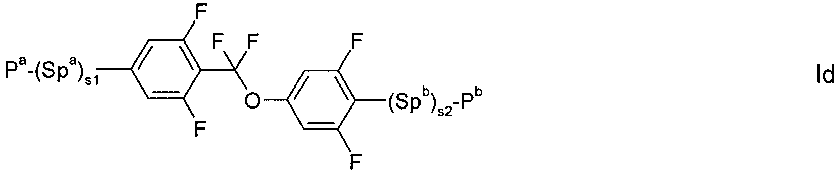 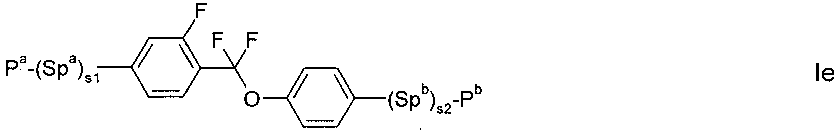 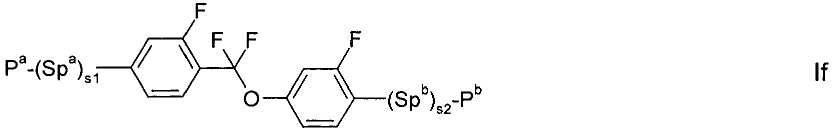 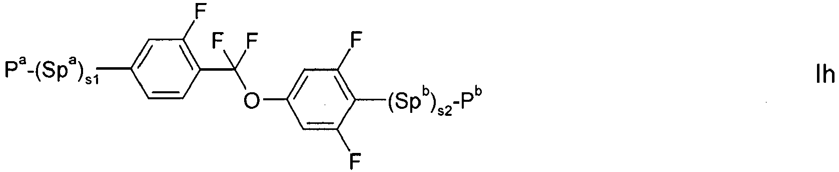 worin P^{a}, P^{b}, Sp^{a}, Sp^{b}, s1 und s2 wie für Formel I definiert sind. Dabei bedeuten bevorzugt Sp^{a/b} eine Alkylengruppe -(CH₂)ₙ- mit n = 3, 4, 5, 6 oder 7, und P^{a/b} eine Metacrylat- oder Acrylatgruppe oder H, insbesondere eine Metacrylat- oder Acrylatgruppe. Besonders bevorzugt sind darunter die Verbindungen der Formeln la, Ib, Id und Ih.

P^{a} und P^{b} bedeuten in den Verbindungen der Formel I sowie deren Unterformeln vorzugsweise Acrylat oder Methacrylat, ferner Fluoracrylat. Sp^{a} und Sp^{b} bedeuten in den Verbindungen der Formeln I und deren Unterformeln vorzugsweise eine Rest ausgewählt aus der Gruppe bestehend aus -(CH₂)ₚ₁-, -(CH₂)ₚ₁-O-, -(CH₂)ₚ₁-O-CO- oder -(CH₂)ₚ₁-O-CO-O- und deren Spiegelbildern, worin p1 eine ganze Zahl von 1 bis 12, vorzugsweise von 1 bis 6, besonders bevorzugt 1, 2 oder 3 bedeutet, wobei diese Gruppen so mit P^{a} oder P^{b} verknüpft sind, dass O-Atome nicht direkt benachbart sind.

Unter den Verbindungen der Formel I sind solche besonders bevorzugt, worin
- die Reste P^{a} und P^{b} ausgewählt sind aus der Gruppe bestehend aus Vinyloxy-, Acrylat-, Methacrylat-, Fluoracrylat-, Chloracrylat-, Oxetan- und Epoxygruppen, besonders bevorzugt Acrylat- oder Methacrylatgruppen,
- die Reste Sp^{a} und Sp^{b} ausgewählt sind aus der Gruppe bestehend aus - (CH₂)ₚ₁-, -(CH₂)ₚ₁-O-, -(CH₂)ₚ₁-O-CO- und -(CH₂)ₚ₁-O-CO-O- und deren Spiegelbildern, worin p1 eine ganze Zahl von 1 bis 12, vorzugsweise von 1 bis 6, besonders bevorzugt 1, 2 oder 3 bedeutet, und wobei diese Reste so mit P^{a} oder P^{b} verknüpft sind, dass O-Atome nicht direkt benachbart sind,

Die Verbindungen der Formel I sowie deren Unterformeln können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Besonders geeignete und bevorzugte Verfahren zur Herstellung von Verbindungen der Formel I sowie deren Unterformeln sind in folgenden Schemata beispielhaft dargestellt und enthalten vorzugsweise einen oder mehrere der nachfolgend beschriebenen Schritte.

Der Fachmann kann die Synthese in geeigneter Weise modifizieren und so zu weiteren erfindungsgemäßen Verbindungen gelangen. Die besonders bevorzugten Verbindungen mit einem Alkoxyspacer oder direkt an den Ring gebundenen Acrylaten werden beispielsweise durch Umsetzung von Phenolderivaten wie z.B. Verbindung **2** mit den Dithianyliumsalzen **3** erhalten. Die dabei zunächst gebildeten Verbindungen **4** werden zu den Verbindungen **5** umgesetzt. Anschließend kann die Hydroxygruppe geeignet funktionalisiert werden, z.B. durch Veresterung mit Methacrylsäure (vgl. **Schema 1**).

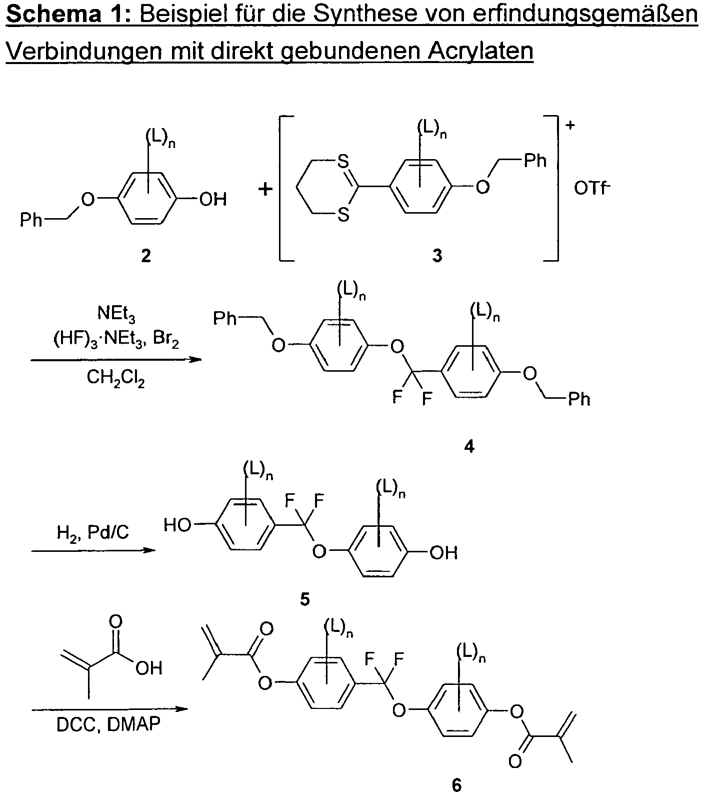

Sollen erfindungsgemäße Verbindungen mit Alkoxyspacern erhalten werden (z.B. Verbindung **8** in **Schema 2**), dann werden die Verbindungen **5** mit Bromalkanolen **7** in Gegenwart einer geeigneten Base umgesetzt. Anschließend kann dann wieder mit Acrylsäuren verestert werden.

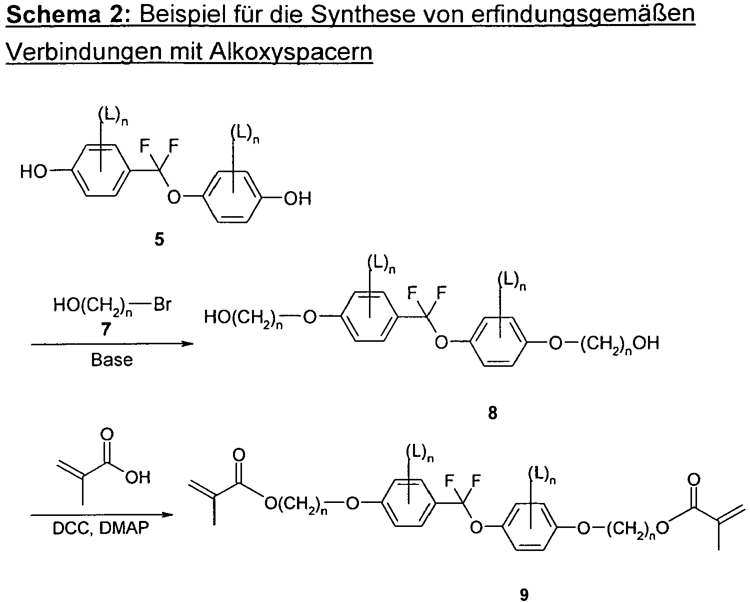

Alternativ kann die Einführung des Spacer auch durch Mitsunobu-Reaktion erfolgen (vgl. **Schema 3**).

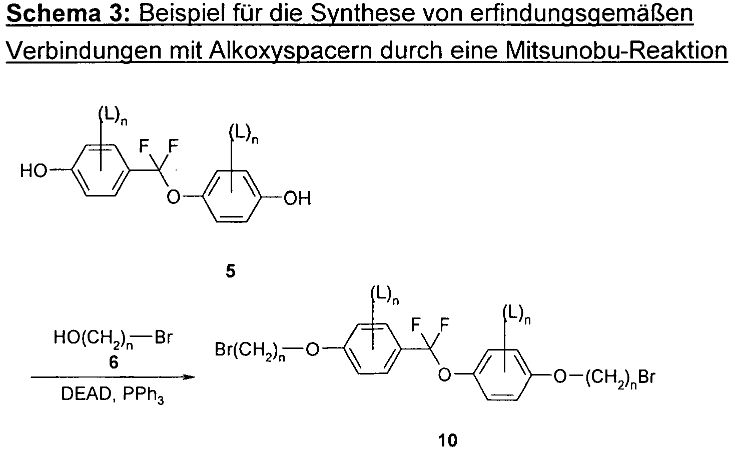

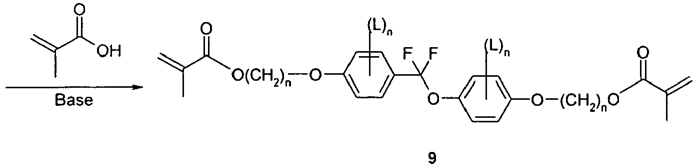

C-C-verknüpfte Spacer können aus den Arylhalogeniden **11,** welche entsprechend den Verbindungen **4** zugänglich sind, z.B. durch Sonogashira-Reaktion mit terminalen Alkinolen erhalten werden (Schema 4). Die anschließende Hydrierung der Verbindungen **12** führt zu den gesättigten Verbindungen **13,** aus welchen die erfindungsgemäßen Acrylate **14** hergestellt werden.

Die polymerisierbaren Verbindungen der Formel I mit nur einer polymerisierbaren Gruppe P^{a} werden ausgehend von Verbindungen hergestellt, die eine der gewünschten nicht polymerisierbaren Gruppen, wie Alkyl oder F, an der entsprechenden Stelle tragen.

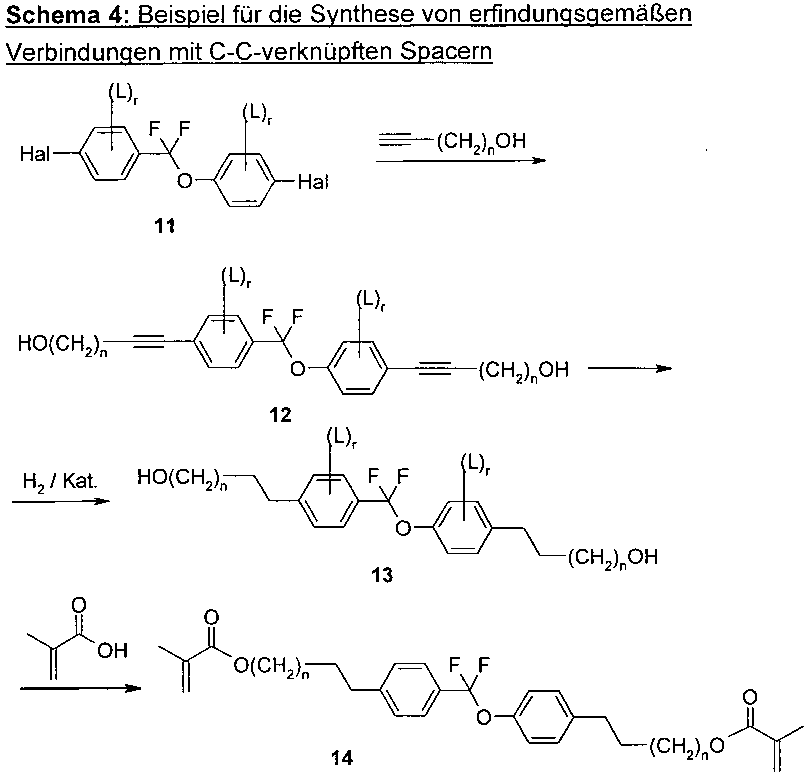

Zur Herstellung von erfindungsgemäßen PSA-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen "pretilt"-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, Irgacure907®, Irgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte. Die Polymerisation kann somit auch ohne Zusatz eines Initiators erfolgen. Somit enthält das FK-Medium in einer bevorzugten Ausführungsform keinen Polymerisationsinitiator.

Die polymerisierbare Komponente oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente, vorzugsweise 10 - 10,000 ppm, besonders bevorzugt 50 - 500 ppm.

Die erfindungsgemäßen polymerisierbaren Verbindungen können einzeln polymerisiert werden, es können aber auch Mischungen polymerisiert werden, welche zwei oder mehr erfindungsgemäße polymerisierbare Verbindungen enthalten, oder Mischungen enthaltend eine oder mehrere erfindungsgemäße polymerisierbare Verbindungen und eine oder mehrere weitere polymerisierbare Verbindungen (Comonomere), welche vorzugsweise mesogen oder flüssigkristallin sind. Bei Polymerisation solcher Mischungen entstehen Copolymere. Bevorzugt wird eine Mischung aus zwei oder mehreren erfindungsgemäßen Verbindungen oder eine Mischung enthaltend eine oder mehrere erfindungsgemäße Verbindungen mit einer oder mehreren weiteren polymerisierbaren Verbindungen verwendet. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte Comonomere für die Verwendung in erfindungsgemäßen Anzeigen sind beispielsweise ausgewählt aus den folgenden Formeln: 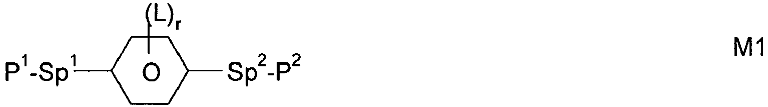 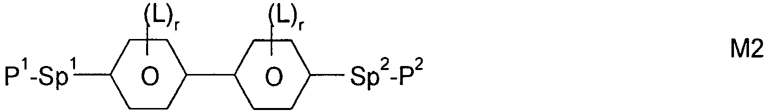 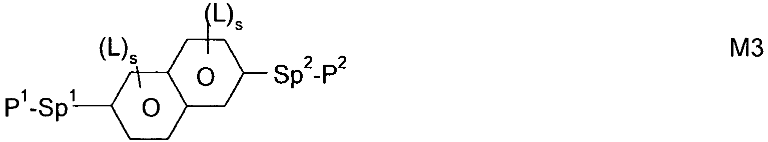 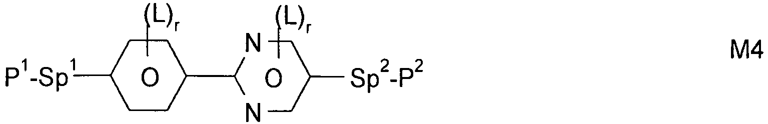 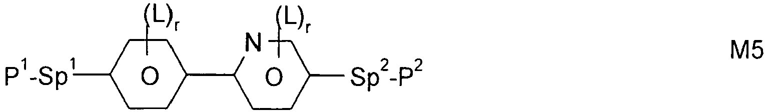 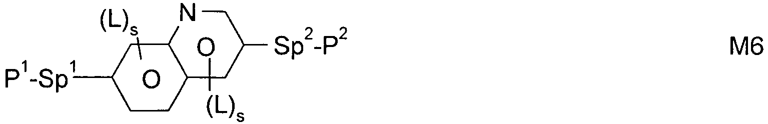 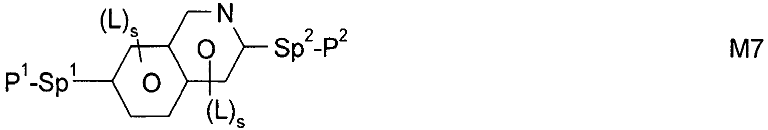 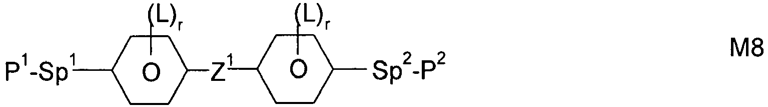 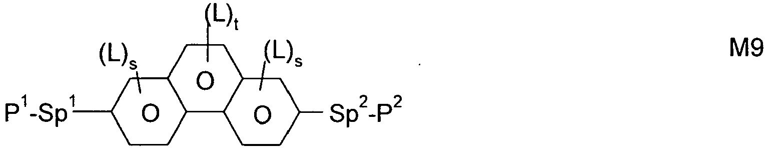 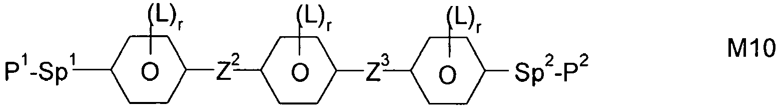 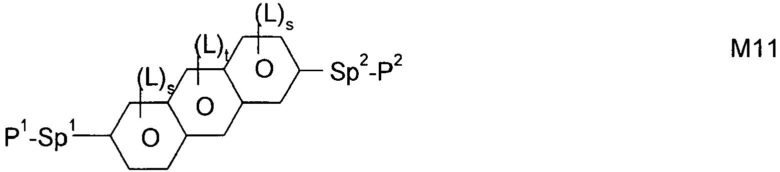 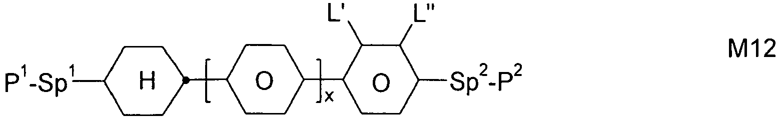 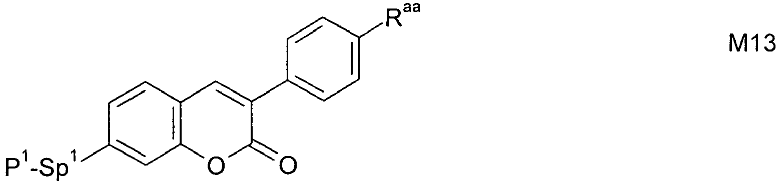 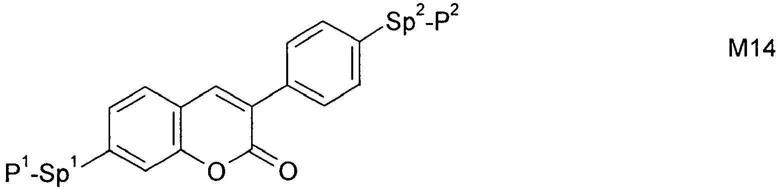 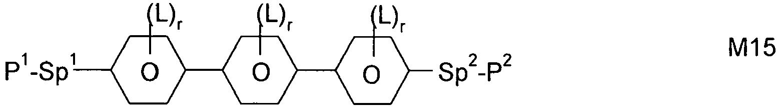 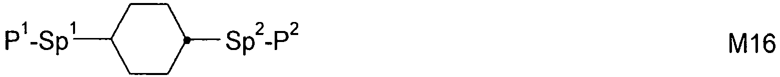 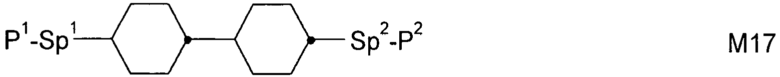 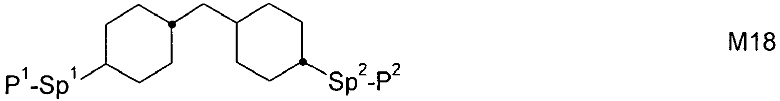 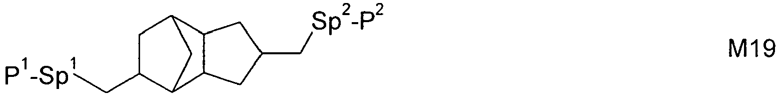 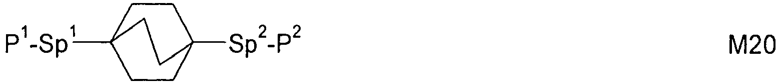 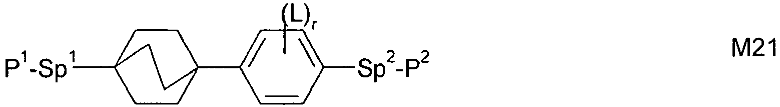 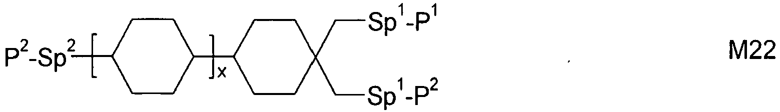 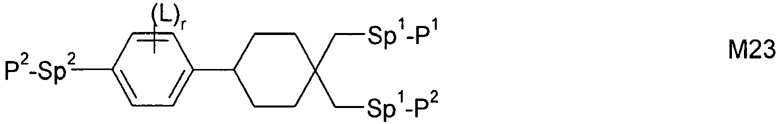 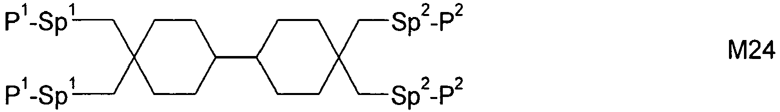 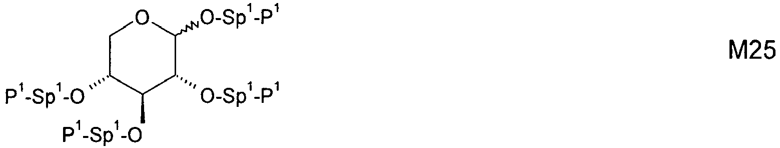 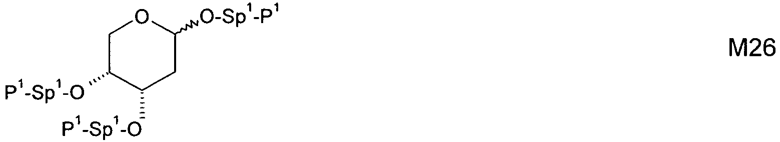 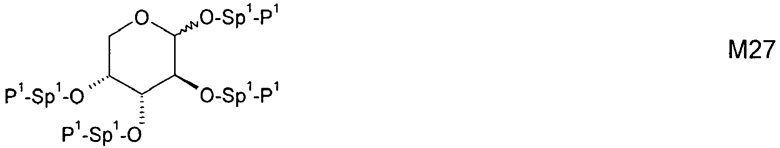 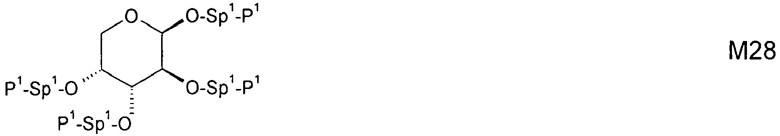 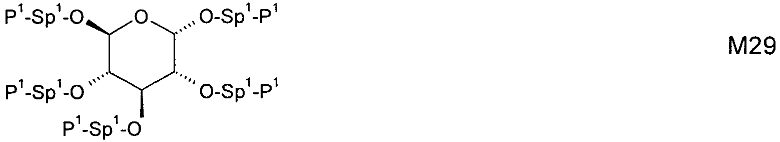 worin die einzelnen Reste folgende Bedeutung besitzen:
- P¹ und P²: jeweils unabhängig voneinander eine polymerisierbare Gruppe, vorzugsweise mit einer der vor- und nachstehend für P^{a} angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp^{a} angegebenen Bedeutungen, und besonders bevorzugt -(CH₂)p₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt,
wobei auch einer oder mehrere der Reste P¹-Sp¹- und P²-Sp²- einen Rest R^{aa} bedeuten können, mit der Maßgabe dass mindestens einer der vorhandenen Reste P¹-Sp¹- und P²-Sp²- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-,oder -CF₂CF₂-,
- Z² und Z³: jeweils unabhängig voneinander-CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

Geeignete und bevorzugte Comonomere für die Verwendung in erfindungsgemäßen Anzeigen in der blauen Phase sind beispielsweise ausgewählt aus monoreaktiven Verbindungen, bevorzugt in einer Menge von 1 bis 9%, besonders bevorzugt 4 bis 7%. Bevorzugte monoreaktive Verbindungen sind solche der Formeln M1 bis M29, worin einer oder mehrere der Reste P¹-Sp¹- und P²-Sp²- einen Rest R^{aa} bedeuten, sodass nur eine reaktive Gruppe vorliegt.

Bevorzugte monoreaktive Verbindungen sind Verbindungen der Formeln 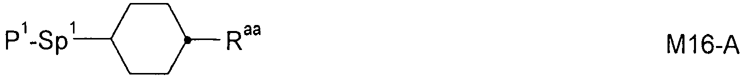 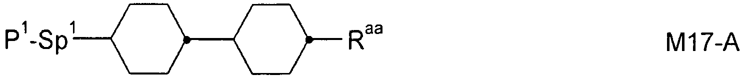 worin P¹, Sp¹ und R^{aa} wie vorangehend definiert sind.

Vorzugsweise enthält das FK-Medium oder die polymerisierbare Komponente für eine PS- oder PSA-Anzeige zusätzlich zu den Verbindungen der Formel I eine oder mehrere Verbindungen ausgewählt aus der Gruppe bestehend aus den Formeln M16-M29, besonders bevorzugt bestehend aus den Formeln M16-M21, ganz besonders bevorzugt bestehend aus den Formeln M16, M17 und M18. Für diese Anzeigen besteht die polymerisierbare Komponente vorzugsweise überwiegend aus direaktiven Verbindungen.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA-und OCB-Anzeigen geeignete FK-Mischung.

Geeignete FK-Mischungen sind dem Fachmann bekannt und in der Literatur beschrieben. FK-Medien für VA-Anzeigen sind in EP 1 378 557 A1, FK-Medien für OCB-Anzeigen sind in EP 1 306 418 A1 und DE 102 24 046 A1 beschrieben. FK-Medien für FK-Anzeigen mit blauer Phase sind in WO 2006/063662 A1 sowie den darin zitierten Dokumenten beschrieben.

Besonders bevorzugte FK-Medien zur Verwendung in FK-Anzeigen mit blauer Phase werden im Folgenden beschrieben:

Vorzugsweise enthält ein erfindungsgemäßes FK-Medium mit blauer Phase
- eine polymerisierbare Komponente A, vorzugsweise in einer Konzentration von 1 bis 25%, besonders bevorzugt von 2 bis 20%, ganz besonders bevorzugt von 3 bis 10%, enthaltend, vorzugsweise hauptsächlich bestehend aus, ganz besonders bevorzugt ausschließlich bestehend aus, einer oder mehrerer Verbindungen der Formel I und optional einer oder mehrerer zusätzlicher polymerisierbarer Verbindungen, und
- eine flüssigkristalline Komponente B, enthaltend eine oder mehrere, vorzugsweise zwei oder mehr niedermolekulare (d.h. monomere und unpolymerisierbare) Verbindungen, vorzugsweise in einer Konzentration von 5-100%, vorzugsweise mit einer positiven dielektrischen Anisotropie, vorzugsweise hauptsächlich bestehend aus, ganz besonders bevorzugt ausschließlich bestehend aus, einer oder mehrerer Verbindungen der Formel II 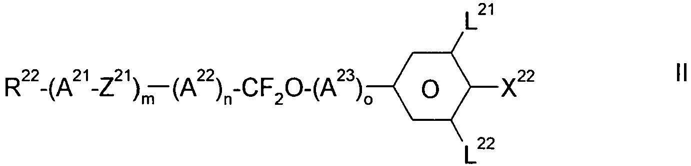 worin die einzelnen Reste folgende Bedeutung besitzen
   - R²²: H, F, Cl oder geradkettiges oder verzweigtes Alkyl mit 1 bis 20 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -SiR⁰¹R⁰²-, -CO-, -COO-, -OCO-, -OCO-O-, -CY⁰¹=CY⁰²- oder -C≡C- so ersetzt sein können, dass O- und/oder S-Atome nicht direkt miteinander verknüpft sind,
   - Y⁰¹, Y⁰²: jeweils unabhängig voneinander F, Cl oder CN, einer der Reste Y⁰¹ und Y⁰² auch H,
   - R⁰¹, R⁰²: jeweils unabhängig voneinander H oder Alkyl mit 1 bis 12 C-Atomen,
   - A²¹, A²², A²³: jeweils unabhängig voneinander und bei jedem Auftreten gleich oder verschieden

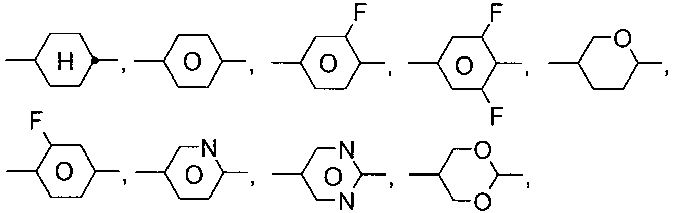

   - Z²¹: jeweils unabhängig voneinander, und bei jedem Auftreten gleich oder verschieden, eine Einfachbindung, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- oder -O-CO-,
   - X²²: F, Cl, -CN, -NCS, -SF₅, -SO₂CF₃, oder Alkyl, Alkenyl, Alkenyloxy, Alkylalkoxy oder Alkoxy mit 1 bis 3 C-Atomen, welches durch F, Cl oder CN ein- oder mehrfach substituiert ist,
   - L²¹, L²²: jeweils unabhängig voneinander H oder F,
   - m: 0, 1 oder 2,
   - n: 1, 2 oder 3,
   - o: 0, 1 oder 2, wobei
   - m + n + o: 1, 2, 3 oder 4, vorzugsweise 2, 3 oder 4 bedeutet,
- optional eine flüssigkristalline Komponente C, enthaltend eine oder mehrere, vorzugsweise zwei oder mehr niedermolekulare (d.h. monomere und unpolymerisierbare) Verbindungen, vorzugsweise in einer Konzentration von 1 bis 25 %, vorzugsweise hauptsächlich bestehend aus, ganz besonders bevorzugt ausschließlich bestehend aus, einer oder mehrerer Verbindungen der Formel III 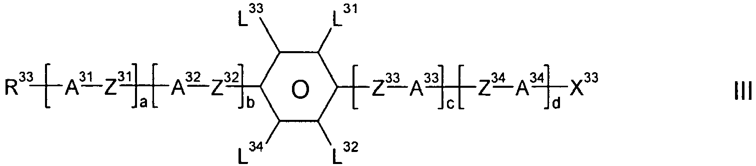 worin
   - a, b, c, d: jeweils unabhängig voneinander 0, 1 oder 2, wobei
   - a + b + c + d: 0, 1, 2, 3 oder 4 ist,
   - A³¹, A³², A³³,: A³⁴ jeweils unabhängig voneinander, und bei jedem Auftreten gleich oder verschieden,

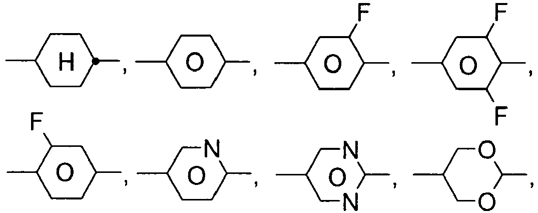

   - Z³¹, Z³², Z³³, Z³⁴: jeweils unabhängig voneinander, und bei jedem Auftreten gleich oder verschieden, eine Einfachbindung, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- oder -O-CO-,
   - R³³: Alkyl oder Alkoxy mit 1 bis 15 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, -CH=CH-, -C≡C-, -CO-O- und/oder -O-CO- so ersetzt sein können, dass O- und/oder S-Atome nicht direkt miteinander verknüpft sind, vorzugsweise ein geradkettiger Alkyl-, Alkoxy-, Alkenyl-, Alkenyloxy- oder -O-Alkylen-O-Rest mit bis zu 10 C-Artomen, welcher unsubstituiert oder ein- oder mehrfach durch F oder Cl substituiert ist,
   - L³¹, L³², L³³, L³⁴: jeweils unabhängig voneinander H, F, Cl, CN, oder Alkyl oder Alkoxy mit 1 bis 15 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, -CH=CH-, -C≡C-, -CO-O- und/oder -O-CO- so ersetzt sein können, dass O- und/oder S-Atome nicht direkt miteinander verknüpft sind, mit der Massgabe dass mindestens einer der Reste L³¹, L³², L³³ und L³⁴ von H verschieden ist,
   - X³³: F, Cl, CF₃, OCF₃, CN, NCS, -SF₅ oder -SO₂-R^{z},
   - R^{x} und R^{y}: jeweils unabhängig voneinander H, Alkyl oder Alkoxy mit 1 bis 7 C-Atomen, vorzugsweise Methyl, Ethyl, Propyl oder Butyl, und
   - R^{z}: Alkyl mit 1 bis 7 C-Atomen, welches unsubstituiert, oder durch F oder Cl ein- oder mehrfach substituiert ist, vorzugsweise CF₃, C₂F₅ oder n-C₄F₉,
- eine Komponente D, vorzugsweise in einer Konzentration von 1-20%, enthaltend eine oder mehrere optisch aktive und/oder chirale Verbindungen, vorzugsweise mit einer HTP ≥ 20 µm⁻¹, vorzugsweise ≥ 40 µm⁻¹, ganz besonders bevorzugt ≥ 60 µm⁻¹.

Die chirale Komponente D enthält vorzugsweise eine oder mehrere chirale Verbindungen mit mesogener Struktur und weist vorzugsweise eine oder mehrere Mesophasen auf, besonders bevorzugt mindestens cholesterische Phase. Bevorzugte chirale Verbindungen der Komponente D sind beispielsweise aus dem Stand der Technik bekannte und/oder kommerziell erhältliche chirale Dotierstoffe wie Cholesteryl-nonanoat (CN), R/S-811, R/S-1011, R/S-2011, R/S-3011, R/S-4011, R/S-5011 oder CB-15 (Merck KGaA, Darmstadt). Besonders bevorzugt sind chirale Dotierstoffe mit einer oder mehreren chiralen Gruppen und einer oder mehreren mesogenen Gruppen, wie beispielsweise in DE 34 25 503,
DE 35 34 777, DE 35 34 778, DE 35 34 779, DE 35 34 780,
DE 43 42 280, EP 01 038 941 und DE 195 41 820 offenbart. Ferner bevorzugt sind Sorbitole wie z.B. in WO 98/00428 A1 beschrieben, Hydrobenzoine wie z.B. in GB 2 328 207 A beschrieben, chirale Binaphthole wie z.B. WO 02/94805 A1 beschrieben, chirale Binaphtholacetale wie z.B. in WO 02/34739 A1 beschrieben, chirale TADDOLe wie z.B. in WO 02/06265 A1 beschrieben, oder chirale Verbindungen mit fluorierten Brückengruppen wie z.B. in WO 02/06196 A1 oder WO 02/06195 A1 beschrieben.

Der Klärpunkt des erfindungsgemäßen FK-Mediums mit blauer Phase vor der Polymersiation liegt vorzugsweise im Bereich von 10 °C bis 100 °C, besonders bevorzugt im Bereich 10 °C bis 60 °C, und ganz besonders bevorzugt im Bereich von 20 °C bis 40 °C.

Das FK-Medium enthält vorzugsweise eine, zwei, drei, vier oder mehr als vier chirale Verbindungen. Das FK-Medium enthält vorzugsweise chirale Verbindungen in einer Gesamtkonzentration von 0.01 bis 25%, bevorzugt 0.1-20%, besonders bevorzugt 0.5 bis 20%, ganz besonders 3-15%.

Der Anteil der Verbindungen der Formel I am Gesamtgehalt aller polymerisierbaren Verbindungen im FK-Medium, bzw. der Anteil der Verbindungen der Formel I an der polymerisierbaren Komponente A), beträgt vorzugsweise 20 bis 80%, besonders bevorzugt 40 bis 60%.

In einer weiteren bevorzugten Ausführungsform beträgt der Anteil der Verbindungen der Formel I am Gesamtgehalt aller polymerisierbaren Verbindungen im FK-Medium, bzw. der Anteil der Verbindungen der Formel I an der polymerisierbaren Komponente A), mindestens 50%, und besonders bevorzugt 60% bis 100%.

Weitere besonders bevorzugte Ausführungsformen sind nachfolgend beschrieben:
- Das FK-Medium enthält eine, zwei oder drei Verbindungen der Formel I;
- Die Komponente B enthält zusätzlich zu den Verbindungen der Formel II eine oder mehrere Esterverbindungen der Formel Z: 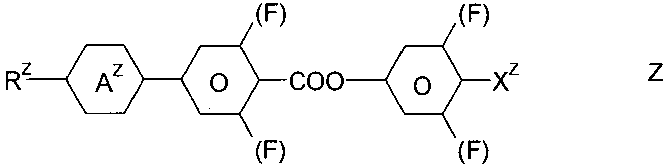 worin R^{z} eine der für R²² in Formel II angegebenen Bedeutungen hat, 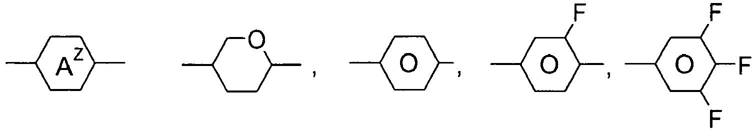
X^{z} F, Cl, CN, NCS, OCF₃, CF₃ or SF₅ bedeutet, und (F) F oder H bedeutet,
vorzugsweise in einer Konzentration von 5 bis 35%, besonders bevorzugt 10 bis 30%, ganz besonders bevorzugt von 10 bis 20%.

Besonders bevorzugt sind FK-Medien, welche neben einer oder mehreren Verbindungen der Formel I eine oder mehrere Verbindungen der Formel II enthalten, insbesondere worin X²² F, Cl, CN, NCS, CF₃ oder OCF₃ bedeutet. Die Verbindungen der Formeln I, II, III und Z sind farblos, stabil und gut untereinander oder mit anderen flüssigkristallinen Substanzen mischbar.

Die einzelnen Komponenten und Verbindungen der Formeln I, II, III und Z der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren.

Die Herstellung der erfindungsgemäßen FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Die erfindungsgemäßen FK-Medien für FK-Anzeigen mit blauer Phase können auch weitere, dem Fachmann bekannte und in der Literatur beschriebene Zusätze oder Additive enthalten, wie beispielsweise Polymerisationsinitiatoren, Inhibitoren, Stabilisatoren oder oberflächenaktive Substanzen. Diese können polymerisierbar oder unpolymerisierbar sein. Polymerisierbare Additive werden dementsprechend der polymerisierbaren Komponente zugerechnet. Unpolymerisierbare Additive werden dementsprechend der flüssigkristallinen Komponente zugerechnet.

Der Aufbau der erfindungsgemäßen FK-Anzeigen mit blauer Phase mit Polarisatoren, Elektrodensubstraten und oberflächenbehandelten Elektrodenschichten entspricht dem konventionellen und dem Fachmann bekannten Aufbau für Anzeigen dieses Typs, wie im Stand der Technik beschrieben, beispielsweise in DE 102 17 273 A1, DE 102 41 301, DE 102 17 273 A1, DE 102 41 301, DE 102 536 06, DE 103 13 979.

Eine erfindungsgemäße FK-Anzeige enthält vorzugsweise folgende Komponenten
- ein oder zwei Substrate,
- eine Elektrodenanordnung mit zwei Elektroden auf nur einem der beiden Substrate oder mit jeweils einer Elektrode auf je einem der beiden Substrate,
- ein oder zwei Polarisatoren, und
- einer zwischen den beiden Substraten befindlichen Schicht eines erfindungsgemäßen FK-Mediums,
wobei die Anzeige bei einer Temperatur betrieben wird, bei der das FK-Medium im nicht-geschalteten Zustand eine optisch isotrope Phase, vorzugsweise eine blaue Phase aufweist.

Der Phasenübergang des FK-Mediums in die blaue Phase erfolgt üblicherweise ausgehend von einer bei tieferen Temperaturen als die blaue Phase existierenden cholesterischen Phase. Die Betriebstemperatur der erfindungsgemäßen FK-Anzeige (d.h. nach Polymerstabilisierung) liegt vorzugsweise über der Temperatur des Phasenübergangs des FK-Mediums in die blaue Phase (d.h. üblicherweise der Übergang cholesterische Phase - blaue Phase), besonders bevorzugt von 0,1 bis 50°, ganz besonders bevorzugt von 0,5 bis 40° über dieser Phasenübergangstemperatur. Weiterhin liegt die Betriebstemperatur der FK-Anzeige vorzugsweise unterhalb der Temperatur des Phasenübergangs des FK-Mediums von der blauen Phase in die isotrope Phase (auch als Klärpunkt bezeichnet). Die Anzeige kann jedoch auch in der isotropen Phase, d.h. oberhalb des Klärpunkts, betrieben werden.

Die erfindungsgemäßen FK-Medien mit blauer Phase können zusätzlich zu den obengenannten Verbindungen der Formeln II und III, und zusätzlich oder alternativ zu den obengenannten Verbindungen der Formeln Z, auch weitere flüssigkristalline Verbindungen enthalten, um z.B. die physikalischen Eigenschaften anzupassen. Solche Verbindungen sind dem Fachmann bekannt. Ihre Konzentration in den FK-Medien ist vorzugsweise 0 bis 30%, besonders bevorzugt 0 bis 20%, ganz besonders bevorzugt 5 bis 15 %.

Vorzugsweise weist die flüssigkristalline Komponente eines erfindungsgemäßen FK-Mediums (d.h. vor Polymerstabilisierung) einen Temperaturbereich der blauen Phase auf, bzw. beim Auftreten mehrerer sequentieller blauer Phasen einen kombinierten Temperaturbereich aller blauen Phasen, dessen gesamte Breite 2°C oder mehr, bevorzugt 5°C oder mehr, besonders bevorzugt 10°C oder mehr, ganz besonders bevorzugt 20°C oder mehr, beträgt.

Vorzugsweise zeigt die flüssigkristalline Komponente eines erfindungsgemäßen FK-Mediums (d.h. vor Polymerstabilisierung) einen Temperaturbereich der blauen Phase(n) mindestens im Bereich von 30°C bis 60°C, besonders bevorzugt von 30°C bis 70°C, und ganz besonders bevorzugt von 40°C bis 90°C.

Dabei bedeutet "mindestens" vor- und nachstehend, dass sich die blaue(n) Phase(n) auch unterhalb des jeweils angegebenen unteren Grenzwertes und/oder oberhalb des jeweils angegebenen oberen Grenzwertes erstrecken können.

Vorzugsweise zeigt ein erfindungsgemäßes FK-Medium mit der polymerisierten Komponente (d.h. nach Polymerstabilisierung) einen Temperaturbereich der blauen Phase(n) mindestens im Bereich von 30°C bis 70°C, bevorzugt von 20°C bis 70°C, besonders bevorzugt von 0°C bis 80°C, ganz besonders bevorzugt von -20°C bis 90°C.

Die Phasenübergangstemperaturen eines erfindungsgemäßen FK-Mediums enthaltend die polymerisierbare Komponente, insbesondere der Klärpunkt und/oder die Temperatur des Übergangs von der cholesterischen Phase in die blaue Phase (T(Ch,BP), auch als T(N*,BP) bezeichnet) und/oder die Temperatur des Übergangs von der blauen Phase in die isotrope Phase (T(BP,I)), werden vorzugsweise durch die Polymerisation der polymerisierbaren Komponente nicht verringert. Das bedeutet, dass die Polymerstabilisierung die blaue(n) Phase(n) vorzugsweise so erfolgt, dass eine oder mehrere der oben angegebenen Phasenübergangstemperaturen (T(Ch,BP), T(BP,I)) nicht zu tieferen Temperaturen verschoben werden, d.h. die blaue(n) Phase(n) wird vorzugsweise mindestens zu tieferen Temperaturen hin, und besonders bevorzugt sowohl zu tieferen als auch zu höheren Temperaturen hin verbreitert.

Besonders bevorzugte FK-Medien zur Verwendung in PSA-Anzeigen, insbesondere in PSA-VA-, PSA-OCB-, PSA-IPS-, PSA-FFS- oder PSA-TN-Anzeigen, werden im Folgenden beschrieben.

Vorzugsweise enthält ein erfindungsgemäßes FK-Medium zur Verwendung in erfindungsgemäßen PSA-VA-, PSA-OCB-, PSA-IPS-, PSA-FFS- oder PSA-TN-Anzeigen:
- < 5%, besonders bevorzugt < 1%, ganz besonders bevorzugt < 0,5% der polymerisierbaren Komponente,
- > 95%, besonders bevorzugt > 99% der flüssigkristallinen Komponente,
- < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln I oder deren Unterformeln,
- eine, zwei oder drei erfindungsgemäße polymerisierbare Verbindungen der Formel I oder deren Unterformeln,
- eine polymerisierbare Komponente, die ausschließlich erfindungsgemäße polymerisierbare Verbindungen der Formel I oder deren Unterformeln enthält,
- eine flüssigkristalline Komponente, die eine FK-Verbindung oder eine FK-Mischung ist, die eine nematische Flüssigkristallphase aufweist,
- eine polymerisierbare und/oder flüssigkristalline Komponente, die ausschließlich achirale Verbindungen enthält,
- eine polymerisierbare Komponente, die eine oder mehrere polymerisierbare Verbindungen mit einer polymerisierbaren Gruppe (monoreaktiv) und eine oder mehrere erfindungsgemäße polymerisierbare Verbindungen mit zwei oder mehr, vorzugsweise zwei polymerisierbaren Gruppen (di- oder multireaktiv) enthält, vorzugsweise ausgewählt aus Verbindungen der Formel I oder deren Unterformeln, und optional aus den oben genanten Comonomeren ausgewählt aus der Liste enthaltend die Formeln M1-M29,
- eine polymerisierbare Komponente, die ausschließlich erfindungsgemäße polymerisierbare Verbindungen mit zwei polymerisierbaren Gruppen (direaktiv) enthält, vorzugsweise ausgewählt aus Verbindungen der Formel I oder deren Unterformeln, und optional zusätzlich aus den oben genanten Comonomeren der Liste enthaltend die Formeln M1-M29,
- außer den erfindungsgemäßen polymerisierbaren Verbindungen, insbesondere der Formel I oder deren Unterformeln, sowie den Comonomeren, keine Verbindungen, die eine endständige Vinyloxygruppe (-O-CH=CH₂) aufweisen,
- 1 bis 5, vorzugsweise 1, 2 oder 3 polymerisierbare Verbindungen, vorzugsweise ausgewählt aus erfindungsgemäßen polymerisierbare Verbindungen, insbesondere der Formel I oder deren Unterformeln.

Besonders bevorzugte FK-Medien zur Verwendung in PSA-VA-Anzeigen werden im Folgenden genannt:
a) FK-Medium, welches eine oder mehrere Verbindungen der Formel CY und/oder PY enthält: 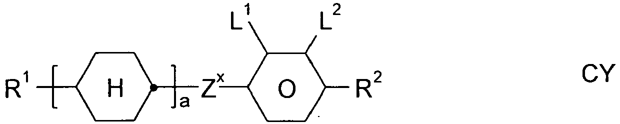 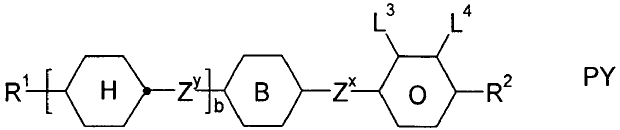 worin die einzelnen Reste folgende Bedeutung besitzen
   - a: 1 oder 2,
   - b: 0 oder 1,

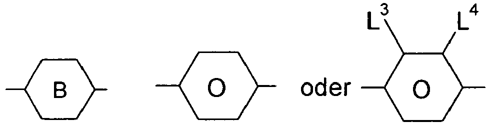

   - R¹und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder-COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander-CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.

Vorzugsweise bedeuten beide Reste L¹ und L² F, oder einer der Reste L¹ und L² F und der andere Cl, bzw. beide Reste L³ und L⁴ F, oder einer der Reste L³ und L⁴ F und der andere Cl.

Die Verbindungen der Formel CY sind vorzugsweise ausgewählt aus der Gruppe bestehend aus folgenden Unterformeln 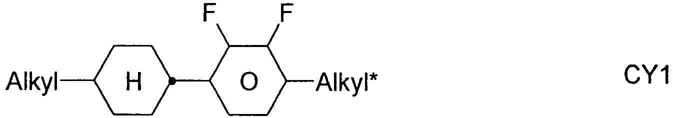 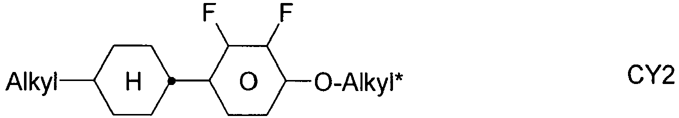 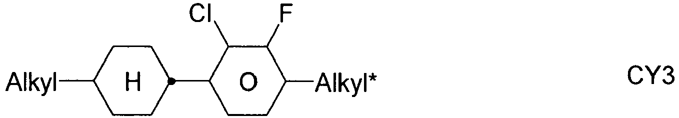 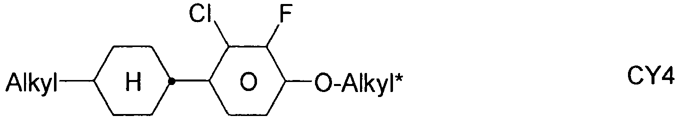 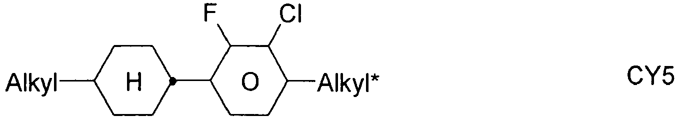 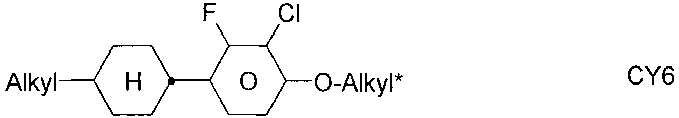 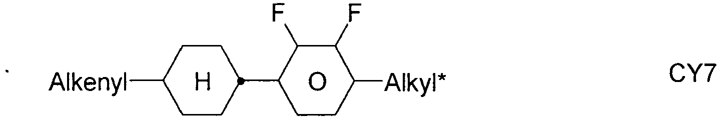 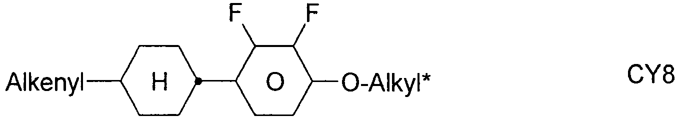 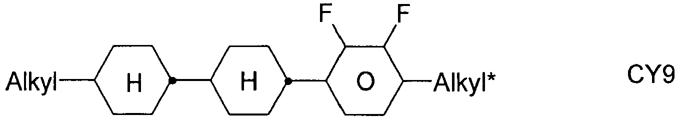 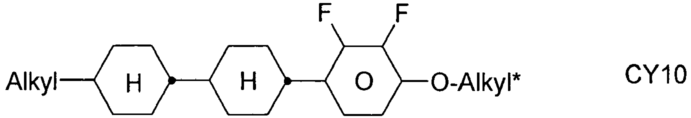 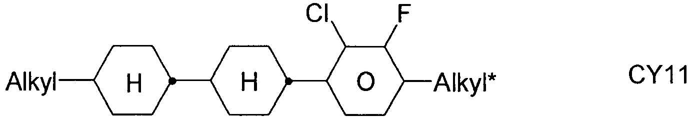 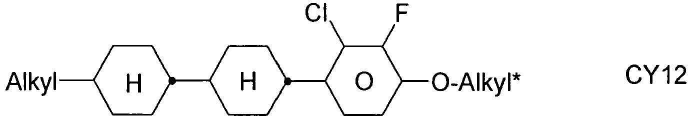 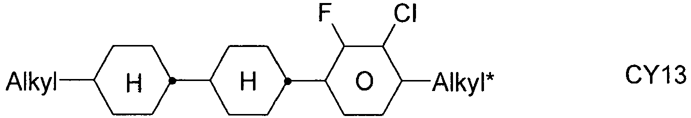 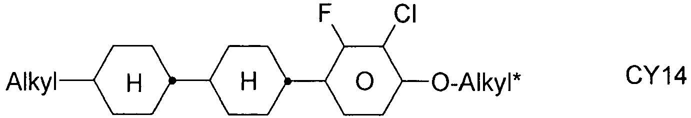 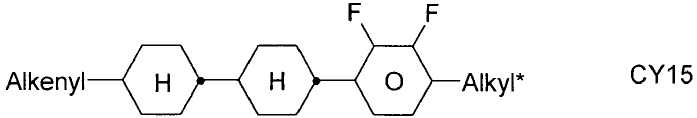 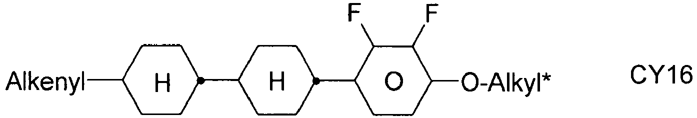 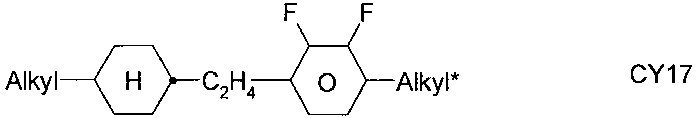 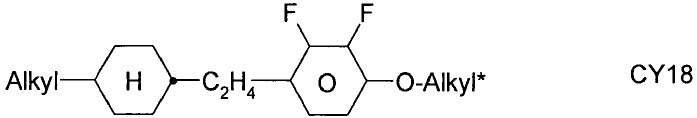 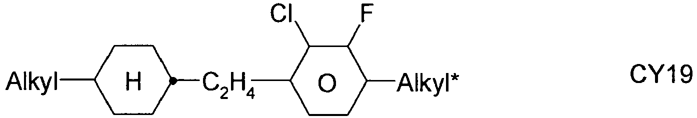 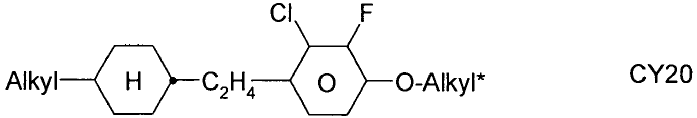 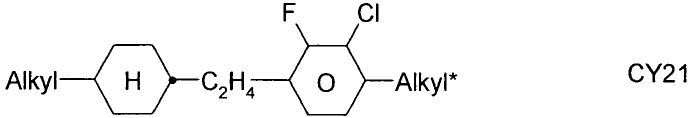 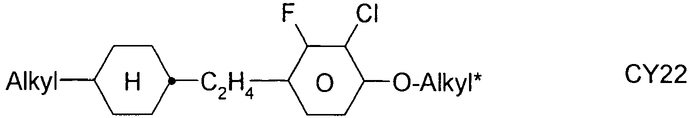 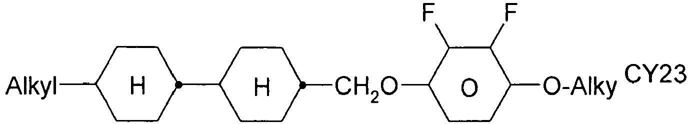 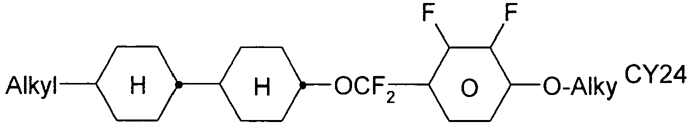 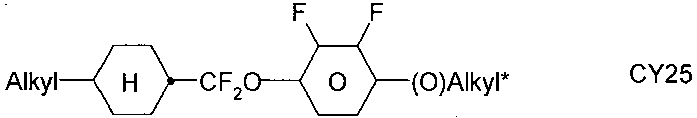 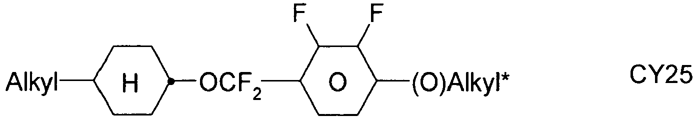 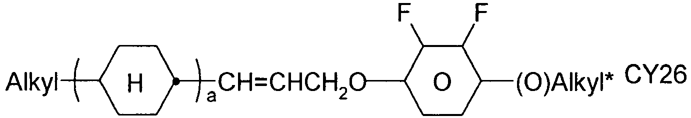 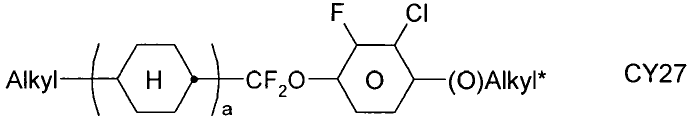 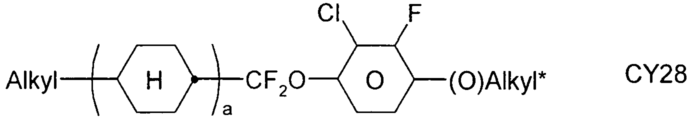 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Die Verbindungen der Formel PY sind vorzugsweise ausgewählt aus der Gruppe bestehend aus folgenden Unterformeln: 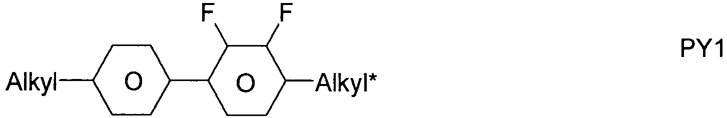 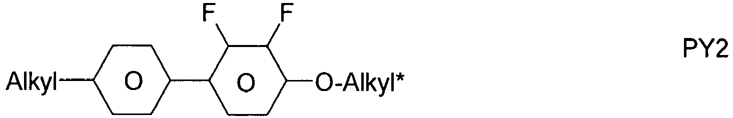 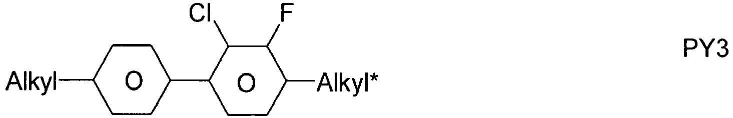 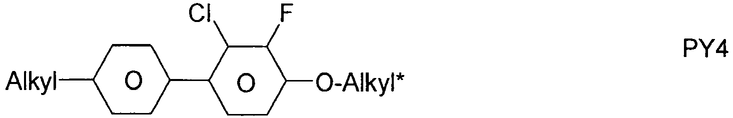 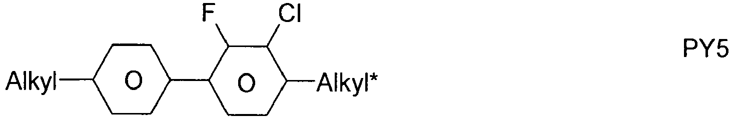 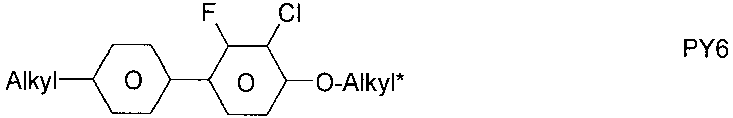 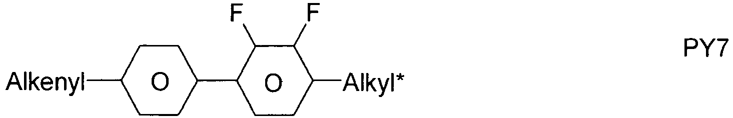 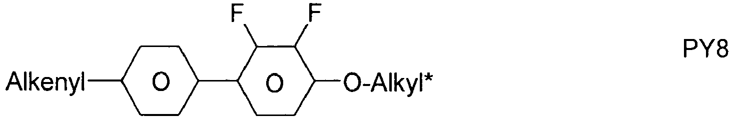 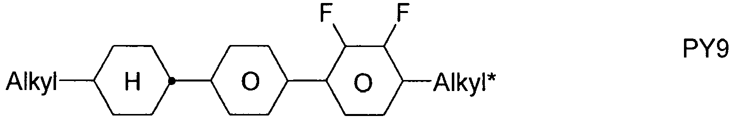 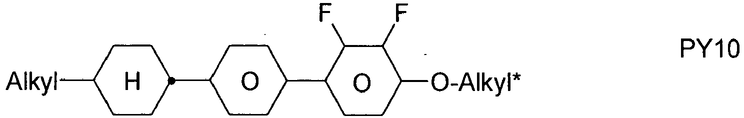 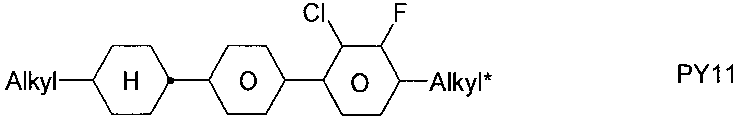 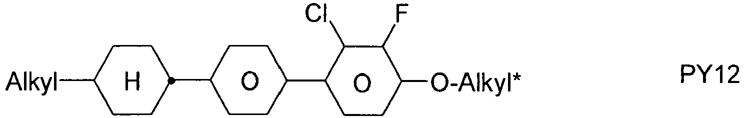 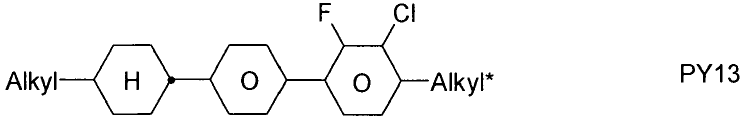 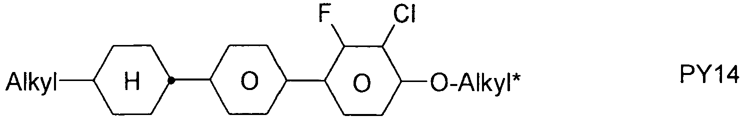 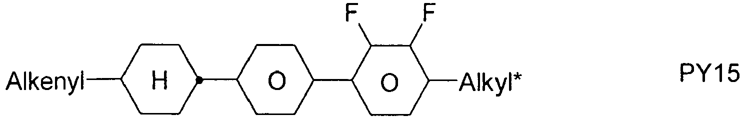 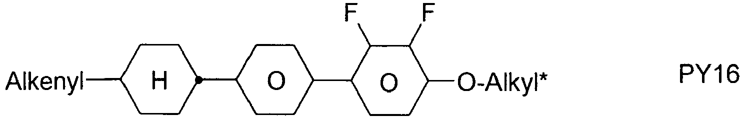 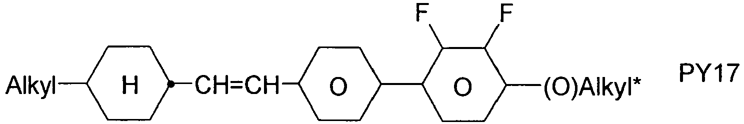 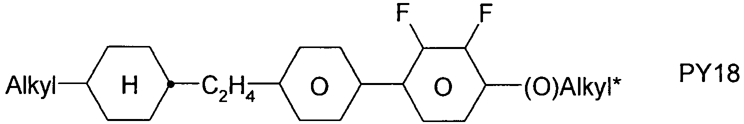 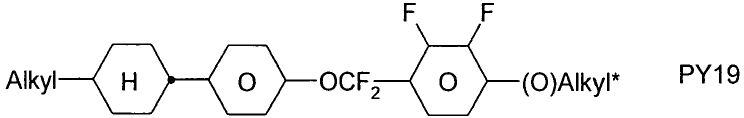 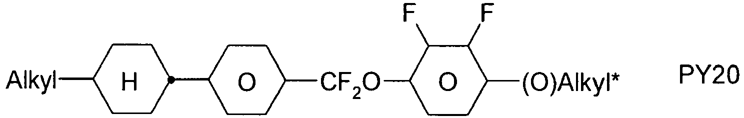 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich eine oder mehrere Verbindungen der folgenden Formel enthält: 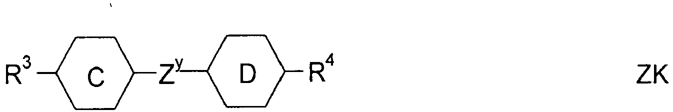 worin die einzelnen Reste folgende Bedeutung besitzen 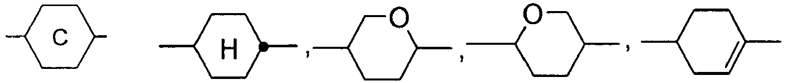 oder 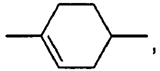 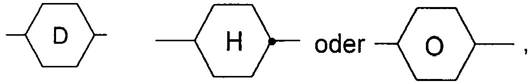
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-. -OCH₂-, -CO-O-, -O-CO-, -C₂F₄-, -CF=CF-, -CH=CH-CH₂O-, oder eine Einfachbindung, vorzugsweise eine Einfachbindung.

Die Verbindungen der Formel ZK sind vorzugsweise ausgewählt aus der Gruppe bestehend aus folgenden Unterformeln: 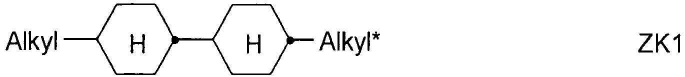 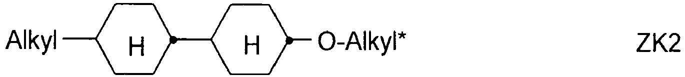 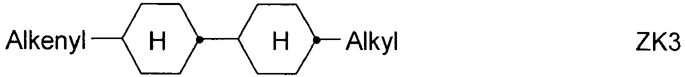 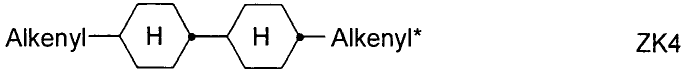 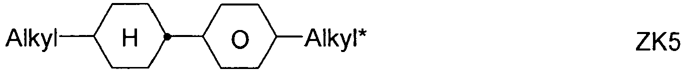 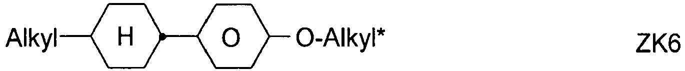 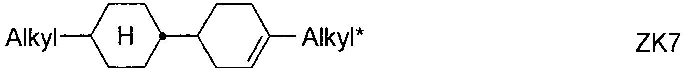 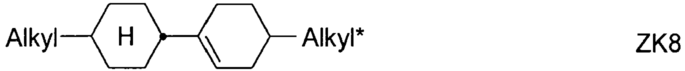 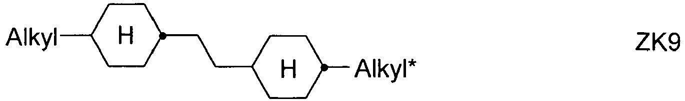 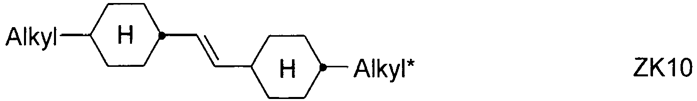 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 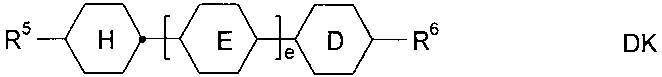 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen, 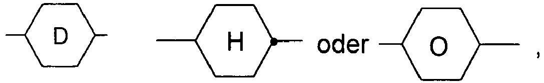 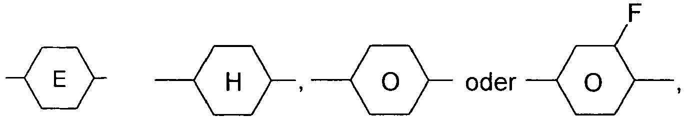
   und
   - e: 1 oder 2.

Die Kombination von Verbindungen der oben genannten bevorzugten Ausführungsformen a)-c) mit den oben beschriebenen polymerisierten Verbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die schnelle Einstellung eines besonders niedrigen "pretilt"-Wnkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Erfindungsgemäße FK-Medien zur Verwendung in Anzeigen des PSA-VA-Typs weisen eine negative dielektrische Anisotropie Δε auf, vorzugsweise von etwa -0,5 bis -10, insbesondere von etwa -2,5 bis -7,5 bei 20°C und 1 kHz.

In erfindungsgemäßen Anzeigen des VA-Typs sind die Moleküle in der Schicht des FK-Mediums im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle mit den Moleküllängsachsen parallel zu den Elektrodenflächen statt.

Die Doppelbrechung Δn in erfindungsgemäßen FK-Medien zur Verwendung in Anzeigen des VA-Typs liegt vorzugsweise unter 0,16, besonders bevorzugt zwischen 0,06 und 0,14, insbesondere zwischen 0,07 und 0,12.

Die erfindungsgemäßen FK-Medien können auch weitere, dem Fachmann bekannte und in der Literatur beschriebene Zusätze oder Additive enthalten, wie beispielsweise Polymerisationsinitiatoren, Inhibitoren, Stabilisatoren, oberflächenaktive Substanzen oder chirale Dotierstoffe. Diese können polymerisierbar oder unpolymerisierbar sein. Polymerisierbare Additive werden dementsprechend der polymerisierbaren Komponente zugerechnet. Unpolymerisierbare Additive werden dementsprechend der flüssigkristallinen Komponente zugerechnet.

Die FK-Medien können beispielsweise einen oder mehrere chirale Dotierstoffe enthalten, vorzugsweise solche ausgewählt aus der Gruppe bestehend aus Verbindungen der nachfolgenden Tabelle B.

Ferner können den FK-Medien beispielsweise 0 bis 15 Gew.-% pleochroitische Farbstoffe zugesetzt werden, ferner Nanopartikel, Leitsalze, vorzugsweise Ethyl-dimethyldodecylammonium-4-hexoxybenzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der bevorzugten Ausführungsformen a)-c) der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen PSA-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

**Tabelle A**

| | |
|---|---|
| 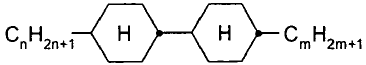 | 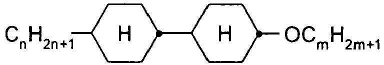 |
| CCH-nm | CCH-nOm |
| 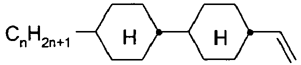 | 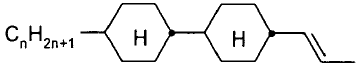 |
| CC-n-V | CC-n-V1 |
| 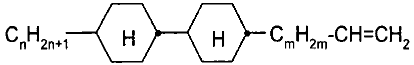 | 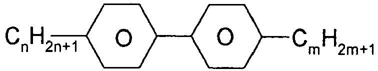 |
| CC-n-mV | PP-n-m |
| 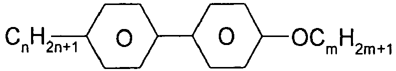 | 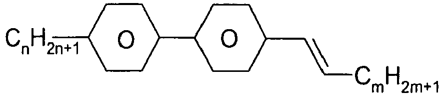 |
| PP-n-Om | PP-n-Vm |
| 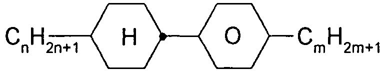 | 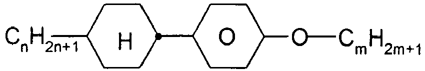 |
| PCH-nm | PCH-nOm |
| 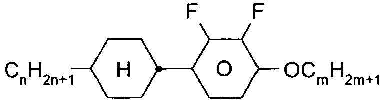 | 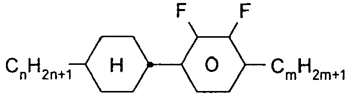 |
| CY-n-Om | CY-n-m |
| 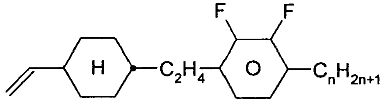 | 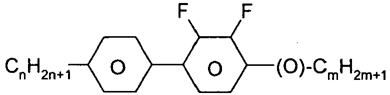 |
| CEY-V-m | PY-n-(O)m |
| 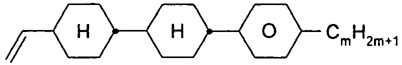 | 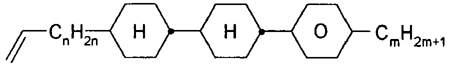 |
| CCP-V-m | CCP-Vn-m |
| 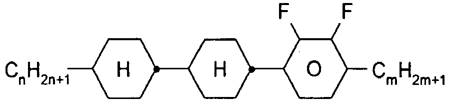 | 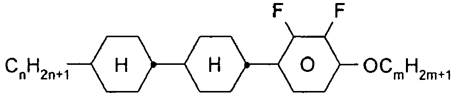 |
| CCY-n-m | CCY-n-Om |
| 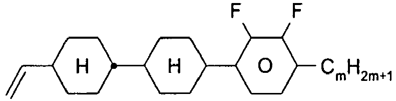 | 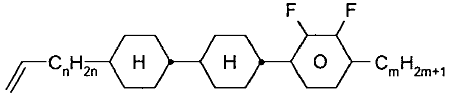 |
| CCY-V-m | CCY-Vn-m |
| 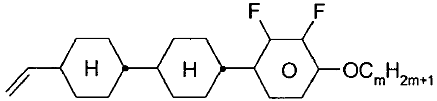 | 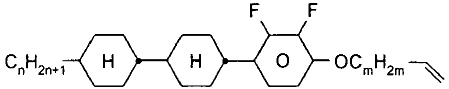 |
| CCY-V-Om | CCY-n-OmV |
| 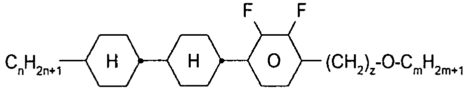 | 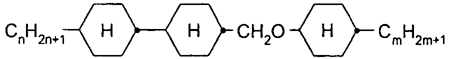 |
| CCY-n-zOm | CCOC-n-m |
| 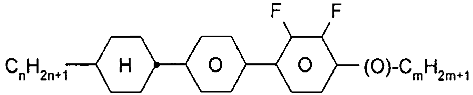 | 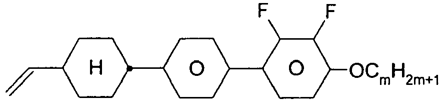 |
| CPY-n-(O)m | CPY-V-Om |
| 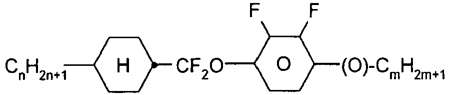 | 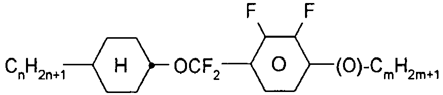 |
| CQY-n-(O)m | CQIY-n-(O)m |
| 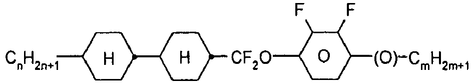 | 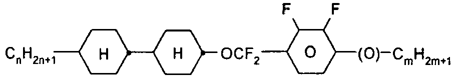 |
| CCQY-n-(O)m | CCQIY-n-(O)m |
| 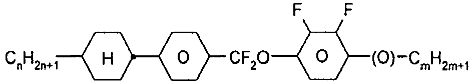 | 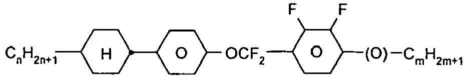 |
| CPQY-n-(O)m | CPQIY-n-Om |
| 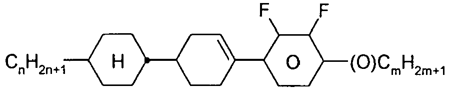 | 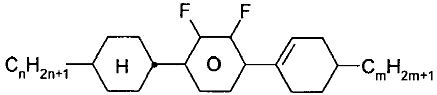 |
| CLY-n-(O)m | CYLI-n-m |
| 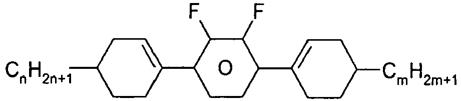 | 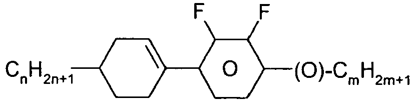 |
| LYLI-n-m | LY-n-(O)m |
| 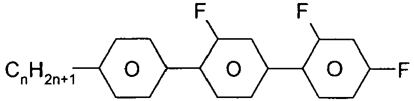 | 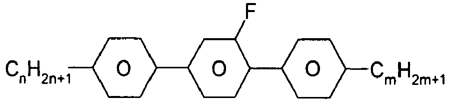 |
| PGIGI-n-F | PGP-n-m |
| 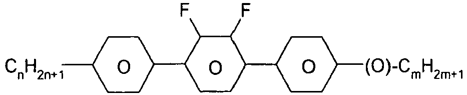 | 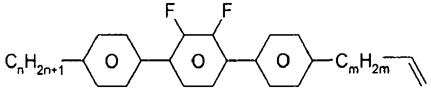 |
| PYP-n-(O)m | PYP-n-mV |
| 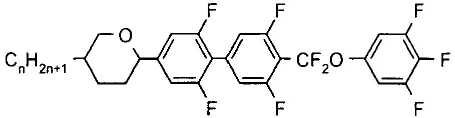 | 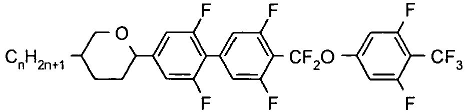 |
| AUUQU-n-F | AUUQU-n-T |
| 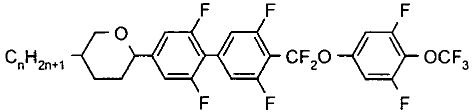 | 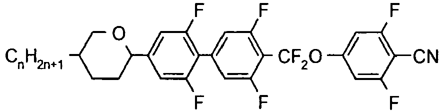 |
| AUUQU-n-OT | AUUQU-n-N |
| 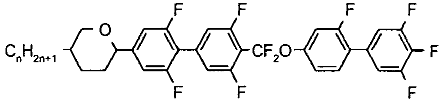 | 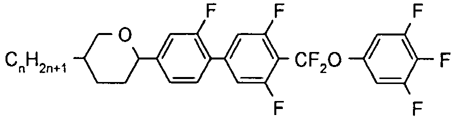 |
| AUUQGU-n-F | AGUQU-n-F |
| 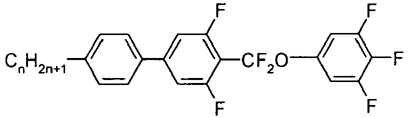 | 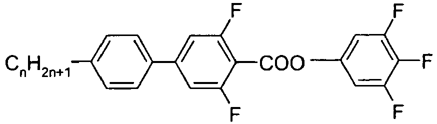 |
| PUQU-n-F | PUZU-n-F |

In einer bevorzugten Ausführungsform der vorliegenden Erfindung enthalten die erfindungsgemäßen FK-Medien eine oder mehrere Verbindungen ausgewählt aus der Gruppe bestehend aus Verbindungen der Tabelle A.

**Tabelle B**

| | |
|---|---|
| In der Tabelle B werden mögliche chirale Dotierstoffe angegeben, die den erfindungsgemäßen FK-Medien zugesetzt werden können. | |
| 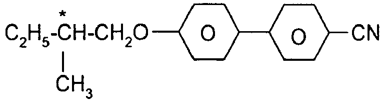 | 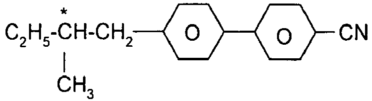 |
| **C 15** | **CB 15** |
| 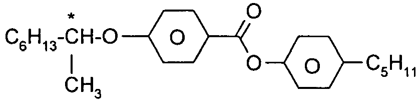 | 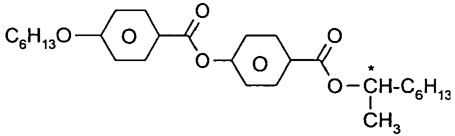 |
| **CM 21** | **R/S-811** |
| 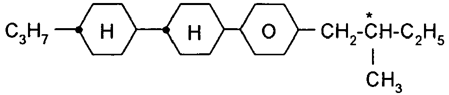 | 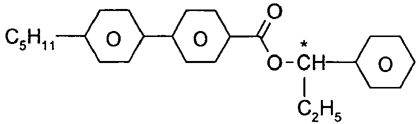 |
| **CM 44** | **CM 45** |
| 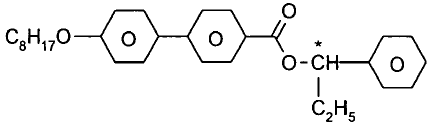 | 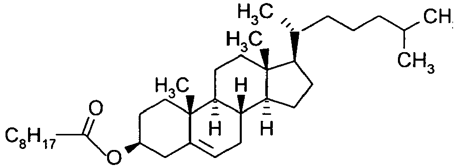 |
| **CM 47** | **CN** |
| 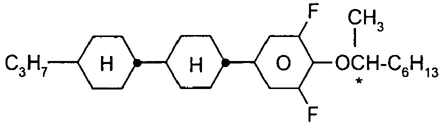 | 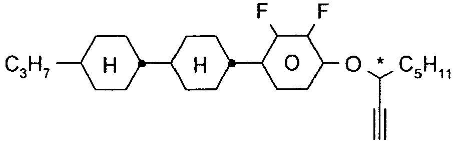 |
| **R**/**S**-**2011** | **R**/**S**-**3011** |
| 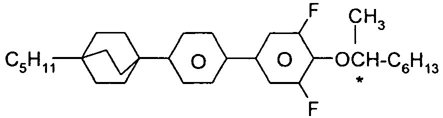 | 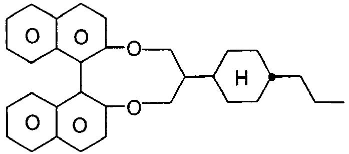 |
| **R/S-4011** | **R/S-5011** |
| 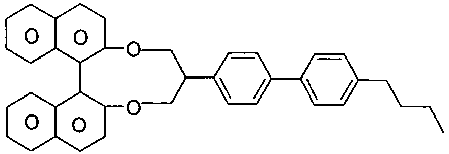 | |
| **R/S** | |
| 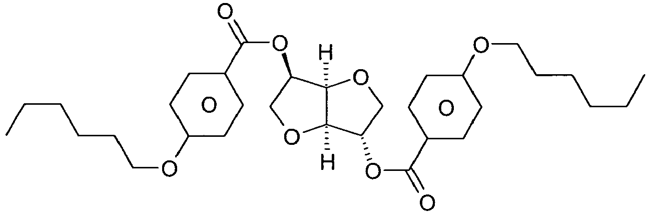 | |
| **R/S** | |
| 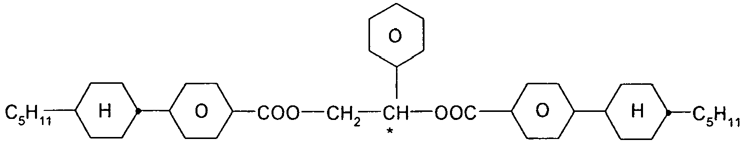 | |
| **R/S-1011** | |

Vorzugsweise enthalten die FK-Medien 0 bis 10 Gew.%, insbesondere 0,01 bis 5 Gew.%, besonders bevorzugt 0,1 bis 3 Gew.% an Dotierstoffen. Vorzugsweise enthalten die FK-Medien einen oder mehrere Dotierstoffe ausgewählt aus der Gruppe bestehend aus Verbindungen der Tabelle B.

**Tabelle C**

| | |
|---|---|
| In der Tabelle C werden mögliche Stabilisatoren angegeben, die den erfindungsgemäßen FK-Medien zugesetzt werden können. (n bedeutet hier eine ganze Zahl von 1 bis 12, vorzugsweise 1, 2, 3, 4, 5, 6, 7 oder 8, endständige Methylgruppen sind nicht gezeigt). | |
| 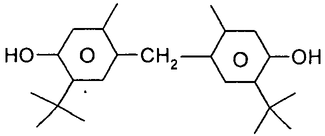 | 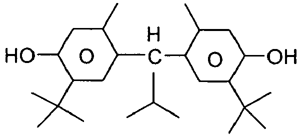 |
| 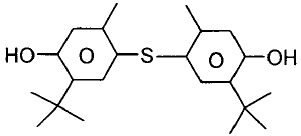 | 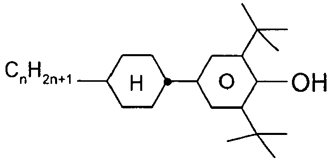 |
| 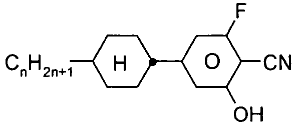 | 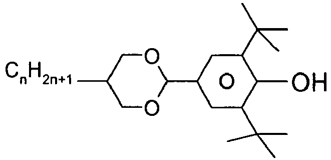 |
| 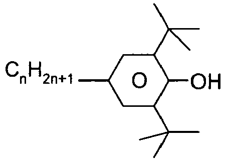 | 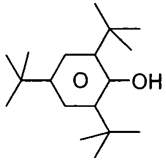 |
| 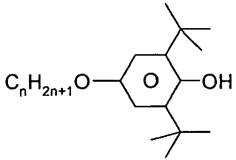 | 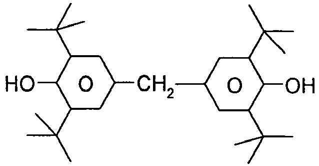 |
| 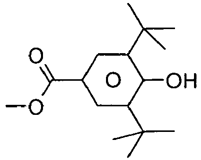 | 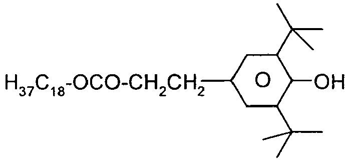 |
| 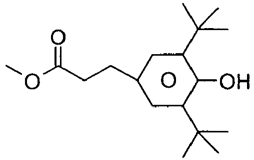 | 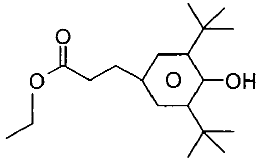 |
| 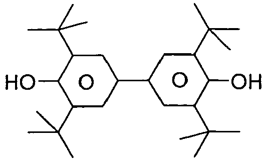 | 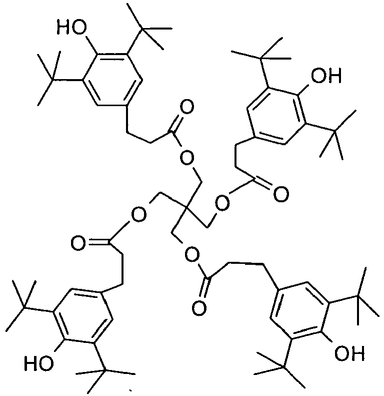 |
| 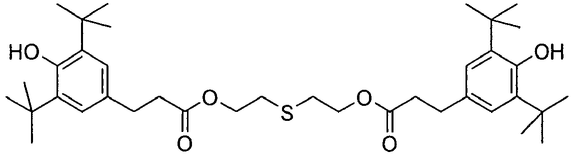 | |
| 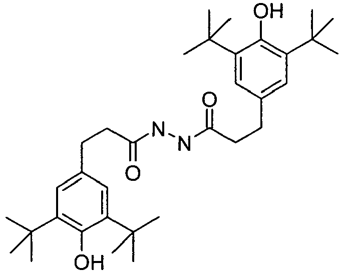 | 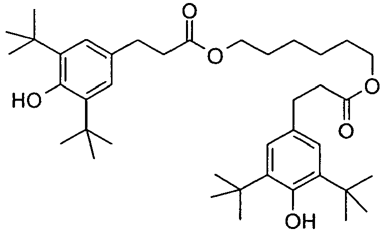 |
| 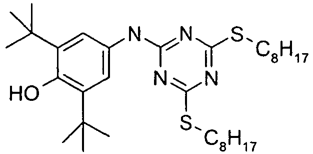 | 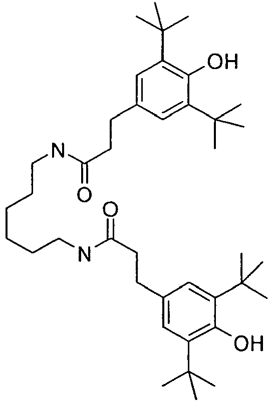 |
| 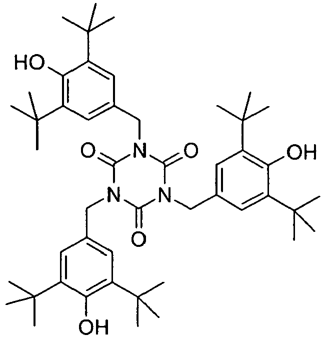 | 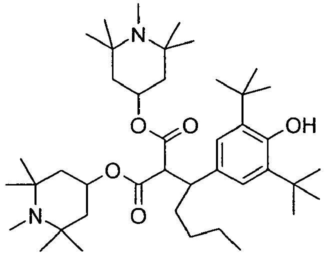 |
| 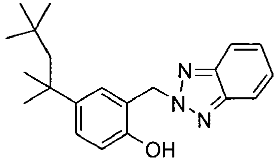 | 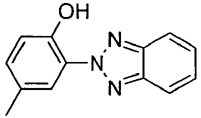 |
| 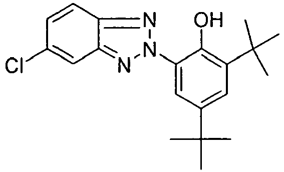 | 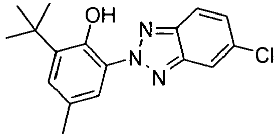 |
| 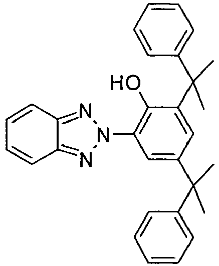 | 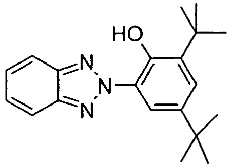 |
| 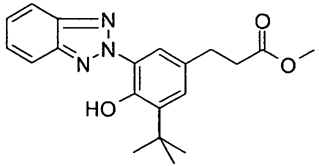 | 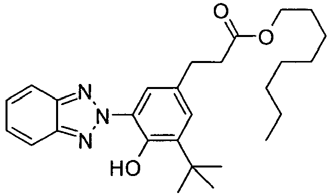 |
| 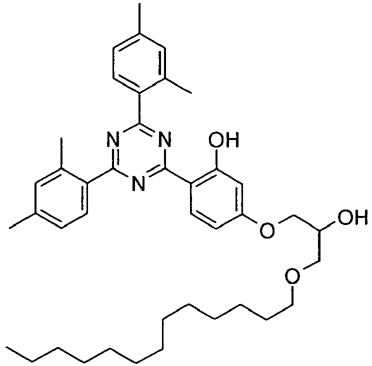 | 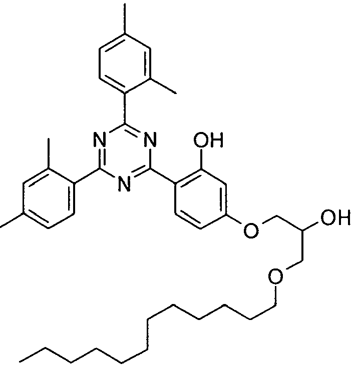 |
| 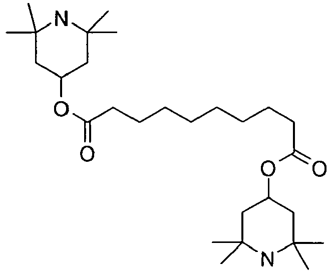 | 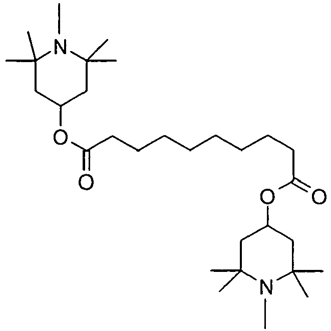 |

Vorzugsweise enthalten die FK-Medien 0 bis 10 Gew.%, insbesondere 1 ppm bis 5 Gew.%, besonders bevorzugt 1 ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle C.

**Tabelle D**

| | |
|---|---|
| In der Tabelle E sind Beispielverbindungen zusammengestellt, die in den FK-Medien gemäß der vorliegenden Erfindung vorzugsweise als reaktive mesogene Verbindungen verwendet werden können. | |
| 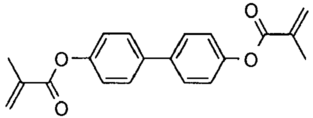 | 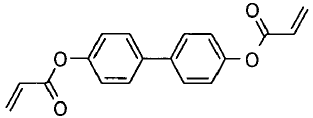 |
| **RM-1** | **RM-2** |
| 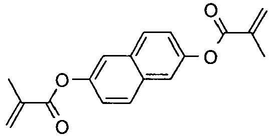 | 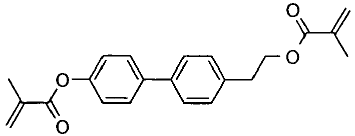 |
| **RM-3** | **RM-4** |
| 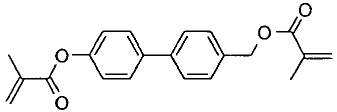 | 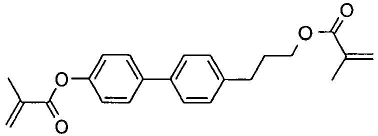 |
| **RM-5** | **RM-6** |
| 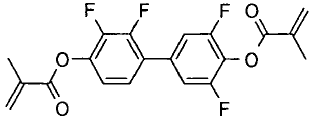 | |
| **RM-7** | |
| 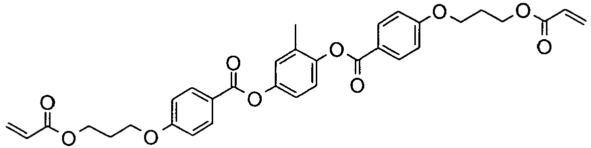 | |
| **RM-8** | |
| 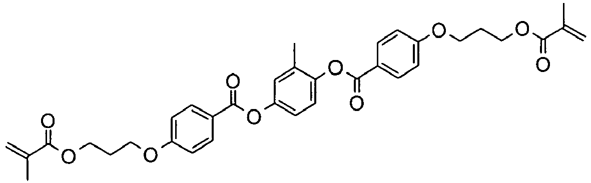 | |
| **RM-9** | |
| 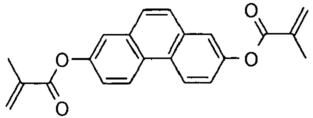 | 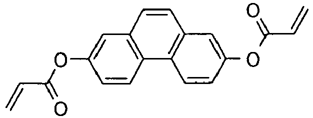 |
| **RM-10** | **RM-11** |
| 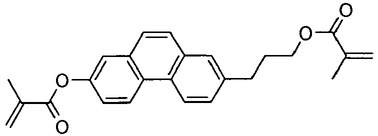 | 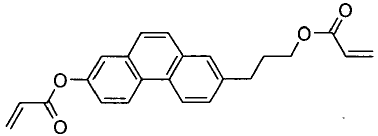 |
| **RM-12** | **RM-13** |
| 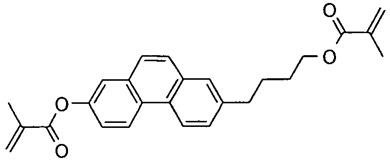 | |
| **RM-14** | |
| 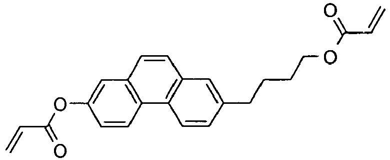 | |
| **RM-15** | |
| 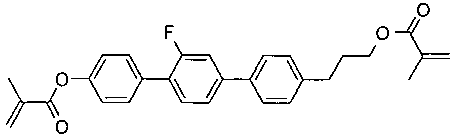 | |
| **RM-16** | |
| 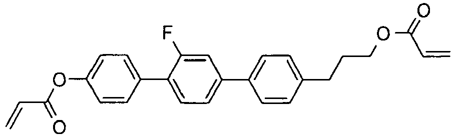 | |
| **RM-17** | |
| 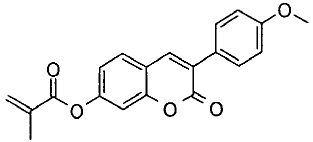 | 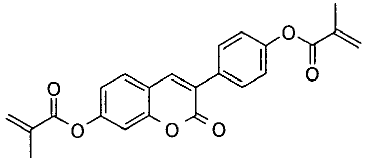 |
| **RM-18** | **RM-19** |
| 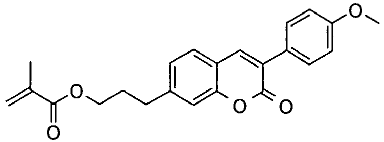 | |
| **RM-20** | |
| 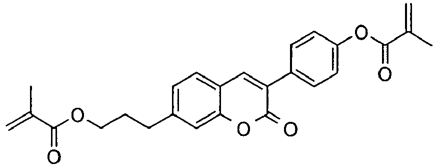 | |
| **RM-21** | |
| 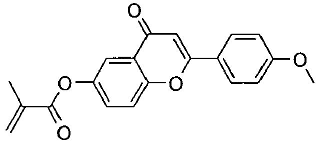 | 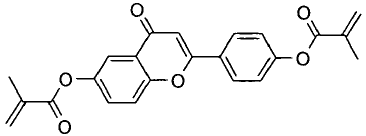 |
| **RM-22** | **RM-23** |
| 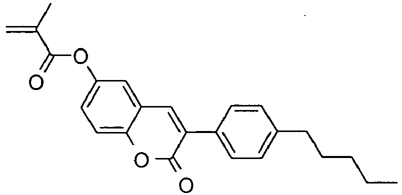 | |
| **RM-24** | |
| 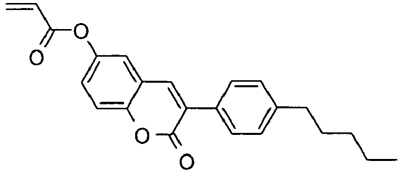 | |
| **RM-25** | |
| 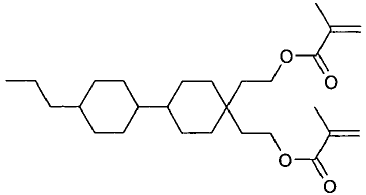 | |
| **RM-26** | |
| 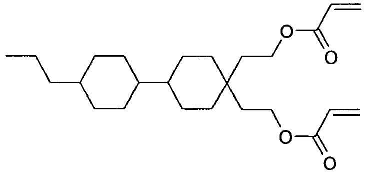 | |
| **RM-27** | |
| 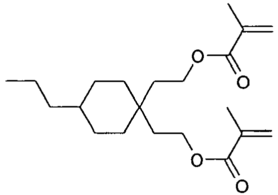 | |
| **RM-28** | |
| 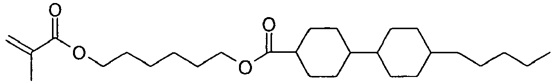 | |
| **RM-29** | |
| 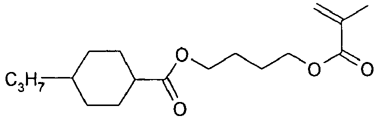 | |
| **RM-30** | |
| 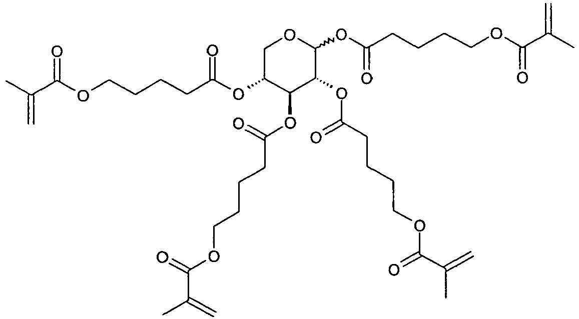 | |
| **RM-31** | |
| 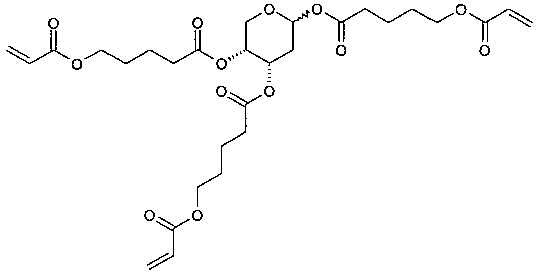 | |
| **RM-32** | |
| 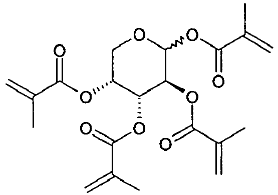 | |
| **RM-33** | |

In einer bevorzugten Ausführungsform der vorliegenden Erfindung enthalten die mesogenen Medien eine oder mehrere Verbindungen ausgewählt aus der Gruppe der Verbindungen der Tabelle D.

Außerdem werden folgende Abkürzungen und Symbole verwendet:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε_{∥}: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten Tg = Glaszustand, K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.
Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 50 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d ≈ 6 µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

Zur Untersuchung der Tieftemperaturstabilität, auch als "LTS" (low temperature stability) bezeichnet, d.h. der Stabilität der FK-Mischung gegen spontane Auskristallisation einzelner Komponenten bei tiefen Temperaturen, werden Fläschchen mit 1 g FK/RM-Mischung bei -10°C eingelagert und es wird regelmäßig überprüft, ob die Mischungen auskristallisiert waren.

Die sogenannte "HTP" ("helical twisting power") bezeichnet das helikale Verdrillungsvermögen einer optisch aktiven bzw. chiralen Substanz in einem FK-Medium (in µm). Falls nicht anders angegeben, wird die HTP in der kommerziell erhältlichen nematischen FK-Hostmischung MLD-6260 (Merck KGaA) bei einer Temperatur von 20°C gemessen.

### Beispiele

### Beispiel 1. Acrylsäure-6-(4-{[4-(6-acryloyloxy-hexyl)-phenoxy]-difluormethyl}-3,5-difluor-phenyl)-hexylester

### 1.1 5-Brom-2-[(4-bromphenoxy)-difluormethyl]-1,3-difluorbenzol

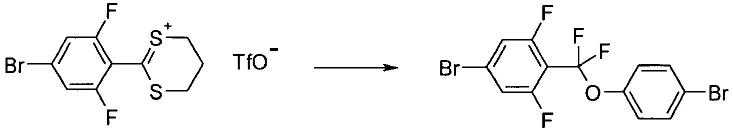

92,0 g (0,200 mol) 2-(4-Brom-2,6-difluorphenyl)-5,6-dihydro-4H-[1,3]dithiin-1-yliumtriflat werden in 600 ml Dichlormethan vorgelegt und bei -70 °C mit einer Lösung von 52,0 g (0,300 mol) 4-Bromphenol in 200 ml Dichlormethan und 45 ml Triethylamin versetzt. Nach beendeter Zugabe wird 1 h bei -70 °C nachgerührt, mit 160 ml (1,00 mol) Triethylamintrishydrofluorid versetzt und anschließend eine Lösung von 51,0 ml (0,996 mol) Brom in 200 ml Dichlormethan zutropfen gelassen. Nach 1 h wird die Kühlung entfernt und der Ansatz nach dem Erwärmen auf -10 °C auf eine Lösung von 310 ml 32proz. Natronlauge in 2 I Eiswasser gegeben. Die org. Phase wird abgetrennt und mit Wasser gewaschen. Die wäßrige Phase wird mit Dichlormathan extrahiert und die vereinigten org. Phasen werden über Natriumsulfat getrocknet. Das Lösungsmittel wird i. Vak. entfernt und der Rückstand mit Heptan über Kieselgel filtriert. Man erhält 5-Brom-2-[(4-bromphenoxy)-difluormethyl]-1,3-difluorbenzol als gelbes Öl.
¹⁹F-NMR (CDCl₃, 235 MHz)
δ = -63,1 ppm (t, J = 26,7 Hz, 2 F, -CF₂O-) -112 (dt, J = 9,7 Hz, J = 26,7 Hz, 2 F, Ar-F).

### 1.2 6-(4-{Difluor-[4-(6-hydroxyhex-1-inyl)-phenoxy]-methyl}-3,5-difluorphenyl)-hex-5-in-1-ol

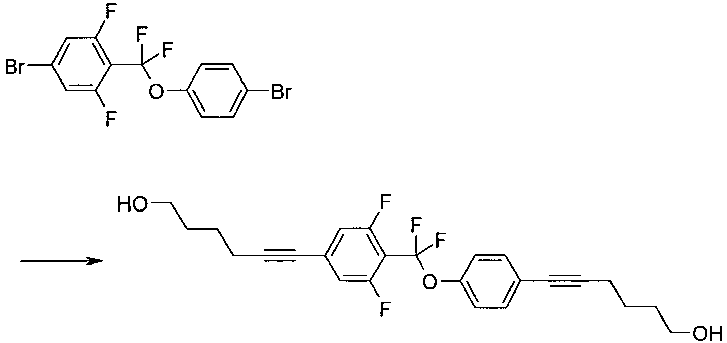

10,7 g (25,8 mmol) 5-Brom-2-[(4-bromphenoxy)-difluormethyl]-1,3-difluorbenzol und 8,00 g (81,5 mmol) Hex-5-in-1-ol werden in 11,3 ml Triethylamin und 500 ml Toluol vorgelegt, mit 1,50 g (2 mmol) Bis(triphenylphosphin)palladium(II)chlorid und 0,700 g (3,68 mmol) Kupfer(I)iodid versetzt und über Nacht unter Rückfluß erhitzt. Anschließend wird der Ansatz auf Wasser gegeben, mit 2 N Salzsäure neutralisiert und dreimal mit Toluol extrahiert. Die vereinigten org. Phasen werden über Natriumsulfat getrocknet, das Lösungsmittel i.Vak. entfernt und der Rückstand zunächst mit Toluol und dann mit Toluol/Essigester (4:1) an Kieselgel chromatographiert. Man erhält 6-(4-{Difluor-[4-(6-hydroxyhex-1-inyl)-phenoxy]-methyl}-3,5-difluorphenyl)-hex-5-in-1-ol als farblosen Feststoff.

### 1.3 6-(4-{Difluor-[4-(6-hydroxyhexyl)-phenoxy]-methyl}-3,5-difluorphenyl)-hexan-1-ol

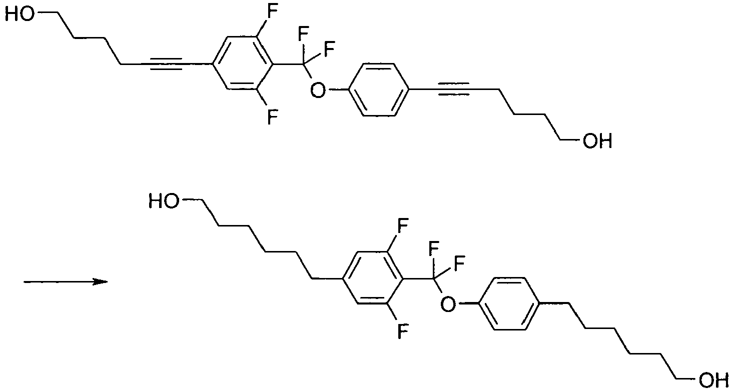

6-(4-{Difluor-[4-(6-hydroxy-hex-1-inyl)-phenoxy]-methyl}-3,5-difluorphenyl)-hex-5-in-1-ol werden in THF an Palladium-Aktivkohlekatalysator bis zum Stillstand hydriert. Der Katalysator wird abfiltriert, das Lösungsmittel i. Vak. entfernt und das Rohprodukt mit Toluol/Essigester (1:2) an Kieselgel chromatographiert. Man erhält 6-(4-{Difluor-[4-(6-hydroxy-hexyl)-phenoxy]-methyl}-3,5-difluorphenyl)-hexan-1-ol als farblosen Feststoff.
¹⁹F-NMR (CDCl₃, 235 MHz):
δ = -60,8 ppm (t, J = 26,3 Hz, 2 F, -CF₂O-), -112 (dt, J = 10,0 Hz, J = 26,3 Hz, 2 F, Ar-F).

### 1.4 Acrylsäure-6-(4-{[4-(6-acryloyloxy-hexyl)-phenoxy]-difluormethyl}-3,5-difluor-phenyl)-hexylester

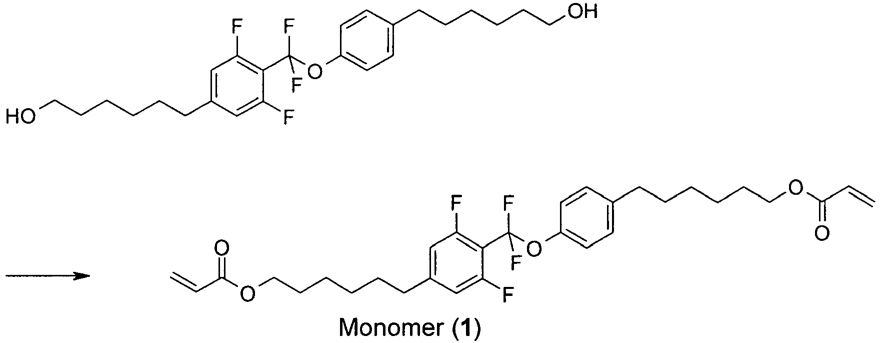

17,0 g (37,2 mmol) 6-(4-{Difluor-[4-(6-hydroxy-hexyl)-phenoxy]-methyl}-3,5-difluorphenyl)-hexan-1-ol, 8,05 g (112 mmol) Acrylsäure und 0,5 g DMAP werden in 300 ml Dichlormethan vorgelegt und unter Eiskühlung eine Lösung von 17,3 g (112 mmol) EDC in 75 ml Dichlormethan zutropfen gelassen. Nach 1 h wird die Kühlung entfernt und der Ansatz über Nacht bei Raumtemp. rühren gelassen. Das Lösungsmittel wird i.Vak. weitestgehend entfernt und der Rückstand mit Dichlormethan an Kieselgel chromatographiert. Man erhält den Acrylsäure-6-(4-{[4-(6-acryloyloxy-hexyl)-phenoxy]-difluormethyl}-3,5-difluor-phenyl)-hexylester als farbloses Öl.
Phasenverhalten Tg -71 K 13 I
¹H-NMR(CDCl₃, 250 MHz):
δ = 1,25 - 1,48 ppm (m, 8 H, CH₂), 1,50 - 1,74 ppm (m, 8 H, CH₂), 2,60 (m, 4 H, 2 -Ar-CH₂-), 4,13 (t, J = 6,7 Hz, 2 H, -C*H*₂O-), 4,15 (t, J = 6,7 Hz, 2 H, -C*H*₂O-), 5,81 (dt, J = 10,4 Hz, J = 1,8 Hz, 2 H, 2 C*H*H=CH-COO-), 6,11 (m_{c}, 2 H, 2 CH₂=C*H*-COO-), 6,39 (2 CH*H*=CH-COO-), 6,78 (d, J = 10,0 Hz, 2 H, Ar-H), 7,15 (m_{c}, 4 H, Ar-H).
¹⁹F-NMR(CDCl₃, 235 MHz)
δ = -60,9 ppm (t, J = 26,4 Hz, 2 F, -CF₂O-), -112,0 (dt, J = 26,4, J = 10,0 Hz, 2 F, Ar-F).

### Beispiel 2. 2-Methyl-acrylsäure-6-[4'-(difluor-{4-[6-(2-methyl-acryloyloxy)-hexyl]-phenoxy}-methyl)-2,3',5'-trifluorbiphenyl-4-yl]-hexylester

### 2.1 2-(4'-Brom-3,5,2'-trifluor-biphenyl-4-yl)-5,6-dihydro-4H-[1,3]dithiin-1-yliumtriflat

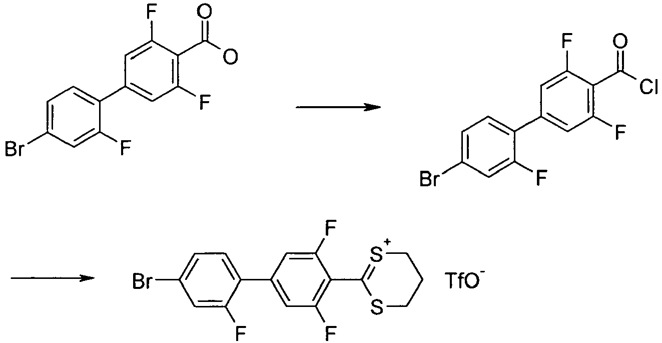

10,4 g (30,1 mmol) 4'-Brom-3,5,2'-trifluorbiphenyl-4-carbonsäure (CAS-Nr. 1196677-06-1) werden mit 4,4 ml (61 mmol) Thionylchlorid 2 h auf 90 °C erhitzt. Nach dem Erkalten wird überschüssiges Thionylchlorid i. Vak. entfernt, unter Eiskühlung 3,2 ml (30 mmol) 1,3-Propandithiol in wenig Dichlormethan hinzugefügt und nach 1 h 8,1 ml (92,3 mmol) Trifluormethansulfonsäure zutropfen gelassen. Nach beendeter Zugabe werden 20 ml Acetanhydrid vorsichtig hinzugegeben und 200 ml Ether hinzugefügt. Nach 30 min wird der Ansatz auf -60 °C gekühlt, das ausgefallene Produkt abgesaugt, mit Ether gewaschen und i. Vak. getrocknet. Man erhält 2-(4'-Brom-3,5,2'-trifluor-biphenyl-4-yl)-5,6-dihydro-4H-[1,3]dithiin-1-yliumtriflat als gelben Feststoff, der ohne weitere Aufreinigung in der nächsten Stufe eingesetzt wird.

### 2.2 4'-Brom-4-[difluor-(4-iodphenoxy)-methyl]-3,5,2'-trifluorbiphenyl

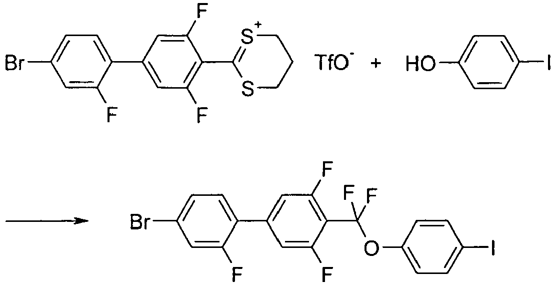

25,8 g (46,6 mmol) 2-(4'-Brom-3,5,2'-trifluor-biphenyl-4-yl)-5,6-dihydro-4H-[1,3]dithiin-1-yliumtriflat werden in 200 ml Dichlormethan vorgelegt und bei -70 °C mit einer Lösung von 15,0 g (68,2 mmol) 4-lodphenol in 50 ml Dichlormethan und 12 ml Triethylamin versetzt. Nach beendeter Zugabe wird 1 h bei -70 °C nachgerührt, mit 38,0 ml (0,236 mol) Triethylamintrishydrofluorid versetzt und anschließend eine Lösung von 12,0 ml (0,234 mol) Brom in 100 ml Dichlormethan zutropfen gelassen. Nach 1 h wird die Kühlung entfernt und der Ansatz nach dem Erwärmen auf -10°C auf eine Lösung von 75 ml 32proz. Natronlauge in 500 ml Eiswasser gegeben. Die org. Phase wird abgetrennt und mit Wasser gewaschen. Die wäßrige Phase wird mit Dichlormethan extrahiert und die vereinigten org. Phasen werden über Natriumsulfat getrocknet. Das Lösungsmittel wird i. Vak. entfernt, der Rückstand mit Heptan über Kieselgel filtriert und aus Ethanol umkristallisiert. Man erhält 4'-Brom-4-[difluor-(4-iodphenoxy)-methyl]-3,5,2'-trifluorbiphenyl als gelbes Öl.
¹⁹F-NMR (CDCl₃, 235 MHz)
δ = -59,2 ppm (t, J = 26,4 Hz, 2 F, -CF₂O-), -108 (dt, J = 9,8 Hz, J = 26,4 Hz, 2 F, Ar-F), - 113 (dt, J = 9,1 Hz, J = 10,2 Hz, 1 F, Ar-F).

### 2.3 6-(4'-{Difluor-[4-(6-hydroxyhex-1-inyl)-phenoxy]-methyl}-2,3',5'-trifluorbiphenyl-4-yl)-hex-5-in-1-ol

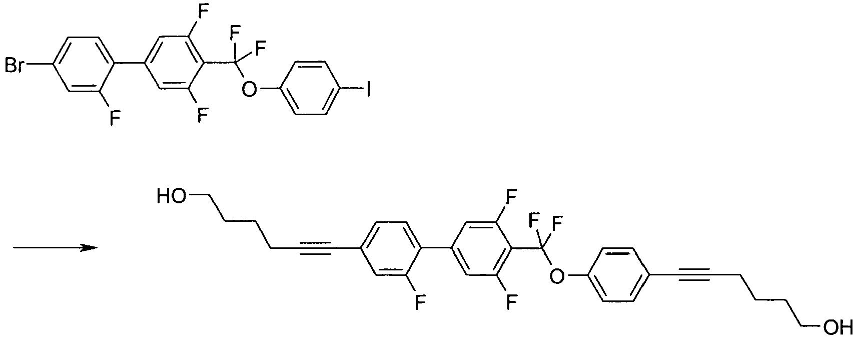

6,00 g (10,8 mmol) 4'-Brom-4-[difluor-(4-iodphenoxy)-methyl]-3,5,2'-trifluorbiphenyl und 4,00 g (40,8 mmol) Hex-5-in-1-ol werden in 6 ml Triethylamin und 300 ml Toluol vorgelegt, mit 0,700 g (1,00 mmol) Bis(triphenylphosphin)palladium(II)chlorid und 0,100 g (0,525 mmol) Kupfer(I)iodid versetzt und über Nacht unter Rückfluß erhitzt. Anschließend wird der Ansatz auf Wasser gegeben, mit 2 N Salzsäure neutralisiert und dreimal mit Toluol extrahiert. Die vereinigten org. Phasen werden über Natriumsulfat getrocknet, das Lösungsmittel i.Vak. entfernt und der Rückstand zunächst mit Toluol und dann mit Toluol/Essigester (4:1) an Kieselgel chromatographiert. Man erhält 6-(4-{Difluor-[4-(6-hydroxyhex-1-inyl)-phenoxy]-methyl}-3,5-difluorphenyl)-hex-5-in-1-ol als farblosen Feststoff.

### 2.4 6-(4'-{Difluor-[4-(6-hydroxyhexyl)-phenoxy]-methyl)-2,3',5'-trifluorbiphenyl-4-yl)-hexan-1-ol

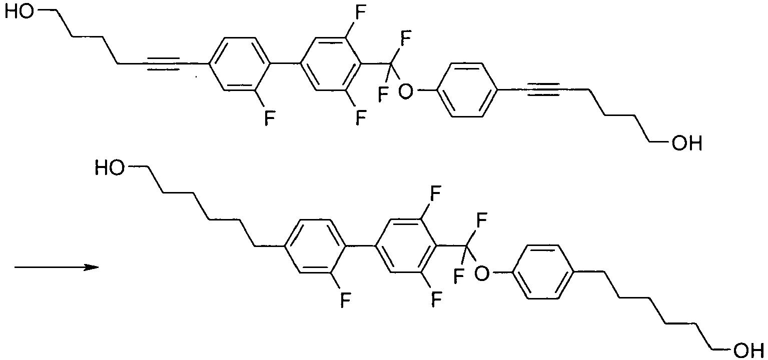

6-(4-{Difluor-[4-(6-hydroxyhex-1-inyl)-phenoxy]-methyl}-3,5-difluorphenyl)-hex-5-in-1-ol wird in THF an Palladium-Aktivkohlekatalysator bis zum Stillstand hydriert. Der Katalysator wird abfiltriert, das Lösungsmittel i. Vak. entfernt und das Rohprodukt ohne weitere Aufreinigung in der nächsten Stufe eingesetzt.

### 2.5 2-Methyl-acrylsäure-6-[4'-(difluor-{4-[6-(2-methyl-acryloyloxy)-hexyl]-phenoxy}-methyl)-2,3',5'-trifluorbiphenyl-4-yl]-hexylester

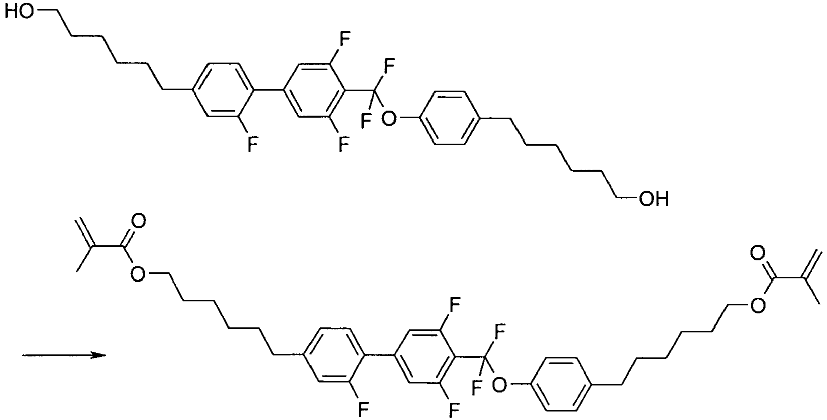

1,00 g (1,82 mmol) 6-(4'-{Difluor-[4-(6-hydroxy-hexyl)-phenoxy]-methyl}-2,3',5'-trifluorbiphenyl-4-yl)-hexan-1-ol werden in 15 ml Dichlormethan unter Eiskühlung vorgelegt, mit 1 ml (7,21 mmol) Triethylamin versetzt und eine Lösung von 0,700 g (6,73 mmol) Methacrylsäurechlorid in 5 ml Dichlormethan zutropfen gelassen. Der Ansatz wird über Nacht rühren gelassen, das Lösungsmittel i. Vak. entfernt und der Rückstand durch Chromatographie an Kieselgel mit Toluol/Dichlormethan (1:1) gereinigt. Man erhält den 2-Methyl-acrylsäure-6-[4'-(difluor-{4-[6-(2-methyl-acryloyloxy)-hexyl]-phenoxy}-methyl)-2,3',5'-trifluorbiphenyl-4-yl]-hexylester als fabloses Öl. Phasenverhalten Tg -59 N -28,5 I.
¹H-NMR (CDCl₃, 250 MHz):
δ = 1,24 - 1,48 ppm (m, 8 H, CH₂), 1,55 - 1,79 ppm (m, 8 H, CH₂), 1,94 (m_{c}, 6 H, 2 CH₃) 2,63 (m, 4 H, 2 -Ar-C*H*₂-), 4,13 (t, J = 6,6 Hz, 2 H, - C*H*₂O-), 4,15 (t, J = 6,6 Hz, 2 H, -C*H*₂O-), 5,54 (m_{c}, 2 H, 2 C*H*H=CH-COO-), 6,09 (m_{c}, 2 CH₂=C*H*-COO-), 6,95 - 7,09 (m, 2 H, Ar-H), 7,09 - 7,24 (m, 6 H, Ar-H), 7,32 (t, J = 7,9 Hz, 1 H, Ar-H).
¹⁹F-NMR(CDCl₃, 235 MHz)
δ = -60,9 ppm (t, J = 26,5 Hz, 2 F, -CF₂O-), -111 (dt, J = 10,0, J = 26,4 Hz, 2 F, Ar-F), -118 (dd, J = 8,2 Hz, J = 12,0 Hz, 1 F, Ar-F).

### Beispiel 3. 2-Methyl-acrylsäure-4-[4'-(difluor-{4-[4-(2-methyl-acryloyloxy)-butyl]-phenoxy}-methyl)-2,3',5'-trifluor-biphenyl-4-yl]-butylester

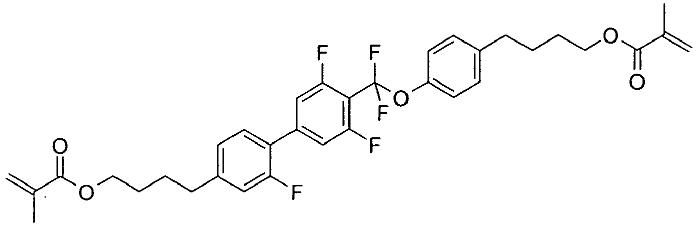

In Analogie zu Beispiel 2 erhält man 2-Methyl-acrylsäure-4-[4'-(difluor-{4-[4-(2-methyl-acryloyloxy)-butyl]-phenoxy}-methyl)-2,3',5'-trifluorbiphenyl-4-yl]-butylester als farblose Kristalle vom Schmp. 128 °C.

### Beispiel 4 Acrylsäure-6-(4-{[4-(6-acryloyloxy-butyl)-phenoxy]-difluormethyl}-3,5-difluor-phenyl)-butylester

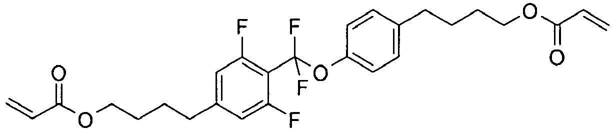

Die Verbindung wir analog zu Beispiel 1 hergestellt. Schmp. 128 °C

### Beispiel 5 Methyl-acrylsäure-6-{4'-(3,4,5-trifluorphenoxy)-difluormethyl]-3',5'-difluor-biphenyl-4-yl}-hexylester

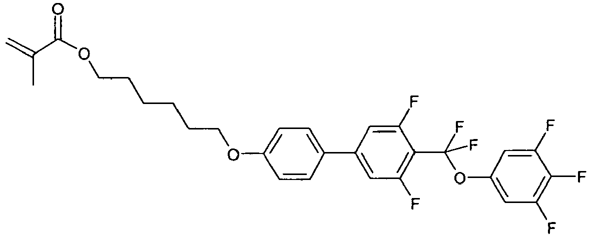

Die Verbindung wir analog zu Beispiel 2 hergestellt. Schmp. 58 °C.

### Beispiel 6 Acrylsäure-6-(4-{[4-(6-acryloyloxy-hexyl)-3,5-difluorphenoxy]-difluormethyl}-3,5-difluor-phenyl)-hexylester

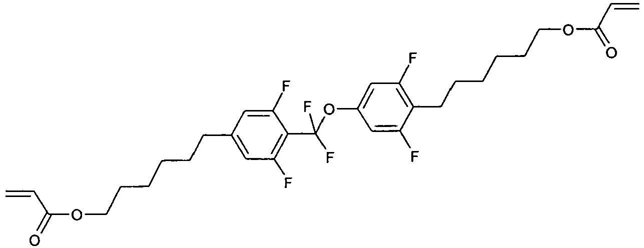

Die Verbindung wir analog zu Beispiel 1 hergestellt.

### Anwendungsbeispiel 1

Die folgenden Monomere werden verwendet:

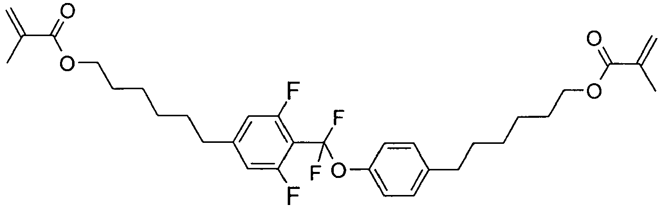

### Monomer (1) aus Beispiel 1

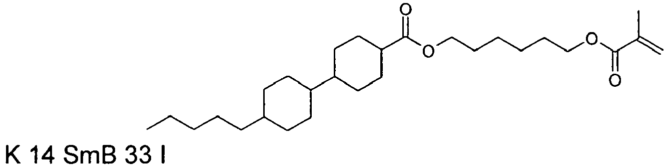

### RM-CC

Die folgenden Zusatzstoffe werden verwendet:

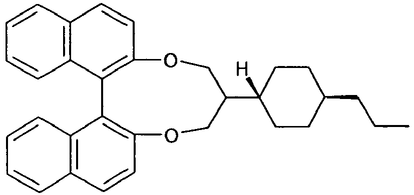

### Dp (chiraler Dotierstoff)

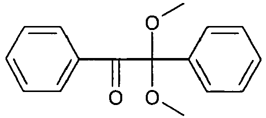

### In (Ciba Irgacure ® 651, Photoinitiator)

Die folgende Basismischung (Host) H1 wird verwendet:

| Zusammensetzung | | Eigenschaften | |
|---|---|---|---|
| Komponente | Anteil | T(N, I): | 71 °C |
| Akronym | Gew.-% | Δn (20°C, 589 nm): | 0,152 |
| AUUQU-2-F | 10 | | |
| AUUQU-3-F | 11 | | |
| AUUQU-4-F | 7 | | |
| AUUQU-5-F | 6 | | |
| AUUQU-7-F | 7 | | |
| AUUQU-3-T | 10 | | |
| AUUQU-3-OT | 11 | | |
| PUZU-2-F | 7 | | |
| PUZU-3-F | 11 | | |
| PUZU-5-F | 11 | | |
| AGUQU-3-F | 4 | | |
| AUUQU-3-N | 5 | | |
| Σ | 100.00 | | |

Aus der Basismischung H1 werden durch Beimischung der oben aufgeführten Monomere und Zusatzstoffe die erfindungsgemäße polymerisierbare FK-Mischung M1, enthaltend das erfindungsgemäße Monomer (1) aus Beispiel 1 hergestellt. Die Zusammensetzungen der Mischungen sind in Tabelle 1 gezeigt.

**Tabelle 1**

| **M1** | |
|---|---|
| **Komponente** | **Anteil [Gew.-%]** |
| H1 | 85 |
| Dp | 3,8 |
| In | 0,2 |
| (1) | 4,5 |
| RM-CC | 6,5 |

Die Mischungen werden vor der Polymerisation wie nachfolgend beschrieben charakterisiert. Danach werden die reaktiven Komponenten durch einmalige Bestrahlung (180 s) in der blauen Phase polymerisiert, und die erhaltenen Medien werden erneut charakterisiert.

### Beschreibung der Polymerisation

Vor der Polymerisation einer Probe werden die Phaseneigenschaften des Mediums in einer Testzelle von ca. 10 Mikrometer Dicke und einer Fläche von 2 x 2,5 cm festgestellt. Die Füllung erfolg durch Kapillarwirkung bei einer Temperatur von 75 °C. Die Messung erfolgt unter einem Polarisationsmikroskop mit Heiztisch bei einem Temperaturverlauf von 1 °C/min.

Die Polymerisation der Medien wird durch Bestrahlung mit einer UV-Lampe (Dymax, Bluewave 200, 365 nm Interferenzfilter) mit einer effektiven Leistung von ca. 3,0 mW/cm² für 180 Sekunden durchgeführt. Die Polymerisation erfolgt direkt in der elektrooptischen Testzelle.

Die Polymerisation erfolgt anfangs bei einer Temperatur, in der das Medium in der blauen Phase I (BP-I) vorliegt. Die Polymerisation erfolgt in mehreren Teilschritten, die nach und nach zu einer vollständigen Polymerisation führen. Der Temperaturbereich der blauen Phase ändert sich in der Regel während der Polymerisation. Zwischen jedem Teilschritt wird daher die Temperatur so angepasst, dass das Medium nach wie vor in der blauen Phase vorliegt. In der Praxis kann dies so erfolgen, dass nach jedem Bestrahlungsvorgang von ca. 5 s oder länger die Probe unter dem Polarisationsmikroskop beobachtet wird. Wird die Probe dunkler, so deutet dies auf einen Übergang in die isoptrope Phase hin. Die Temperatur für den nächsten Teilschritt wird entsprechend verringert.

Die gesamte Bestrahlungszeit, die zu der maximalen Stabilisierung führt, beträgt typischerweise 180 s bei der angegebenen Bestrahlungsleistung. Weitere Polymerisationen können nach einem optimierten Bestrahlungs-Temperatur-Programm durchgeführt werden.

Alternativ kann die Polymerisation auch in einem einzigen Bestrahlungsschritt durchgeführt werden, insbesondere wenn schon vor der Polymerisation eine breite blaue Phase vorliegt.

### Elektrooptische Charakterisierung

Nach der oben beschriebenen Polymerisation und Stabilisierung der blauen Phase wird die Phasenbreite der blauen Phase bestimmt. Die elektrooptische Charakterisierung erfolgt anschließend bei verschiedenen Temperaturen innerhalb und ggf. auch außerhalb dieses Bereichs.

Die verwendeten Testzellen sind auf einer Seite mit Interdigitalelektroden auf der Zellenoberfläche ausgestattet. Der Zellspalt, der Elektrodenabstand und die Elektrodenbreite betragen typischerweise jeweils 10 Mikrometer. Dieses einheitliche Maß wird nachfolgend als Spaltbreite bezeichnet. Die mit Elektroden belegte Fläche beträgt ca. 0,4 cm². Die Testzellen besitzen keine Orientierungsschicht ('alignment layer').

Die Zelle befindet sich für die elektrooptische Charakterisierung zwischen gekreuzten Polarisationsfiltern, wobei die Längsrichtung der Elektroden einen Winkel von 45° mit den Achsen des Polarisationsfilter einnimmt. Die Messung erfolgt mit einem DMS301 (Autronic-Melchers) im rechten Winkel zur Zellebene, oder mittels einer hochempfindlichen Kamera am Polarisationsmikroskop. Im spannungslosen Zustand ergibt die beschriebene Anordnung ein im Wesentlichen dunkles Bild (Definition 0 % Transmission).

Zuerst werden die charakteristischen Betriebsspannungen und dann die Schaltzeiten an der Testzelle gemessen. Die Betriebsspannung an den Zellelektroden wird in Form von Rechteckspannung mit alternierendem Vorzeichen (Frequenz 100 Hz) und variabler Amplitude wie im Folgenden beschrieben angelegt.

Während die Betriebspannung erhöht wird, wird die Transmission gemessen. Das Erreichen des Maximalwerts der Transmission legt die charakteristische Größe der Betriebsspannung V₁₀₀ fest. Gleichermaßen wird die charakteristische Spannung V₁₀ bei 10 % der maximalen Transmission bestimmt. Diese Werte werden bei verschiedenen Temperaturen im Bereich der blauen Phase gemessen.

Am oberen und unteren Ende des Temperaturbereichs der blauen Phase werden relativ hohe charakteristische Betriebsspannungen V₁₀₀ beobachtet. Im Bereich der minimalen Betriebsspannung steigt V₁₀₀ in der Regel nur langsam mit der Temperatur. Dieser Temperaturbereich, begrenzt durch T₁ und T₂ wird als nutzbarer, flacher Temperaturbereich (FB) bezeichnet. Die Breite dieses "Flachbereichs" (FB) beträgt (T₂ - T₁) und wird als Breite des Flachbereichs (BFB) (engl. 'flat range') bezeichnet. Die genauen Werte von T₁ und T₂ werden durch die Schnittpunkte von Tangenten an den flachen Kurvenabschnitt FB und die benachbarten steilen Kurvenabschnitte im V₁₀₀-Temperatur-Diagramm ermittelt.

Im zweiten Teil der Messung werden die Schaltzeiten beim Ein- und Ausschalten ermittelt (τₒₙ, τ_{off}). Die Schaltzeit τₒₙ ist definiert durch die Zeit bis zum Erreichen von 90 % Intensität nach dem Anlegen einer Spannung der Höhe von V₁₀₀ bei der gewählten Temperatur. Die Schaltzeit τ_{off} ist definiert durch die Zeit bis zur Abnahme um 90 % ausgehend von maximaler Intensität bei V₁₀₀ nach dem Erniedrigen der Spannung auf 0 V. Auch die Schaltzeit wird bei verschiedenen Temperaturen im Bereich der blauen Phase ermittelt.

Als weitere Charakterisierung wird bei einer Temperatur innerhalb des FB die Transmission bei kontinuierlich steigender und fallender Betriebsspannung zwischen 0 V und V₁₀₀ gemessen. Den Unterschied zwischen beiden Kurven bezeichnet man als Hysterese. Die Differenz der Transmissionen bei 0,5·V₁₀₀ bzw. die Differenz der Spannungen bei 50 % Transmission sind beispielsweise charakteristische Hysteresewerte und werden als ΔT₅₀ respektive ΔV₅₀ bezeichnet.

Als weitere Kenngröße kann das Verhältnis der Transmission im spannungslosen Zustand vor und nach Durchlaufen eines Schaltzyklusses gemessen werden. Dieses Transmissionsverhältnis wird als "Memory-Effekt" bezeichnet. Der Wert des Memory-Effekts liegt im Idealzustand bei 1,0. Werte über 1 bedeuten, dass ein gewisser Memory-Effekt in Form einer zu hohen Resttransmission nach dem Ein- und Ausschalten der Zelle vorliegt. Auch dieser Wert wird im Arbeitsbereich der blauen Phase (FB) ermittelt.

Die Ergebnisse sind in Tabelle 2 zusammengefasst.

**Tabelle 2:**

| Messwerte (20 °C) | **M1** |
|---|---|
| Übergangspunkt vor der Polymerisation | 34,4 °C |
| Polymerisations-temperatur | 34,9 °C |
| V₁₀₀ (20 °C) | 48 V |
| ΔV₅₀ (20 °C) | 1,9 |
| Kontrast, Einschalten | 206 |
| Kontrast, Ausschalten | 208 |
| Memory Effekt | 0,99 |

Die polymerisierbare Mischung wird bei einer Temperatur von ca. 30-50 °C am unteren Ende des Temperaturbereichs der blauen Phase in einem einzigen Bestrahlungsschritt polymerisiert (Details vgl. oben).

Die polymerstabilisierten flüssigkristallinen Medien zeigen über einen breiten Temperaturbereich eine blaue Phase.

Das polymerstabilisierte Medium M1, hergestellt unter Verwendung des erfindungsgemäßen Monomers (1), zeigt eine Verringerung der Hysterese (ΔV₅₀) sowie einen guten Kontrast beim Einschalten und beim Ausschalten gegenüber herkömmlichen Medien aus dem Stand der Technik. Insbesondere liegen im erfindungsgemäßen Medium M1 der Kontrast beim Einschalten und der Kontrast beim Ausschalten nahe beieinander, was eine sehr gute Stabilisierung der blauen Phase bedeutet.

Daraus ist ersichtlich, dass sich die erfindungsgemäßen Monomere besonders gut zur Stabilisierung von blauen Phasen, insbesondere bei Medien mit hoher Konzentration an chiralem Dotierstoff, eignen.

## Patentansprüche

1. Verbindungen der Formel I
P^{a}-(Sp^{a})ₛ₁-(A¹)ₙ₁-A²-Q¹-A³-(Sp^{b})ₛ₂-P^{b} I
worin die einzelnen Reste folgende Bedeutung besitzen
P^{a} eine polymerisierbare Gruppe,
P^{b} eine polymerisierbare Gruppe, H oder F,
Sp^{a}, Sp^{b} jeweils unabhängig voneinander eine Abstandsgruppe,
s1, s2 jeweils unabhängig voneinander 0 oder 1,
n1 0 oder 1,
Q¹ -CF₂O-,
A² eine Gruppe der Formel 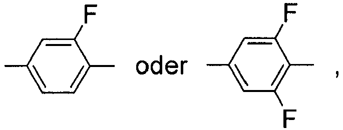
A³ eine Gruppe der Formel 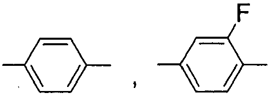 oder 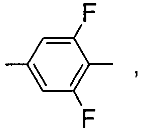
A¹ jeweils unabhängig voneinander ein Rest ausgewählt aus
der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können, und
L bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen.

2. Verbindungen nach Anspruch 1 der Formel I**dadurch gekennzeichnet, dass**
n1 0 ist.

3. Verbindungen nach Anspruch 1 oder 2, **dadurch gekennzeichnet, dass**
s1 und s2 jeweils 1 bedeuten.

4. Verbindungen nach einem oder mehreren der Ansprüche 1 bis 3, **dadurch gekennzeichnet, dass**
A² unabhängig voneinander 1,4-Phenylen oder 1,3-Phenylen, worin auch ein oder mehrere H-Atome durch L ersetzt sein können,
bedeuten.

5. Verbindungen nach einem oder mehreren der Ansprüche 1 bis 4, der Formel 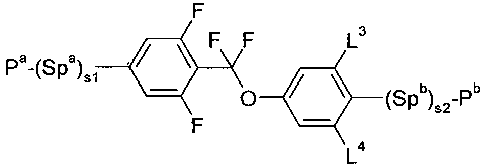 worin P^{a}, P^{b}, Sp^{a}, Sp^{b}, s1, s2 wie für Formel I definiert sind, und L³ und L⁴ unabhängig voneinander H oder F bedeuten.

6. FK-Medium enthaltend eine oder mehrere Verbindungen der Formel I
P^{a}-(Sp^{a})ₛ₁-(A¹)ₙ₁-A²-Q¹-A³-(Sp^{b})ₛ₂-P^{b} I
worin die einzelnen Reste folgende Bedeutung besitzen
P^{a}, P^{b} jeweils unabhängig voneinander eine polymerisierbare Gruppe,
Sp^{a}, Sp^{b} jeweils unabhängig voneinander eine Abstandsgruppe,
s1, s2 jeweils unabhängig voneinander 0 oder 1,
n1 0 oder 1,
Q¹ -CF₂O-,
A² eine Gruppe der Formel 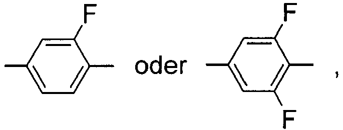
A³ eine Gruppe der Formel 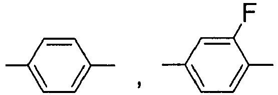
oder 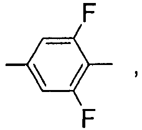
A¹ jeweils unabhängig voneinander ein Rest ausgewählt aus
der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können
L bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
oder ein Polymer erhältlich durch Polymerisation einer oder mehrerer Verbindungen der Formel I.

7. FK-Medium nach Anspruch 6, **dadurch gekennzeichnet, dass** es zusätzlich eine oder mehrere andere polymerisierbare Verbindungen und/oder eine oder mehrere nicht-polymerisierbare flüssigkristalline Verbindungen enthält.

8. FK-Medium nach Anspruch 6 oder 7, **dadurch gekennzeichnet, dass** es folgende Komponenten enthält:
- eine polymerisierbare Komponente A, enthaltend eine oder mehrere Verbindungen der Formel I nach Anspruch 1,
- eine flüssigkristalline Komponente B, enthaltend eine oder mehrere Verbindungen der Formel II 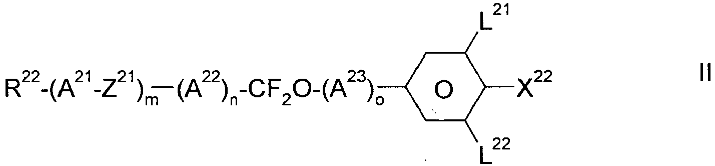 worin die einzelnen Reste folgende Bedeutung besitzen
R²² H, F, Cl oder geradkettiges oder verzweigtes Alkyl mit 1 bis 20 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -NH-, -NR⁰¹-, -SiR⁰¹R⁰²-, -CO-, -COO-, -OCO-, -O-CO-O-, -S-CO-, -CO-S-, -CY⁰¹=CY⁰²- oder -C≡C- so ersetzt sein können, dass O- und/oder S-Atome nicht direkt miteinander verknüpft sind,
Y⁰¹, Y⁰² jeweils unabhängig voneinander F, Cl oder CN, einer der Reste Y⁰¹ und Y⁰² auch H,
R⁰¹, R⁰² jeweils unabhängig voneinander H oder Alkyl mit 1 bis 12 C-Atomen,
A²¹, A²², A²³ jeweils unabhängig voneinander und bei jedem Auftreten gleich oder verschieden 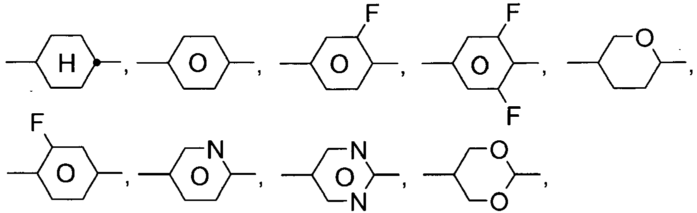
Z²¹ bei jedem Auftreten gleich oder verschieden, eine Einfachbindung, -(CH₂)₄-, -CH₂CH₂-, -CF₂-CF₂-, -CF2-CH₂-, -CH₂-CF₂-, -CH=CH-, -CF=CF-, -CF=CH-, -(CH₂)₃O- -O(CH₂)₃-, -CH=CF-, -C≡C-, -CH₂O-, -OCH₂-, -CF₂O-, -OCF₂-, -CO-O- oder -O-CO-,
X²² F, Cl, -CN, -NCS, -SF₅, -SO₂CF₃, oder Alkyl, Alkenyl, Alkenyloxy, Alkylalkoxy oder Alkoxy mit 1 bis 3 C-Atomen, welches durch F, Cl oder CN ein- oder mehrfach substituiert ist,
L²¹, L²² jeweils unabhängig voneinander H oder F,
m 0, 1 oder 2,
n 1, 2 oder 3,
o 0, 1 oder 2, wobei
m + n + o 1,2,3 oder 4, vorzugsweise 2, 3 oder 4 bedeutet,
- optional eine flüssigkristalline Komponente C, enthaltend eine oder mehrere Verbindungen der Formel III 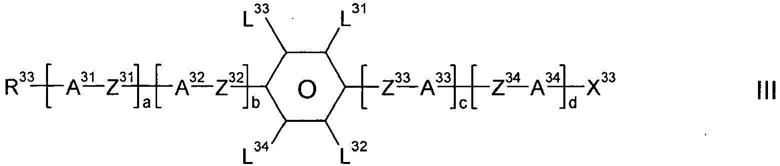 worin
a, b, c, d jeweils unabhängig voneinander 0, 1 oder 2, wobei
a + b + c + d 0, 1, 2, 3 oder 4 ist,
A³¹, A³², A³³, A³⁴ jeweils unabhängig voneinander, und bei jedem Auftreten gleich oder verschieden, 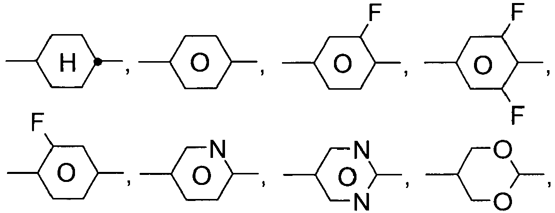
Z³¹, Z³², Z³³, Z³⁴ jeweils unabhängig voneinander, und bei jedem Auftreten gleich oder verschieden, eine Einfachbindung, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡D-, -CH₂O-, -OCH₂-, -CF₂O-, -OCF₂-, -CO-O- oder -O-CO-,
R³³ Alkyl oder Alkoxy mit 1 bis 15 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, -CH=CH-, -C≡C-, -CO-O- und/oder -O-CO- so ersetzt sein können, dass O- und/oder S-Atome nicht direkt miteinander verknüpft sind, vorzugsweise ein geradkettiger Alkyl-, Alkoxy-, Alkenyl-, Alkenyloxy- oder -O-Alkylen-O-Rest mit bis zu 10 C-Artomen, welcher unsubstituiert oder ein- oder mehrfach durch F oder Cl substituiert ist,
L³¹, L³², L³³, L³⁴ jeweils unabhängig voneinander H, F, Cl, CN, oder Alkyl oder Alkoxy mit 1 bis 15 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, -CH=CH-, -C≡C-, -CO-O- und/oder -O-CO- so ersetzt sein können, dass O- und/oder S-Atome nicht direkt miteinander verknüpft sind, mit der Massgabe dass mindestens einer der Reste L³¹, L³², L³³ und L³⁴ von H verschieden ist,
X³³ F, Cl, CF₃, OCF₃, CN, NCS, -SF₅ oder -SO₂-R^{z},
R^{x} und R^{y} jeweils unabhängig voneinander H, Alkyl oder Alkoxy mit 1 bis 7 C-Atomen, vorzugsweise Methyl, Ethyl, Propyl oder Butyl, und
R^{z} Alkyl mit 1 bis 7 C-Atomen, welches unsubstituiert, oder durch F oder Cl ein- oder mehrfach substituiert ist, vorzugsweise CF₃, C₂F₅ oder n-C₄F₉,
- und eine Komponente D, enthaltend eine oder mehrere optisch aktive und/oder chirale Verbindungen.

9. Verfahren zur Herstellung eines FK-Mediums nach einem oder mehreren der Ansprüche 6 bis 8, indem man eine oder mehrere flüssigkristalline Verbindungen oder ein Flüssigkristallmedium mit einer oder mehreren Verbindungen der Formel I nach einem der Ansprüche 1 bis 4, und gegebenenfalls mit weiteren chiralen und/oder optisch aktiven Verbindungen und/oder Additiven mischt.

10. FK-Anzeige, enthaltend enthaltend ein FK-Medium nach einem oder mehreren der Ansprüche 6 bis 8.

11. FK-Anzeige nach Anspruch 10, **dadurch gekennzeichnet, dass** sie eine Anzeige mit blauer Phase, eine PS- oder PSA-Anzeige, eine PSA-VA-, PSA-OCB-, PSA-IPS-, PSA-FFS- oder PSA-TN-Anzeige ist.

12. FK-Anzeige nach Anspruch 10 oder 11, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, **dadurch gekennzeichnet, dass** mindestens eine der polymerisierbaren Verbindungen eine Verbindung der Formel I nach einem der Ansprüche 1 bis 4 ist.

13. Verwendung von Verbindungen der Formel I nach einem oder mehreren der Ansprüche 1 bis 5 und diese Verbindungen enthaltende FK-Medien in FK-Anzeigen zur Stabilisierung der blauen Phase.

## Claims

1. Compounds of the formula I
P^{a}-(Sp^{a})ₛ₁-(A¹)ₙ₁-A²-Q¹-A³-(Sp^{b})ₛ₂-P^{b} I
in which the individual radicals have the following meanings:
P^{a} denotes a polymerisable group,
P^{b} denotes a polymerisable group, H or F,
Sp^{a}, Sp^{b} each, independently of one another, denote a spacer group,
s1, s2 each, independently of one another, denote 0 or 1,
n1 denotes 0 or 1,
Q¹ denotes -CF₂O-,
A² denotes a group of the formula 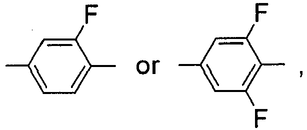
A³ denotes a group of the formula 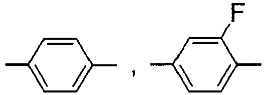
or 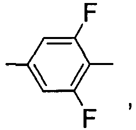
A¹ in each case, independently of one another, denotes a radical selected from the group consisting of 1,4-phenylene and 1,3-phenylene, in which, in addition, one or two CH groups may be replaced by N and in which, in addition, one or more H atoms may be replaced by L, and
L on each occurrence, identically or differently, denotes F, CI, CN, SCN, SF₅ or straight-chain or branched, in each case optionally fluorinated alkyl, alkoxy, alkylcarbonyl, alkoxycarbonyl, alkylcarbonyloxy or alkoxycarbonyloxy having 1 to 12 C atoms.

2. Compounds according to Claim 1 of the formula I, **characterised in that**
n1 is 0.

3. Compounds according to Claim 1 or 2, **characterised in that** s1 and s2 each denote 1.

4. Compounds according to one or more of Claims 1 to 3, **characterised in that**
A², independently of one another, denote 1,4-phenylene or 1,3-phenylene, in which, in addition, one or more H atoms may be replaced by L.

5. Compounds according to one or more of Claims 1 to 4, of the formula 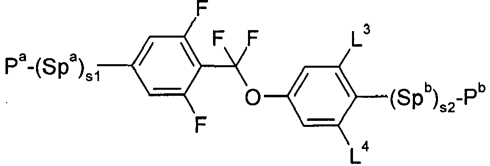 in which P^{a}, P^{b}, Sp^{a}, Sp^{b}, s1, s2 are as defined for formula I, and L³ and L⁴, independently of one another, denote H or F.

6. LC medium comprising one or more compounds of the formula I
P^{a}-(Sp^{a})ₛ₁-(A¹)ₙ₁-A²-Q¹-A³-(Sp^{b})ₛ₂-P^{b} I
in which the individual radicals have the following meanings:
P^{a}, P^{b} each, independently of one another, denote a polymerisable group,
Sp^{a}, Sp^{b} each, independently of one another, denote a spacer group,
s1, s2 each, independently of one another, denote 0 or 1,
n1 denotes 0 or 1,
Q¹ denotes -CF₂O-,
A² denotes a group of the formula 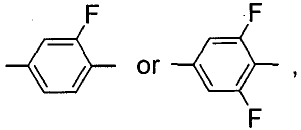
A³ denotes a group of the formula 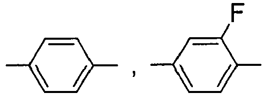
or 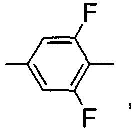
A¹ in each case, independently of one another, denotes a radical selected from
the group consisting of 1,4-phenylene and 1,3-phenylene, in which, in addition, one or two CH groups may be replaced by N and in which, in addition, one or more H atoms may be replaced by L, and
L on each occurrence, identically or differently, denotes F, CI, CN, SCN, SF₅ or straight-chain or branched, in each case optionally fluorinated alkyl, alkoxy, alkylcarbonyl, alkoxycarbonyl, alkylcarbonyloxy or alkoxycarbonyloxy having 1 to 12 C atoms.
or a polymer obtainable by polymerisation of one or more compounds of the formula I.

7. LC medium according to Claim 6, **characterised in that** it additionally comprises one or more other polymerisable compounds and/or one or more unpolymerisable liquid-crystalline compounds.

8. LC medium according to Claim 6 or 7, **characterised in that** it comprises the following components:
- a polymerisable component A comprising one or more compounds of the formula I according to Claim 1,
- a liquid-crystalline component B comprising one or more compounds of the formula II 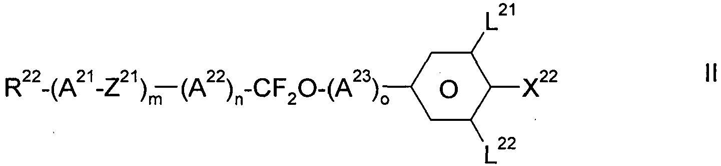 in which the individual radicals have the following meanings:
R²² denotes H, F, CI or straight-chain or branched alkyl having 1 to 20 C atoms, which is unsubstituted or mono- or polysubstituted by F, CI or CN, and in which, in addition, one or more non-adjacent CH₂ groups may also each be replaced, independently of one another, by -O-, -S-, -NH-, -NR⁰¹-, -SiR⁰¹R⁰²-, -CO-, -COO-, -OCO-, -O-CO-O-, -S-CO-, -CO-S-, -CY⁰¹=CY⁰²- or -C≡C- in such a way that O and/or S atoms are not linked directly to one another,
Y⁰¹, Y⁰² each, independently of one another, denote F, CI or CN, one of the radicals Y⁰¹ and Y⁰² also denotes H,
R⁰¹, R⁰² each, independently of one another, denote H or alkyl having 1 to 12 C atoms,
A²¹, A²², A²³ each, independently of one another and identically or differently on each occurrence, denote 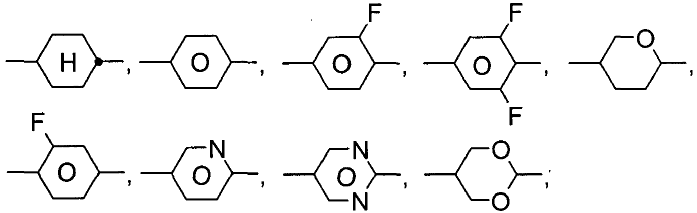
Z²¹ on each occurrence, identically or differently, denotes a single bond, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-,
X²² denotes F, CI, -CN, -NCS, -SF₅, -SO₂CF₃, or alkyl, alkenyl, alkenyloxy, alkylalkoxy or alkoxy having 1 to 3 C atoms which is mono- or polysubstituted by F, CI or CN,
L²¹, L²² each, independently of one another, denote H or F,
m denotes 0, 1 or 2,
n denotes 1, 2 or 3,
o denotes 0, 1 or 2, where
m + n + o denotes 1, 2, 3 or 4, preferably 2, 3 or 4,
- optionally a liquid-crystalline component C comprising one or more compounds of the formula III 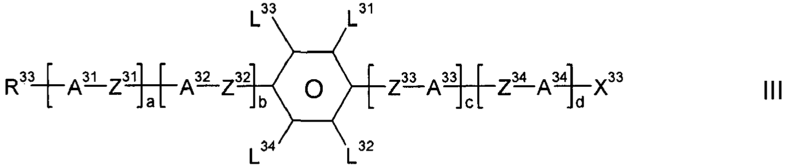 in which
a, b, c, d each, independently of one another, denote 0, 1 or 2, where
a + b + c + d is 0, 1, 2, 3 or 4,
A³¹, A³², A³³, A³⁴ each, independently of one another and identically or differently on each occurrence, denote 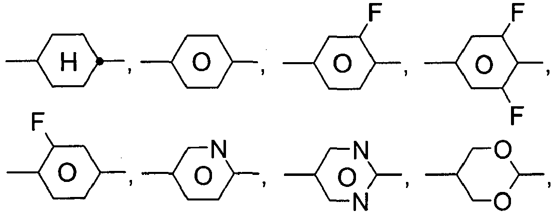
Z³¹, Z³², Z³³, Z³⁴ each, independently of one another and identically or differently on each occurrence, denote a single bond, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-,
R³³ denotes alkyl or alkoxy having 1 to 15 C atoms, which is unsubstituted or mono- or polysubstituted by F, CI or CN, and in which, in addition, one or more non-adjacent CH₂ groups may also each be replaced, independently of one another, by -O-, -S-, -SiR^{x}R^{y}-, -CH=CH-, -C≡C-, -CO-O- and/or -O-CO- in such a way that O and/or S atoms are not linked directly to one another, preferably a straight-chain alkyl, alkoxy, alkenyl, alkenyloxy or-O-alkylene-O-radical having up to 10 C atoms, which is unsubstituted or mono- or polysubstituted by F or Cl,
L³¹, L³², L³³, L³⁴ each, independently of one another, denote H, F, CI, CN, or alkyl or alkoxy having 1 to 15 C atoms, which is unsubstituted or mono- or polysubstituted by F, CI or CN, and in which, in addition, one or more non-adjacent CH₂ groups may also each be replaced, independently of one another, by -O-, -S-, -SiR^{x}R^{y}-, -CH=CH-, -C≡C-, -CO-O- and/or -O-CO- in such a way that O and/or S atoms are not linked directly to one another, with the proviso that at least one of the radicals L³¹, L³², L³³ and L³⁴ is other than H,
X³³ denotes F, Cl, CF₃, OCF₃, CN, NCS, -SF₅ or -SO₂-R^{z},
R^{x} and R^{y} each, independently of one another, denote H, alkyl or alkoxy having 1 to 7 C atoms, preferably methyl, ethyl, propyl or butyl, and
R^{z} denotes alkyl having 1 to 7 C atoms, which is unsubstituted or mono- or polysubstituted by F or CI, preferably CF₃, C₂F₅ or n-C4F9,
- and a component D comprising one or more optically active and/or chiral compounds.

9. Process for the preparation of an LC medium according to one or more of Claims 6 to 8, in which one or more liquid-crystalline compounds or a liquid-crystal medium are mixed with one or more compounds of the formula I according to one of Claims 1 to 4, and optionally with further chiral and/or optically active compounds and/or additives.

10. LC display containing an LC medium according to one or more of Claims 6 to 8.

11. LC display according to Claim 10, **characterised in that** it is a display having a blue phase, a PS or PSA display, a PSA-VA, PSA-OCB, PSA-IPS, PSA-FFS or PSA-TN display.

12. LC display according to Claim 10 or 11, containing an LC cell having two substrates and two electrodes, where at least one substrate allows light to pass through and at least one substrate has one or two electrodes, and a layer, located between the substrates, of an LC medium comprising a polymerised component and a low-molecular-weight component, where the polymerised component is obtainable by polymerisation of one or more polymerisable compounds in the LC medium between the substrates of the LC cell, **characterised in that** at least one of the polymerisable compounds is a compound of the formula I according to one of Claims 1 to 4.

13. Use of compounds of the formula I according to one or more of Claims 1 to 5 and LC media comprising these compounds in LC displays for stabilisation of the blue phase.

## Revendications

1. Composés de la formule I :
P^{a}-(Sp^{a})ₛ₁-(A¹)ₙ₁-A²-Q¹-A³-(Sp^{b})ₛ₂-P^{b} I
dans laquelle les radicaux individuels présentent les significations qui suivent :
P^{a} représente un groupe polymérisable,
P^{b} représente un groupe polymérisable, H ou F,
Sp^{a}, Sp^{b} représentent chacun, indépendamment l'un de l'autre, un groupe d'espaceur,
s1, s2 représentent chacun, indépendamment l'un de l'autre, 0 ou 1,
n1 représente 0 ou 1,
Q¹ représente -CF₂O-,
A² représente un groupe de la formule 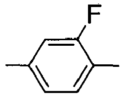
ou 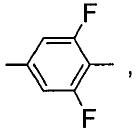
A³ représente un groupe de la formule 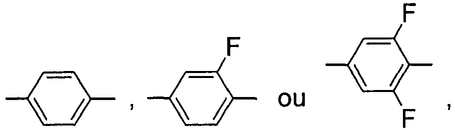
A¹ représente dans chaque cas, de manière indépendante, un radical choisi parmi :
le groupe constitué par 1,4-phénylène et 1,3-phénylène, où, en outre, un ou deux groupe(s) CH peut/peuvent être remplacé(s) par N et où, en outre, un ou plusieurs atome(s) de H peut/peuvent être remplacé(s) par L, et
L représente pour chaque occurrence, de manière identique ou différente, F, CI, CN, SCN, SF₅ ou un radical alkyle, alcoxy, alkylcarbonyle, alcoxycarbonyle, alkylcarbonyloxy ou alcoxycarbonyloxy en chaîne droite ou ramifié, dans chaque cas en option fluoré, comportant 1 à 12 atome(s) deC.

2. Composés selon la revendication 1 de la formule I, **caractérisés en ce que**:
n1 est 0.

3. Composés selon la revendication 1 ou 2, **caractérisés en ce que** :
s1 et s2 représentent chacun 1.

4. Composés selon une ou plusieurs des revendications 1 à 3, **caractérisés en ce que** :
A² représente, de manière indépendante, 1,4-phénylène ou 1,3-phénylène, où, en outre, un ou plusieurs atome(s) de H peut/peuvent être remplacé(s) par L.

5. Composés selon une ou plusieurs des revendications 1 à 4, de la formule : 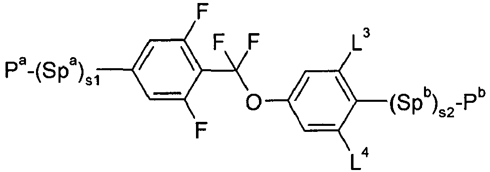 dans laquelle P^{a}, P^{b}, Sp^{a}, Sp^{b}, s1, s2 sont comme défini pour la formule I,
et L³ et L⁴ représentent, indépendamment l'un de l'autre, H ou F.

6. Milieu LC comprenant un ou plusieurs composé(s) de la formule I :
P^{a}-(Sp^{a})ₛ₁-(A¹)ₙ₁-A²-Q¹-A³-(Sp^{b})ₛ₂-P^{b} I
dans laquelle les radicaux individuels présentent les significations qui suivent :
P^{a}, P^{b} représentent chacun, indépendamment l'un de l'autre un groupe polymérisable,
Sp^{a}, Sp^{b} représentent chacun, indépendamment l'un de l'autre, un groupe d'espaceur,
s1, s2 représentent chacun, indépendamment l'un de l'autre, 0 ou 1,
n1 représente 0 ou 1,
Q¹ représente -CF₂O-,
A² représente un groupe de la formule 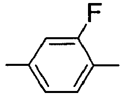 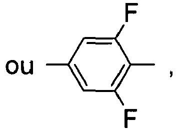
A³ représente un groupe de la formule 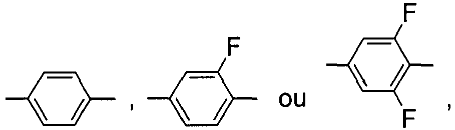
A¹ représente dans chaque cas, de manière indépendante, un radical choisi parmi :
le groupe constitué par 1,4-phénylène et 1,3-phénylène, où, en outre, un ou deux groupe(s) CH peut/peuvent être remplacé(s) par N et où, en outre, un ou plusieurs atome(s) de H peut/peuvent être remplacé(s) par L, et
L représente pour chaque occurrence, de manière identique ou différente, F, CI, CN, SCN, SF₅ ou un radical alkyle, alcoxy, alkylcarbonyle, alcoxycarbonyle, alkylcarbonyloxy ou alcoxycarbonyloxy en chaîne droite ou ramifié, dans chaque cas en option fluoré, comportant 1 à 12 atome(s) de C,
ou un polymère qui peut être obtenu par polymérisation d'un ou de plusieurs composé(s) de la formule I.

7. Milieu LC selon la revendication 6, **caractérisé en ce qu'**il comprend de façon additionnelle un ou plusieurs autre(s) composé(s) polymérisable(s) et/ou un ou plusieurs composé(s) cristallin(s) liquide(s) non polymérisable(s).

8. Milieu LC selon la revendication 6 ou 7, **caractérisé en ce qu'**il comprend les composants qui suivent :
- un composant polymérisable A comprenant un ou plusieurs composé(s) de la formule I selon la revendication 1,
- un composant cristallin liquide B comprenant un ou plusieurs composé(s) de la formule II : 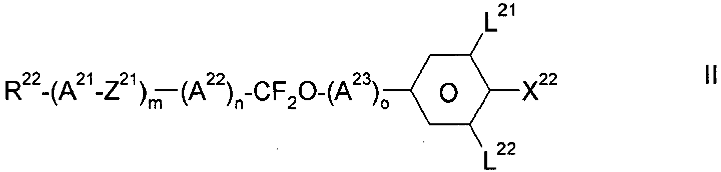 dans laquelle les radicaux individuels présentent les significations qui suivent:
R²² représente H, F, CI ou alkyle en chaîne droite ou ramifié comportant 1 à 20 atome(s) de C, lequel est non substitué ou monosubstitué ou polysubstitué par F, CI ou CN, et dans laquelle, en outre, un ou plusieurs groupe(s) CH₂ non adjacents peut/peuvent chacun également être remplacé(s), indépendamment les uns des autres, par -O-, -S-, -NH-, -NR⁰¹-, -SiR⁰¹R⁰²-, -CO-, -COO-, -OCO-, -O-CO-O-, -S-CO-, -CO-S-, -CY⁰¹=CY⁰²- ou -C≡C- de telle sorte que des atomes de O et/ou de S ne soient pas liés directement les uns aux autres,
Y⁰¹, Y⁰² représentent chacun, indépendamment l'un de l'autre, F, CI ou CN, l'un des radicaux Y⁰¹ et Y⁰² représente également H,
R⁰¹, R⁰² représentent chacun, indépendamment l'un de l'autre, H ou alkyle comportant 1 à 12 atome(s) de C,
A²¹ , A²², A²³ représentent chacun, indépendamment les uns des autres et de manière identique ou différente pour chaque occurrence, 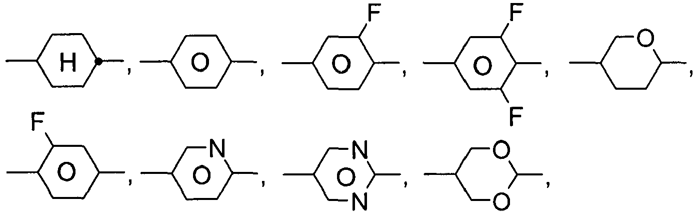
Z²¹ représente pour chaque occurrence, de manière identique ou différente, une liaison simple, -(CH₂)₄-, -CH₂CH₂-, -CF₂-F₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- ou -O-CO-,
X²² représente F, CI, -CN, -NCS, -SF₅, -SO₂CF₃, ou alkyle, alkényle, alkényloxy, alkylalcoxy ou alcoxy comportant 1 à 3 atome(s) de C lequel est monosubstitué ou polysubstitué par F, CI ou CN,
L²¹, L²² représentent chacun, indépendamment l'un de l'autre, H ou F,
m représente 0, 1 ou 2,
n représente 1, 2 ou 3,
o représente 0, 1 ou 2, où :
m + n + o représente 1, 2, 3 ou 4, de façon préférable 2, 3 ou 4,
- en option, un composant cristallin liquide C comprenant un ou plusieurs composé(s) de la formule III : 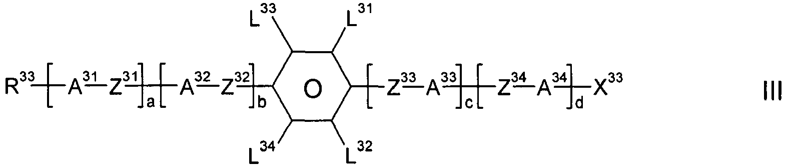 dans laquelle :
a, b, c, d représentent chacun, indépendamment les uns des autres, 0, 1 ou 2, où :
a + b + c + d est 0, 1, 2, 3 ou 4,
A³¹, A³², A³³, A³⁴ représentent chacun, indépendamment les uns des autres et de manière identique ou différente pour chaque occurrence : 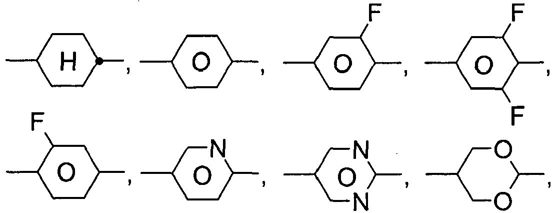
Z³¹, Z³², Z³³, Z³⁴ représentent chacun, indépendamment les uns des autres et de manière identique ou différente pour chaque occurrence, une liaison simple, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- ou -O-CO-,
R³³ représente alkyle ou alcoxy comportant 1 à 15 atome(s) de C, lequel est non substitué ou monosubstitué ou polysubstitué par F, Cl ou CN, et où, en outre, un ou plusieurs groupe(s) CH₂ non adjacents peut/peuvent chacun également être remplacés(s), indépendamment les uns des autres, par -O-, -S-, -SiR^{x}R^{y}-, -CH=CH-, -C≡C-, -CO-O- et/ou -O-CO- de telle sorte que des atomes de O et/ou de S ne soient pas liés directement les uns aux autres, de façon préférable un radical alkyle, alcoxy, alkényle, alkényloxy ou -O-alkylène-O- en chaîne droite comportant jusqu'à 10 atomes de C, lequel est non substitué ou monosubstitué ou polysubstitué par F ou CI,
L³¹, L³², L³³, L³⁴ représentent chacun, indépendamment les uns des autres, H, F, Cl, CN, ou alkyle ou alcoxy comportant 1 à 15 atome(s) de C, lequel est non substitué ou monosubstitué ou polysubstitué par F, CI ou CN, et où, en outre, un ou plusieurs groupe(s) CH₂ non adjacents peut/ peuvent chacun également être remplacé(s), indépendamment les uns des autres, par -O-, -S-, -SiR^{x}R^{y}-, -CH=CH-, -C≡C-, -CO-O- et/ou -O-CO- de telle sorte que des atomes de O et/ou de S ne soient pas liés directement les uns aux autres, étant entendu qu'au moins l'un des radicaux L³¹, L³², L³³ et L³⁴ est autre que H,
X³³ représente F, CI, CF₃, OCF₃, CN, NCS, -SF₅ ou -SO₂-R^{z},
R^{x} et R^{y} représentent chacun, indépendamment l'un de l'autre, H, alkyle ou alcoxy comportant 1 à 7 atome(s) de C, de façon préférable méthyle, éthyle, propyle ou butyle, et
R^{z} représente alkyle comportant 1 à 7 atome(s) de C, lequel est non substitué ou monosubstitué ou polysubstitué par F ou CI, de façon préférable CF₃, C₂F₅ ou n-C₄F₉,
- et un composant D comprenant un ou plusieurs composé(s) optiquement actif(s) et/ou chiral/chiraux.

9. Procédé pour la préparation d'un milieu LC selon une ou plusieurs des revendications 6 à 8, dans lequel un ou plusieurs composé(s) cristallin(s) liquide(s) ou un milieu cristallin liquide est/sont mélangé(s) avec un ou plusieurs composé(s) de la formule I selon l'une des revendications 1 à 4, et en option, avec d'autres composés chiraux et/ou optiquement actifs et/ou d'autres additifs.

10. Affichage LC contenant un milieu LC selon une ou plusieurs des revendications 6 à 8.

11. Affichage LC selon la revendication 10, **caractérisé en ce qu'**il s'agit d'un affichage comportant une phase de bleu, d'un affichage PS ou PSA, d'un affichage PSA-VA, PSA-OCB, PSA-IPS, PSA-FFS ou PSA-TN.

12. Affichage LC selon la revendication 10 ou 11, contenant une cellule LC comportant deux substrats et deux électrodes, dans lequel au moins un substrat permet le passage de la lumière au travers et au moins un substrat comporte une ou deux électrode(s), et une couche, située entre les substrats, en un milieu LC comprenant un composant polymérisé et un composant de poids moléculaire faible, où le composant polymérisé peut être obtenu par polymérisation d'un composé polymérisable ou de plusieurs composés polymérisables dans le milieu LC entre les substrats de la cellule LC, **caractérisé en ce qu'**au moins l'un des composés polymérisables est un composé de la formule I selon l'une des revendications 1 à 4.

13. Utilisation de composés de la formule I selon une ou plusieurs des revendications 1 à 5 et milieux LC comprenant ces composés dans des affichages LC pour la stabilisation de la phase de bleu.
